(19) Europäisches Patentamt
European Patent Office
Office européen des brevets

(11) **EP 4 509 506 A1**

(12) # EUROPEAN PATENT APPLICATION

published in accordance with Art. 153(4) EPC


(43) Date of publication:
**19.02.2025 Bulletin 2025/08**

(21) Application number: **23787814.5**

(22) Date of filing: **14.04.2023**

(51) International Patent Classification (IPC):
***C07D 417/04*** *(2006.01)* ***A61K 31/545*** *(2006.01)*
***A61K 31/5415*** *(2006.01)* ***A61P 35/00*** *(2006.01)*
***A61P 25/28*** *(2006.01)*

(52) Cooperative Patent Classification (CPC):
**A61K 31/5415; A61K 31/545; A61P 25/28; A61P 35/00; C07D 417/04**

(86) International application number:
**PCT/CN2023/088285**

(87) International publication number:
**WO 2023/198172 (19.10.2023 Gazette 2023/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **15.04.2022 CN 202210401425**



(71) Applicant: **Suzhou Genhouse Bio Co., Ltd.**
**Suzhou,Jiangsu 215123 (CN)**

(72) Inventors:
- **ZHANG, Guiping**
  **Suzhou, Jiangsu 215123 (CN)**
- **WANG, Kuifeng**
  **Suzhou, Jiangsu 215123 (CN)**
- **LI, Jiapeng**
  **Suzhou, Jiangsu 215123 (CN)**
- **Faridoon**
  **Suzhou, Jiangsu 215123 (CN)**
- **ZHENG, Jiyue**
  **Suzhou, Jiangsu 215123 (CN)**
- **LIU, Tao**
  **Suzhou, Jiangsu 215123 (CN)**
- **ZHANG, Tao**
  **Suzhou, Jiangsu 215123 (CN)**
- **TAN, Chensheng**
  **Suzhou, Jiangsu 215123 (CN)**
- **DONG, Xue**
  **Suzhou, Jiangsu 215123 (CN)**

(74) Representative: **Turner, Craig Robert**
**Venner Shipley LLP**
**200 Aldersgate**
**London EC1A 4HD (GB)**


(54) **OXADIAZOLE COMPOUND, PHARMACEUTICAL COMPOSITION COMPRISING SAME, AND USE THEREOF**

(57)


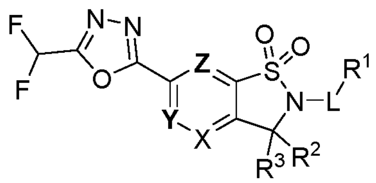

**(I)**

Disclosed are an oxadiazole compound having a structure of formula (I), a pharmaceutical composition comprising same, and the use thereof as an HDAC6 inhibitor.


EP 4 509 506 A1

## Description

### TECHNICAL FIELD

The present application relates to an oxadiazole compound, a pharmaceutical composition comprising the same, and use thereof as an HDAC6 inhibitor.

### BACKGROUND

Histone deacetylases (HDACs) can catalyze the deacetylation of histones or other proteins, and play important roles in a variety of biological processes, primarily through transcriptional inhibition. HDACs in humans can be divided into four classes. Class I includes HDAC1, HDAC2, HDAC3, and HDAC8; class II includes HDAC4, HDAC5, HDAC6, HDAC7, HDAC9, and HDAC10; class III includes SIRT1, SIRT2, SIRT3, SIRT4, SIRT5, SIRT6, and SIRT7; class IV includes HDAC11. HDACs of class II can be further divided into subclasses IIa (HDAC4, HDAC5, HDAC7, and HDAC9) and IIb (HDAC6 and HDAC10). HDAC6 mainly catalyzes the deacetylation of non-histone substrates, such as $\alpha$-tubulin and Hsp90. HDAC6 is involved in the pathological processes of a variety of diseases, including cancer, nervous system diseases, infections, cardiovascular diseases, immune diseases, and inflammation-related diseases.

In the field of tumor therapy, most HDAC inhibitors are broad-spectrum inhibitors, which are not selective for HDAC subtypes. The side effects of broad-spectrum inhibitors of the HDAC family are closely related to their inhibition of class I subtypes, particularly HDAC1 and HDAC2.

### BRIEF SUMMARY

The present application provides an oxadiazole compound, which can be used as an HDAC6 inhibitor for preventing or treating an HDAC6-related disease. The compounds of the present application are highly selective for HDAC6, thus avoiding the side effects of broad-spectrum HDAC inhibitors. In addition, the compounds of the present invention have more excellent properties such as better physicochemical properties (e.g., solubility and physical and/or chemical stability), improved pharmacokinetic properties (e.g., improved bioavailability, improved metabolic stability, and suitable half-life and duration of action), improved safety (less toxicity (e.g., reduced cardiotoxicity) and/or fewer side effects), and less susceptibility to drug resistance.

One aspect of the present invention provides a compound, or a pharmaceutically acceptable salt, an ester, a stereoisomer, a tautomer, a polymorph, a solvate, a metabolite, an isotopically labeled compound or a prodrug thereof, wherein the compound has a structure of formula (I):

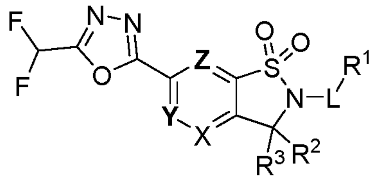

**(I)**

wherein

L is selected from a direct bond, -$C_{1-6}$ alkylene-, -$C_{2-6}$ alkenylene-, and -$C_{2-6}$ alkynylene-;
X is $CR^6$ or N;
Y is $CR^4$ or N;
Z is $CR^5$ or N;
$R^1$ is selected from H, halogen, -OH, -$NH_2$, -CN, -$NO_2$, $C_{1-6}$ alkyl, $C_{2-6}$ alkenyl, $C_{2-6}$ alkynyl, $C_{3-6}$ cyclohydrocarbyl, 3- to 10-membered heterocyclyl, $C_{6-10}$ aryl, 5- to 14-membered heteroaryl, $C_{6-12}$ aralkyl, - C(=O)$R^a$, -OC(=O)$R^a$, -OC(=O)$NR^aR^b$, -C(=O)O$R^a$, -O$R^a$, -S$R^a$, -S(=O)$R^a$, -S(=O)$_2R^a$, -S(=O)$_2NR^aR^b$, - $NR^aR^b$, -C(=O)$NR^aR^b$, -$NR^a$-C(=O)$R^b$, -$NR^a$-C(=O)O$R^b$, -$NR^a$-S(=O)$_2$-$R^b$, -$NR^a$-C(=O)-$NR^aR^b$, -$C_{1-6}$ alkylene-O$R^a$, -$C_{1-6}$ alkylene-$NR^aR^b$, and -O-$C_{1-6}$ alkylene-$NR^aR^b$;
$R^2$ and $R^3$ are each independently selected from H, halogen, -OH, -$NH_2$, -CN, -$NO_2$, $C_{1-6}$ alkyl, $C_{2-6}$ alkenyl, $C_{2-6}$ alkynyl, $C_{3-6}$ cyclohydrocarbyl, 3- to 10-membered heterocyclyl, $C_{6-10}$ aryl, 5- to 14-membered heteroaryl, $C_{6-12}$ aralkyl, -C(=O)$R^a$, -OC(=O)$R^a$, -OC(=O)$NR^aR^b$, -C(=O)O$R^a$, -O$R^a$, -S$R^a$, -S(=O)$R^a$,-S(=O)$_2R^a$, -S(=O)$_2NR^aR^b$, -$NR^aR^b$, -C(=O)$NR^aR^b$, -$NR^a$-C(=O)$R^b$, -$NR^a$-C(=O)O$R^b$, -$NR^a$-S(=O)$_2$-$R^b$, -$NR^a$-C(=O)-$NR^aR^b$, -$C_{1-6}$ alkylene-O$R^a$, -$C_{1-6}$ alkylene-$NR^aR^b$, and -O-$C_{1-6}$ alkylene-$NR^aR^b$; or $R^2$ and $R^3$ together form oxo (=O); or $R^2$ and $R^3$,

together with the carbon atom to which they are attached, form $C_{3-6}$ cyclohydrocarbyl or 3- to 10-membered heterocyclyl;

$R^4$, $R^5$, and $R^6$ are each independently selected from H, halogen, -OH, $-NH_2$, -CN, $-NO_2$, $C_{1-6}$ alkyl, $C_{2-6}$ alkenyl, $C_{2-6}$ alkynyl, $C_{3-6}$ cyclohydrocarbyl, 3- to 10-membered heterocyclyl, $C_{6-10}$ aryl, 5- to 14-membered heteroaryl, $C_{6-12}$ aralkyl, $-C(=O)R^a$, $-OC(=O)R^a$, $-OC(=O)NR^aR^b$, $-C(=O)OR^a$, $-OR^a$, $-SR^a$, $-S(=O)R^a$,$-S(=O)_2R^a$, $-S(=O)_2NR^aR^b$, $-NR^aR^b$, $-C(=O)NR^aR^b$, $-NR^a-C(=O)R^b$, $-NR^a-C(=O)OR^b$, $-NR^a-S(=O)_2-R^b$, $-NR^a-C(=O)-NR^aR^b$, $-C_{1-6}$ alkylene-$OR^a$, $-C_{1-6}$ alkylene-$NR^aR^b$, and $-O-C_{1-6}$ alkylene-$NR^aR^b$;

$R^a$ and $R^b$, at each occurrence, are each independently selected from H, $C_{1-6}$ alkyl, $C_{3-10}$ cyclohydrocarbyl, 3-to 10-membered heterocyclyl, $C_{6-10}$ aryl, 5- to 14-membered heteroaryl, and $C_{6-12}$ aralkyl;

the alkyl, alkylene, alkenyl, alkenylene, alkynyl, alkynylene, cyclohydrocarbyl, heterocyclyl, aryl, heteroaryl, and aralkyl, at each occurrence, are each optionally substituted with one or more substituents independently selected from: halogen, -OH, =O , $-NH_2$, -CN, $-NO_2$, $C_{1-6}$ alkyl, $C_{3-6}$ cyclohydrocarbyl, 3- to 10-membered heterocyclyl, $C_{6-10}$ aryl, 5- to 14-membered heteroaryl, $C_{6-12}$ aralkyl, $-C(=O)R^c$, $-OC(=O)R^c$, $-OC(=O)NR^cR^d$, $-C(=O)OR^c$, $-OR^c$, $-SR^c$, $-S(=O)R^c$, $-S(=O)_2R^c$, $-S(=O)_2NR^cR^d$, $-NR^cR^d$, $-C(=O)NR^cR^d$, $-NR^c-C(=O)R^d$, $- NR^c-C(=O)OR^d$, $-NR^c-S(=O)_2-R^d$, $-NR^c-C(=O)-NR^cR^d$, $-C_{1-6}$ alkylene-$OR^c$, $-C_{1-6}$ alkylene-$NR^cR^d$, and $-O-C_{1-6}$ alkylene-$NR^cR^d$; the alkyl, cyclohydrocarbyl, heterocyclyl, aryl, heteroaryl, and aralkyl are further optionally substituted with one or more substituents independently selected from: halogen, -OH, =O, - C(=O)O-tert-butyl, $-NH_2$, -CN, $-NO_2$, $C_{1-6}$ alkyl, halogenated $C_{1-6}$ alkyl, $-OC_{1-6}$ alkyl, -O-halogenated $C_{1-6}$ alkyl, $C_{3-6}$ cyclohydrocarbyl, 3- to 10-membered heterocyclyl, $C_{6-10}$ aryl, 5- to 14-membered heteroaryl, and $C_{6-12}$ aralkyl;

$R^c$ and $R^d$, at each occurrence, are each independently selected from H, $C_{1-6}$ alkyl, $C_{3-10}$ cyclohydrocarbyl, 3-to 10-membered heterocyclyl, $C_{6-10}$ aryl, 5- to 14-membered heteroaryl, and $C_{6-12}$ aralkyl; the alkyl, cyclohydrocarbyl, heterocyclyl, aryl, heteroaryl, and aralkyl are further optionally substituted with one or more substituents independently selected from: halogen, -OH, =O, -C(=O)O-*tert*-butyl, $-NH_2$, -CN, $-NO_2$, $C_{1-6}$ alkyl, halogenated $C_{1-6}$ alkyl, $C_{3-6}$ cyclohydrocarbyl, 3- to 10-membered heterocyclyl, $C_{6-10}$ aryl, 5- to 14-membered heteroaryl, and $C_{6-12}$ aralkyl.

Another aspect of the present invention provides a pharmaceutical composition comprising the compound, or the pharmaceutically acceptable salt, the ester, the stereoisomer, the tautomer, the polymorph, the solvate, the metabolite, the isotopically labeled compound or the prodrug thereof of the present invention, and one or more pharmaceutically acceptable carriers, wherein the pharmaceutical composition is preferably a solid formulation, a liquid formulation, or a transdermal formulation.

Another aspect of the present invention provides use of the compound, or the pharmaceutically acceptable salt, the ester, the stereoisomer, the tautomer, the polymorph, the solvate, the metabolite, the isotopically labeled compound or the prodrug thereof of the present invention, or the pharmaceutical composition of the present invention in the preparation of a medicament for preventing or treating an HDAC6-related disease. Another aspect of the present invention provides the compound, or the pharmaceutically acceptable salt, the ester, the stereoisomer, the tautomer, the polymorph, the solvate, the metabolite, the isotopically labeled compound or the prodrug thereof of the present invention, or the pharmaceutical composition of the present invention, for use in the prevention or treatment of an HDAC6-related disease.

Another aspect of the present invention provides a method for preventing or treating an HDAC6-related disease, comprising administering to a subject in need thereof an effective amount of the compound, or the pharmaceutically acceptable salt, the ester, the stereoisomer, the tautomer, the polymorph, the solvate, the metabolite, the isotopically labeled compound or the prodrug thereof of the present invention, or the pharmaceutical composition of the present invention.

## DETAILED DESCRIPTION OF THE INVENTION

### Definitions

Unless otherwise defined below, all technical and scientific terms used herein are intended to have the same meaning as commonly understood by those of ordinary skill in the art. Reference to the techniques used herein is intended to refer to techniques commonly understood in the art, including those variations of or alternatives to techniques that would be apparent to those of ordinary skill in the art. Although the following terms are believed to be well understood by those skilled in the art, the following definitions are set forth to better explain the present invention.

The terms "include", "comprise", "have", "contain" or "involve", and other variant forms thereof herein, are inclusive or open-ended and do not exclude other unrecited elements or method steps.

As used herein, the term "alkylene" refers to saturated divalent hydrocarbyl, preferably saturated divalent hydrocarbyl having 1, 2, 3, 4, 5, or 6 carbon atoms, e.g., methylene, ethylene, propylene, or butylene.

As used herein, the term "alkyl" is defined as a linear or branched saturated aliphatic hydrocarbon. In some embodiments, the alkyl has 1-12, e.g., 1-6, carbon atoms. For example, as used herein, the term "$C_{1-6}$ alkyl" refers to a

linear or branched group of 1-6 carbon atoms (e.g., methyl, ethyl, *n*-propyl, isopropyl, *n*-butyl, isobutyl, *sec*-butyl, *tert*-butyl, *n*-pentyl, or *n*-hexyl) that is optionally substituted with 1 or more (such as 1-3) suitable substituents such as halogen (in which case the group is referred to as "haloalkyl") (e.g., $CF_3$, $C_2F_5$, $CHF_2$, $CH_2F$, $CH_2CF_3$, $CH_2Cl$, $-CH_2CH_2CF_3$, or the like). The term "$C_{1-4}$ alkyl" refers to a linear or branched aliphatic hydrocarbon chain of 1-4 carbon atoms (i.e., methyl, ethyl, *n*-propyl, isopropyl, *n*-butyl, isobutyl, *sec*-butyl, or *tert*-butyl).

As used herein, the term "alkenyl" refers to linear or branched monovalent hydrocarbyl containing one or more double bonds and having 2-6 carbon atoms ("$C_{2-6}$ alkenyl"). The alkenyl is, for example, $-CH=CH_2$, - $CH_2CH=CH_2$, $-C(CH_3)=CH_2$, $-CH_2-CH=CH-CH_3$, 2-pentenyl, 3-pentenyl, 4-pentenyl, 2-hexenyl, 3-hexenyl, 4-hexenyl, 5-hexenyl, 2-methyl-2-propenyl, or 4-methyl-3-pentenyl. When the compound of the present invention contains the alkenyl, the compound may be present in a pure E (entgegen) form, a pure Z (zusammen) form, or any mixture thereof. The term "alkenylene" is a corresponding divalent group, including, for example, "$C_{2-6}$ alkenylene", "$C_{2-4}$ alkenylene", and the like, specific examples of which include, but are not limited to: $-CH=CH-$, $-CH_2CH=CH-$, $-C(CH_3)=CH-$, butenylene, pentenylene, hexenylene, and the like.

As used herein, the term "alkynyl" refers to monovalent hydrocarbyl containing one or more triple bonds and preferably having 2, 3, 4, 5, or 6 carbon atoms, e.g., ethynyl, 2-propynyl, 2-butynyl, 1,3-butadiynyl, and the like. The alkynyl is optionally substituted with one or more (such as 1-3) identical or different substituents. The term "alkynylene" is a corresponding divalent group, including, for example, "$C_{2-8}$ alkynylene", "$C_{2-6}$ alkynylene", "$C_{2-4}$ alkynylene", and the like, examples of which include, but are not limited to,

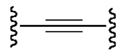,

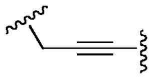, 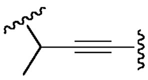, 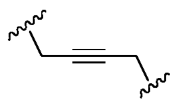,

and the like. The alkynylene is optionally substituted with one or more (such as 1-3) identical or different substituents.

As used herein, the terms "cyclohydrocarbylene", "cyclohydrocarbyl", and "hydrocarbon ring" refer to a saturated (i.e., "cycloalkylene" and "cycloalkyl") or unsaturated (i.e., having one or more double and/or triple bonds within the ring) monocyclic or polycyclic hydrocarbon ring having, for example, 3-10 (suitably 3-8, and more suitably 3-6) ring carbon atoms, including, but not limited to, cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclohexenyl(ene) (ring), and the like.

As used herein, the term "cycloalkyl" refers to a saturated monocyclic or polycyclic (such as bicyclic) hydrocarbon ring (e.g., monocyclic ring, such as cyclopropyl, cyclobutyl, cyclopentyl, cyclohexyl, cycloheptyl, cyclooctyl, or cyclononyl, or bicyclic ring, including spiro ring, fused ring, or bridged ring system (such as bicyclo[1.1.1]pentyl, bicyclo[2.2.1]heptyl, bicyclo[3.2.1]octyl, bicyclo[5.2.0]nonyl, or decahydronaphthyl) optionally substituted with 1 or more (such as 1-3) suitable substituents. The cycloalkyl has 3-15 carbon atoms. For example, the term "$C_{3-6}$ cycloalkyl" refers to a saturated monocyclic or polycyclic (such as bicyclic) hydrocarbon ring of 3-6 ring-forming carbon atoms (e.g., cyclopropyl, cyclobutyl, cyclopentyl, or cyclohexyl) optionally substituted with 1 or more (such as 1-3) suitable substituents, for example, cyclopropyl substituted with methyl.

As used herein, the term "heterocyclyl" refers to a saturated or unsaturated monovalent monocyclic or bicyclic group having 2, 3, 4, 5, 6, 7, 8, or 9 carbon atoms in the ring and one or more (e.g., one, two, three, or four) heteroatom-containing groups selected from O, S, S(=O), $S(=O)_2$, and $NR^a$, wherein $R^a$ represents a hydrogen atom or $C_{1-6}$ alkyl or halogenated $C_{1-6}$ alkyl; the heterocyclyl may be attached to the rest of the molecule through any of the carbon atoms or the nitrogen atom (if present). In particular, 3- to 10-membered heterocyclyl is a group having 3-10 carbon atoms and heteroatoms in the ring, for example, but not limited to, oxiranyl, aziridinyl, azetidinyl, oxetanyl, tetrahydrofuranyl, dioxolinyl, pyrrolidinyl, pyrrolidinonyl, imidazolidinyl, pyrazolidinyl, pyrrolinyl, tetrahydropyranyl, piperidinyl, morpholinyl, dithianyl, thiomorpholinyl, piperazinyl, or trithianyl.

As used herein, the term "heterocyclyl" encompasses fused ring structures, the point of attachment of which to other groups may be on any one of the fused ring structures. Thus, the heterocyclyl of the present invention also includes, but are not limited to, heterocyclyl-heterocyclyl, heterocyclyl-cycloalkyl, monoheterocyclyl-monoheterocyclyl, and monoheterocyclyl-monocycloalkyl, e.g., 3- to 7-membered (mono)heterocyclyl-3- to 7-membered (mono)heterocyclyl, 3- to 7-membered (mono)heterocyclyl-cycloalkyl, and 3- to 7-membered (mono)heterocyclyl-$C_{4-6}$ (mono)cycloalkyl, examples of which include, but are not limited to, pyrrolidinyl-cyclopropyl, cyclopentyl-aziridinyl, pyrrolidinyl-cyclobutyl, pyrrolidinyl-pyrrolidinyl, pyrrolidinyl-piperidinyl, pyrrolidinyl-piperazinyl, piperidinyl-morpholinyl,

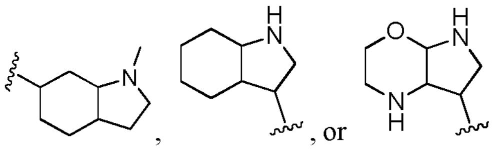, , or .

As used herein, the term "heterocyclyl" encompasses bridged heterocyclyl and spiro heterocyclyl.

As used herein, the term "bridged heterocyclic ring" refers to a cyclic structure containing one or more (e.g., 1, 2, 3, or 4) heteroatoms (e.g., oxygen atoms, nitrogen atoms, and/or sulfur atoms) formed by two saturated rings that share two ring atoms not directly connected, including, but not limited to, a 7- to 10-membered bridged heterocyclic ring, a 8- to 10-membered bridged heterocyclic ring, a 7- to 10-membered nitrogen-containing bridged heterocyclic ring, a 7- to 10-membered oxygen-containing bridged heterocyclic ring, a 7-to 10-membered sulfur-containing bridged heterocyclic ring, and the like, e.g.,

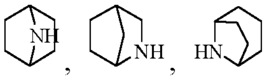, , ,

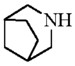, 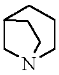, 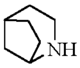, 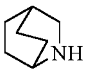, 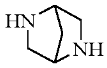, 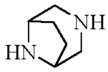, 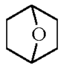, 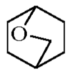, 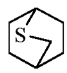, 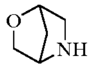, 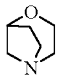, 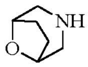,

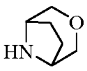, 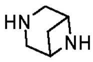, 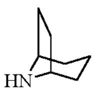, 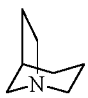, 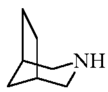, 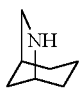, 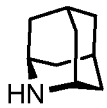,

and the like. The "nitrogen-containing bridged heterocyclic ring", "oxygen-containing bridged heterocyclic ring", or "sulfur-containing bridged heterocyclic ring" optionally further contains one or more other heteroatoms selected from oxygen, nitrogen, and sulfur.

As used herein, the term "spiro heterocyclic ring" refers to a cyclic structure containing one or more (e.g., 1, 2, 3, or 4) heteroatoms (e.g., oxygen atoms, nitrogen atoms, sulfur atoms) formed by two or more saturated rings that share one ring atom, including, but not limited to, a 5- to 10-membered spiro heterocyclic ring, a 6- to 10-membered spiro heterocyclic ring, a 6- to 10-membered nitrogen-containing spiro heterocyclic ring, a 6- to 10-membered oxygen-containing spiro heterocyclic ring, a 6- to 10-membered sulfur-containing spiro heterocyclic ring, and the like, e.g.,

, 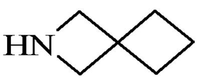, 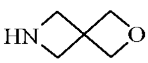, 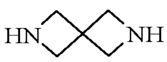, 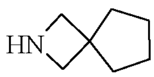,

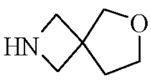, 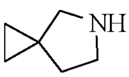, 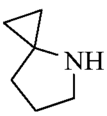, 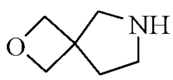, 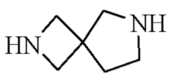, 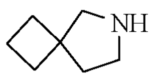, 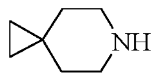,

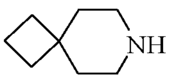, 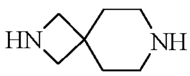, 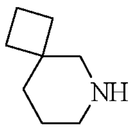, 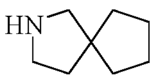, 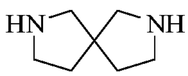, 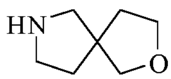,

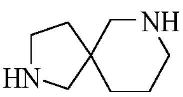, and 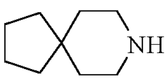.

The "nitrogen-containing spiro heterocyclic ring", "oxygen-containing spiro heterocyclic ring", or "sulfur-containing spiro heterocyclic ring" optionally further contains one or more other heteroatoms selected from oxygen, nitrogen, and sulfur.

The term "6- to 10-membered nitrogen-containing spiro heterocyclyl" refers to spiro heterocyclyl containing a total of 6-10 ring atoms, at least one of which is a nitrogen atom.

As used herein, the term "aryl" refers to an all-carbon monocyclic or fused polycyclic aromatic group having a conjugated $\pi$-electron system. For example, as used herein, the term "$C_{6-14}$ aryl" refers to an aromatic group containing 6-14 carbon atoms, such as phenyl or naphthyl. The aryl is optionally substituted with 1 or more (such as 1-3) suitable substituents (e.g., halogen, -OH, -CN, $-NO_2$, $C_{1-6}$ alkyl, and the like).

The term "aralkyl" preferably refers to alkyl substituted with aryl, wherein the aryl and the alkyl are as defined herein. Generally, the aryl may have 6-14 carbon atoms and the alkyl may have 1-6 carbon atoms. Exemplary aralkyl includes, but is not limited to, benzyl, phenylethyl, phenylpropyl, and phenylbutyl.

As used herein, the term "heteroaryl" or "heteroaromatic ring" refers to a monocyclic or polycyclic aromatic group containing one or more identical or different heteroatoms, including monocyclic heteroaryl and a bicyclic or polycyclic ring system containing at least one heteroaromatic ring (an aromatic ring system containing at least one heteroatom), which may have 5, 6, 7, 8, 9, 10, 11, 12, 13, or 14 ring atoms, e.g., 5, 6, 7, 8, 9, or 10 ring atoms. The heteroatoms may be oxygen, nitrogen, or sulfur. The carbon atoms and heteroatoms on the heteroaryl are optionally substituted with oxo groups (e.g., to form C=O, S(=O), or $S(=O)_2$).

As used herein, the term "5- to 10-membered heteroaryl" or "5- to 10-membered heteroaromatic ring" refers to heteroaryl (heteroaromatic ring) containing 5-10 (e.g., 5-6) ring atoms, including 5- to 10-membered nitrogen-containing heteroaryl, 5- to 10-membered oxygen-containing heteroaryl, 5- to 10-membered sulfur-containing heteroaryl, 5- to 6-membered nitrogen-containing heteroaryl, 5- to 6-membered oxygen-containing heteroaryl, 5- to 6-membered sulfur-containing heteroaryl, and the like. The "nitrogen-containing heteroaryl", "oxygen-containing heteroaryl", and "sulfur-containing heteroaryl" each optionally contain one or more other heteroatoms selected from oxygen, nitrogen, and sulfur, examples of which include, but are not limited to, thienyl, furanyl, pyrrolyl, oxazolyl, thiazolyl, imidazolyl, pyrazolyl, isoxazolyl, isothiazolyl, triazolyl, tetrazolyl, oxadiazolyl, thiadiazolyl, and the like, or pyridyl, pyridazinyl, pyrimidinyl, pyrazinyl, triazinyl, and the like, and 5- to 10-membered fused ring groups containing these groups.

As used herein, the term "heteroaryl" encompasses fused ring structures, the point of attachment of which to other groups may be on any one of the fused ring structures. Thus, the heteroaryl of the present invention also includes, but is not limited to, (mono)heteroaryl-(mono)heteroaryl, (mono)heteroaryl-(monocyclic) aryl, (mono)heteroaryl-(mono)heterocyclyl, and (mono)heteroaryl-(mono)cycloalkyl, e.g., 5- to 6-membered (mono)heteroaryl-5- to 6-membered (mono) heteroaryl, 5- to 6-membered (mono)heteroaryl-phenyl, 5- to 6-membered (mono)heteroaryl-5- to 6-membered (mono) heterocyclyl, or 5- to 6-membered (mono)heteroaryl-$C_{4-6}$ (mono)cycloalkyl (e.g., 5- to 6-membered heteroaryl-cyclobutyl, 5- to 6-membered heteroaryl-cyclopentyl, or 5- to 6-membered heteroaryl-cyclohexyl), examples of which include, but are not limited to, indolyl, isoindolyl, indazolyl, benzimidazole, quinolyl, isoquinolyl,

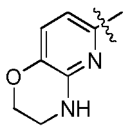, 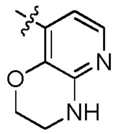, 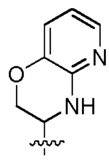,

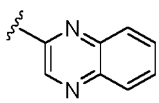, 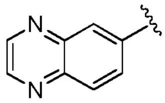, 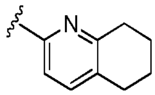, 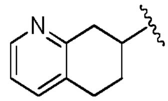, 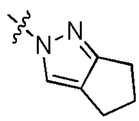, 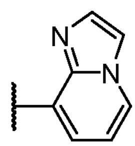,

and the like.

As used herein, the term "halo" or "halogen" group is defined to include F, Cl, Br, or I.

As used herein, the term "alkylthio" refers to alkyl as defined above that is attached to the parent molecular moiety through a sulfur atom. Representative examples of $C_{1-6}$ alkylthio include, but are not limited to, methylthio, ethylthio, *tert*-butylthio, and hexylthio.

As used herein, the term "nitrogen-containing heterocyclic ring" refers to a saturated or unsaturated monocyclic or bicyclic group having 2, 3, 4, 5, 6, 7, 8, 9, 10, 11, 12, or 13 carbon atoms and at least one nitrogen atom in the ring, which may also optionally contain one or more (e.g., one, two, three, or four) ring members selected from N, O, C=O, S, S=O, and $S(=O)_2$. The nitrogen-containing heterocyclic ring is attached to the rest of the molecule through a nitrogen atom. The nitrogen-containing heterocyclic is preferably a saturated nitrogen-containing monocyclic ring. In particular, a 3- to 14-membered nitrogen-containing heterocyclic ring is a group having 3-14 carbon atoms and heteroatoms (at least one of which is a nitrogen atom) in the ring, including, but not limited to, a three-membered nitrogen-containing heterocyclic ring

(e.g., aziridinyl), a four-membered nitrogen-containing heterocyclic ring (e.g., azetidinyl), a five-membered nitrogen-containing heterocyclic ring (e.g., pyrrolyl, pyrrolidinyl (pyrrolidine ring), pyrrolinyl, pyrrolidinonyl, imidazolyl, imidazolidinyl, imidazolinyl, pyrazolyl, or pyrazolinyl), a six-membered nitrogen-containing heterocyclic ring (e.g., piperidinyl (piperidine ring), morpholinyl, thiomorpholinyl, or piperazinyl), a seven-membered nitrogen-containing heterocyclic ring, and the like.

The term "substitution" means that one or more (e.g., one, two, three, or four) hydrogen atoms on a specified atom are replaced with a selection from the designated group, with the proviso that the normal valency of the atom specified under the present circumstances is not exceeded and that the replacement results in a stable compound. A combination of substituents and/or variables is permissible only if the combination results in a stable compound.

If a substituent is described as "optionally substituted with...", the substituent may be (1) unsubstituted or (2) substituted. If a carbon of a substituent is described as being optionally substituted with one or more of a list of substituents, then one or more hydrogen atoms on the carbon (to the extent of any hydrogen atoms present) may be replaced individually and/or together with an independently selected optional substituent. If a nitrogen of a substituent is described as being optionally substituted with one or more of a list of substituents, then one or more hydrogen atoms on the nitrogen (to the extent any hydrogen atoms present) may each be replaced with an independently selected optional substituent.

If a substituent is described as being "independently selected from" one group, each substituent is selected independently of the other. Thus, each substituent may be identical to or different from another (other) substituent(s).

As used herein, the term "one or more" refers to 1 or more than 1, such as 2, 3, 4, 5, or 10, under reasonable conditions.

Unless otherwise indicated, as used herein, the point of attachment of a substituent may be from any suitable position of the substituent.

When a bond of a substituent is shown to pass through a bond connecting two atoms in the ring, the substituent may be bonded to any one of the ring-forming atoms in the substitutable ring.

The present invention also includes all pharmaceutically acceptable isotopically-labeled compounds, which are identical to the compounds of the present invention, except that one or more atoms are replaced by an atom having the same atomic number, but an atomic mass or mass number different from the atomic mass or mass number predominant in nature. Examples of isotopes suitable for incorporation into the compounds of the present invention include, but are not limited to, isotopes of hydrogen (e.g., deuterium (D, $^{2}H$), tritium (T, $^{3}H$)); isotopes of carbon (e.g., $^{11}C$, $^{13}C$, and $^{14}C$); isotopes of chlorine (e.g., $^{36}Cl$); isotopes of fluorine (e.g., $^{18}F$); isotopes of iodine (e.g., $^{123}I$ and $^{125}I$); isotopes of nitrogen (e.g., $^{13}N$ and $^{15}N$); isotopes of oxygen (e.g., $^{15}O$, $^{17}O$, and $^{18}O$); isotopes of phosphorus (e.g., $^{32}P$); and isotopes of sulfur (e.g., $^{35}S$). Certain isotopically-labeled compounds of the present invention (e.g., those into which a radioactive isotope is incorporated) can be used in drug and/or substrate tissue distribution studies (e.g., assays). The radioactive isotopes tritium (i.e., $^{3}H$) and carbon-14 (i.e., $^{14}C$) are particularly useful for this purpose because of their ease of incorporation and detection. Substitution with positron emitting isotopes (e.g., $^{11}C$, $^{18}F$, $^{15}O$, and $^{13}N$) can be used to examine substrate receptor occupancy in positron emission tomography (PET) studies. The isotopically labeled compounds of the present invention can be prepared by processes analogous to those described in the accompanying routes and/or in the examples and preparations by using an appropriate isotopically labeled reagent in place of the non-labeled reagent previously employed. The pharmaceutically acceptable solvates of the present invention include those in which the crystallization solvent may be isotopically substituted, e.g., $D_2O$, acetone-$d_6$, or DMSO-$d_6$.

It should be understood that certain compounds of the present invention may be present in free form for use in therapy or, where appropriate, in the form of a pharmaceutically acceptable derivative thereof. In the present invention, the pharmaceutically acceptable derivatives include, but are not limited to, pharmaceutically acceptable salts, esters, solvates, metabolites or prodrugs, which upon administration to a patient in need thereof are capable of providing, directly or indirectly, the compound of the present invention or a metabolite or residue thereof. Thus, when reference is made herein to "the compound of the present invention", it is also intended to encompass the various derivative forms of the compound described above. The pharmaceutically acceptable salts of the compounds of the present invention include acid addition salts and base addition salts thereof.

A review of suitable salts is found in Stahl and Wermuth, "Handbook of Pharmaceutical Salts: Properties, Selection, and Use" (Wiley-VCH, 2002). Methods for preparing the pharmaceutically acceptable salts of the compounds of the present invention are known to those skilled in the art.

As used herein, the term "ester" refers to an ester derived from the compounds of formulas in the present application, including physiologically hydrolyzable esters (which can be hydrolyzed under physiological conditions to release the compounds of the present invention in the form of free acids or alcohols). The compounds of the present invention may themselves also be esters.

The compounds of the present invention may be present in the form of solvates (preferably hydrates), and the compounds of the present invention contain a polar solvent as a structural element of the crystal lattice of the compound, particularly, for example, water, methanol, or ethanol. The amount of the polar solvent, especially water, may be present in a stoichiometric or non-stoichiometric ratio.

Also included within the scope of the present invention are metabolites of the compounds of the present invention, i.e., substances formed *in vivo* upon administration of the compounds of the present invention. Such products may result, for example, from oxidation, reduction, hydrolysis, amidation, deamidation, esterification, defatting, enzymatic digestion, and the like of the administered compound. Thus, the present invention includes metabolites of the compounds of the present invention, including compounds made by the process of contacting the compounds of the present invention with a mammal for a time sufficient to produce metabolites thereof.

Also included within the scope of the present invention are prodrugs of the compounds of the present invention, which are certain derivatives of the compounds of the present invention that may themselves have little or no pharmacological activity and, when administered into or onto the body, are converted to the compounds of the present invention having the desired activity, for example by hydrolysis. Generally, such prodrugs will be functional derivatives of the compounds, which are readily converted into desired therapeutically active compounds *in vivo.* Additional information on the use of prodrugs can be found in "Pro-drugs as Novel Delivery Systems", volume 14, ACS Symposium Series (T. Higuchi and V. Stella) and "Bioreversible Carriers in Drug Design", Pergamon Press, 1987 (E. B. Roche eds., American Pharmaceutical Association). The prodrugs of the present invention may be prepared, for example, by replacing appropriate functional groups present in the compounds of the present invention with certain moieties known to those skilled in the art as "pro-moieties", for example as described in "Design of Prodrugs", H. Bundgaard (Elsevier, 1985).

The present invention further encompasses the compounds of the present invention containing a protective group. In any process of preparing the compounds of the present invention, it may be necessary and/or desirable to protect sensitive or reactive groups on any of the molecules concerned, thereby forming a chemically protected form of the compounds of the present invention. This can be achieved by conventional protective groups, as described, for example, in Protective Groups in Organic Chemistry, ed. J.F.W McOmie, Plenum Press, 1973; and T.W. Greene & P.G.M. Wuts, Protective Groups in Organic Synthesis, John Wiley & Sons, 1991, which are incorporated herein by reference. The protective groups may be removed at an appropriate subsequent stage by methods known in the art.

As used herein, the term "about" means within ± 10%, preferably within ± 5%, and more preferably within ± 2% of the stated numerical value.

<u>**Compound**</u>

In some embodiments, the present invention provides a compound, or a pharmaceutically acceptable salt, an ester, a stereoisomer, a tautomer, a polymorph, a solvate, a metabolite, am isotopically labeled compound or a prodrug thereof, wherein the compound has a structure of formula (I):

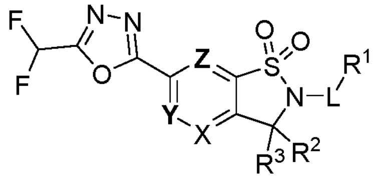

**(I)**

wherein

L is selected from a direct bond, $-C_{1-6}$ alkylene-, $-C_{2-6}$ alkenylene-, and $-C_{2-6}$ alkynylene-;
X is $CR^6$ or N;
Y is $CR^4$ or N;
Z is $CR^5$ or N;
$R^1$ is selected from H, halogen, -OH, $-NH_2$, -CN, $-NO_2$, $C_{1-6}$ alkyl, $C_{2-6}$ alkenyl, $C_{2-6}$ alkynyl, $C_{3-6}$ cyclohydrocarbyl, 3- to 10-membered heterocyclyl, $C_{6-10}$ aryl, 5- to 14-membered heteroaryl, $C_{6-12}$ aralkyl, - $C(=O)R^a$, $-OC(=O)R^a$, $-OC(=O)NR^aR^b$, $-C(=O)OR^a$, $-OR^a$, $-SR^a$, $-S(=O)R^a$, $-S(=O)_2R^a$, $-S(=O)_2NR^aR^b$, - $NR^aR^b$, $-C(=O)NR^aR^b$, $-NR^a-C(=O)R^b$, $-NR^a-C(=O)OR^b$, $-NR^a-S(=O)_2-R^b$, $-NR^a-C(=O)-NR^aR^b$, $-C_{1-6}$ alkylene-$OR^a$, $-C_{1-6}$ alkylene-$NR^aR^b$, and $-O-C_{1-6}$ alkylene-$NR^aR^b$;
$R^2$ and $R^3$ are each independently selected from H, halogen, -OH, $-NH_2$, -CN, $-NO_2$, $C_{1-6}$ alkyl, $C_{2-6}$ alkenyl, $C_{2-6}$ alkynyl, $C_{3-6}$ cyclohydrocarbyl, 3- to 10-membered heterocyclyl, $C_{6-10}$ aryl, 5- to 14-membered heteroaryl, $C_{6-12}$ aralkyl, $-C(=O)R^a$, $-OC(=O)R^a$, $-OC(=O)NR^aR^b$, $-C(=O)OR^a$, $-OR^a$, $-SR^a$, $-S(=O)R^a$,$-S(=O)_2R^a$, $-S(=O)_2NR^aR^b$, $-NR^aR^b$, $-C(=O)NR^aR^b$, $-NR^a-C(=O)R^b$, $-NR^a-C(=O)OR^b$, $-NR^a-S(=O)_2-R^b$, $-NR^a-C(=O)-NR^aR^b$, $-C_{1-6}$ alkylene-$OR^a$, $-C_{1-6}$ alkylene-$NR^aR^b$, and $-O-C_{1-6}$ alkylene-$NR^aR^b$; or $R^2$ and $R^3$ together form oxo (=O); or $R^2$ and $R^3$, together with the carbon atom to which they are attached, form $C_{3-6}$ cyclohydrocarbyl or 3- to 10-membered heterocyclyl;

$R^4$, $R^5$, and $R^6$ are each independently selected from H, halogen, -OH, $-NH_2$, -CN, $-NO_2$, $C_{1-6}$ alkyl, $C_{2-6}$ alkenyl, $C_{2-6}$ alkynyl, $C_{3-6}$ cyclohydrocarbyl, 3- to 10-membered heterocyclyl, $C_{6-10}$ aryl, 5- to 14-membered heteroaryl, $C_{6-12}$ aralkyl, $-C(=O)R^a$, $-OC(=O)R^a$, $-OC(=O)NR^aR^b$, $-C(=O)OR^a$, $-OR^a$, $-SR^a$, $-S(=O)R^a$,$-S(=O)_2R^a$, $-S(=O)_2NR^aR^b$, $-NR^aR^b$, $-C(=O)NR^aR^b$, $-NR^a-C(=O)R^b$, $-NR^a-C(=O)OR^b$, $-NR^a-S(=O)_2-R^b$, $-NR^a-C(=O)-NR^aR^b$, $-C_{1-6}$ alkylene-$OR^a$, $-C_{1-6}$ alkylene-$NR^aR^b$, and $-O-C_{1-6}$ alkylene-$NR^aR^b$;

$R^a$ and $R^b$, at each occurrence, are each independently selected from H, $C_{1-6}$ alkyl, $C_{3-10}$ cyclohydrocarbyl, 3-to 10-membered heterocyclyl, $C_{6-10}$ aryl, 5- to 14-membered heteroaryl, and $C_{6-12}$ aralkyl;

the alkyl, alkylene, alkenyl, alkenylene, alkynyl, alkynylene, cyclohydrocarbyl, heterocyclyl, aryl, heteroaryl, and aralkyl, at each occurrence, are each optionally substituted with one or more substituents independently selected from: halogen, -OH, =O , $-NH_2$, -CN, $-NO_2$, $C_{1-6}$ alkyl, $C_{3-6}$ cyclohydrocarbyl, 3- to 10-membered heterocyclyl, $C_{6-10}$ aryl, 5- to 14-membered heteroaryl, $C_{6-12}$ aralkyl, $-C(=O)R^c$, $-OC(=O)R^c$, $-OC(=O)NR^cR^d$, $-C(=O)OR^c$, $-OR^c$, $-SR^c$, $-S(=O)R^c$, $-S(=O)_2R^c$, $-S(=O)_2NR^cR^d$, $-NR^cR^d$, $-C(=O)NR^cR^d$, $-NR^c-C(=O)R^d$, $- NR^c-C(=O)OR^d$, $-NR^c-S(=O)_2-R^d$, $-NR^c-C(=O)-NR^cR^d$, $-C_{1-6}$ alkylene-$OR^c$, $-C_{1-6}$ alkylene-$NR^cR^d$, and $-O-C_{1-6}$ alkylene-$NR^cR^d$; the alkyl, cyclohydrocarbyl, heterocyclyl, aryl, heteroaryl, and aralkyl are further optionally substituted with one or more substituents independently selected from: halogen, -OH, =O, - C(=O)O-tert-butyl, $-NH_2$, -CN, $-NO_2$, $C_{1-6}$ alkyl, halogenated $C_{1-6}$ alkyl, $-OC_{1-6}$ alkyl, -O-halogenated $C_{1-6}$ alkyl, $C_{3-6}$ cyclohydrocarbyl, 3- to 10-membered heterocyclyl, $C_{6-10}$ aryl, 5- to 14-membered heteroaryl, and $C_{6-12}$ aralkyl;

$R^c$ and $R^d$, at each occurrence, are each independently selected from H, $C_{1-6}$ alkyl, $C_{3-10}$ cyclohydrocarbyl, 3-to 10-membered heterocyclyl, $C_{6-10}$ aryl, 5- to 14-membered heteroaryl, and $C_{6-12}$ aralkyl; the alkyl, cyclohydrocarbyl, heterocyclyl, aryl, heteroaryl, and aralkyl are further optionally substituted with one or more substituents independently selected from: halogen, -OH, =O, -C(=O)O-tert-butyl, $-NH_2$, -CN, $-NO_2$, $C_{1-6}$ alkyl, halogenated $C_{1-6}$ alkyl, $C_{3-6}$ cyclohydrocarbyl, 3- to 10-membered heterocyclyl, $C_{6-10}$ aryl, 5- to 14-membered heteroaryl, and $C_{6-12}$ aralkyl.

In a preferred embodiment, the present invention provides a compound, or a pharmaceutically acceptable salt, an ester, a stereoisomer, a tautomer, a polymorph, a solvate, a metabolite, an isotopically labeled compound or a prodrug thereof, wherein the compound has a structure of formula (II) or formula (III):

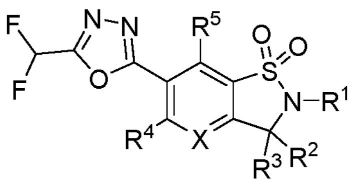

(II)

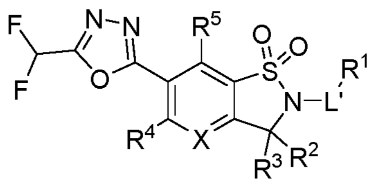

(III)

wherein:

L' is $-C_{1-6}$ alkylene-, wherein the alkylene is optionally substituted with one or more substituents independently selected from: -OH, $C_{3-6}$ cyclohydrocarbyl, 3- to 10-membered heterocyclyl, $C_{6-10}$ aryl, and 5-to 14-membered heteroaryl; preferably, the alkylene is optionally substituted with one or more substituents independently selected from: -OH, cyclopropyl, and phenyl optionally substituted with one or more halogen;

the other groups are as defined herein.

In some embodiments, the present invention provides a compound of formula (I), formula (II), or formula (III), or a pharmaceutically acceptable salt, an ester, a stereoisomer, a tautomer, a polymorph, a solvate, a metabolite, an isotopically labeled compound or a prodrug thereof, wherein L is a direct bond or $-C_{1-6}$ alkylene-, wherein the alkylene is optionally substituted with one or more substituents independently selected from: -OH, $-OCH_3$, $C_{1-6}$ alkyl, $C_{3-6}$ cyclohydrocarbyl, 3- to 10-membered heterocyclyl, $C_{6-10}$ aryl, and 5- to 14-membered heteroaryl; the alkyl, cyclohydrocarbyl, heterocyclyl, aryl, and heteroaryl are further optionally substituted with one or more halogen or $C_{1-6}$ alkyl.

In a preferred embodiment, L is a direct bond, methylene, or ethylene, wherein the methylene and ethylene are optionally substituted with one or more substituents independently selected from: -OH, $-OCH_3$, methyl, cyclopropyl, phenyl, pyrazolyl, pyridinyl, pyrimidinyl, and pyridazinyl; the groups described above are optionally further substituted with one or more halogen and/or methyl.

In a most preferred embodiment, L is a direct bond, $-CH_2-$,

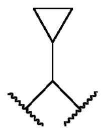, 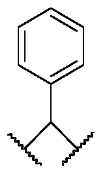,

$-CH_2CH_2-$.

, 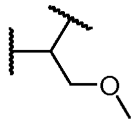,

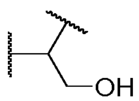, 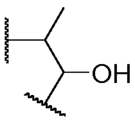, 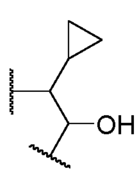, 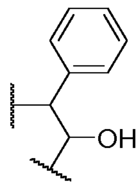, 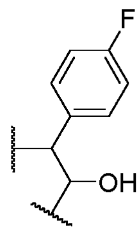, 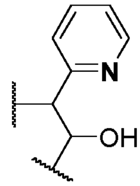, 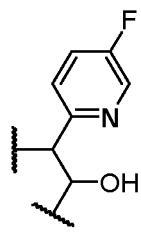,

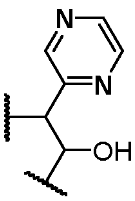, 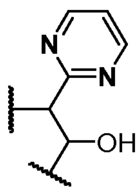, or 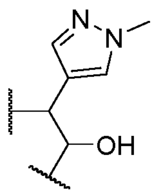.

In some embodiments, the present invention provides a compound of formula (I), formula (II), or formula (III), or a pharmaceutically acceptable salt, an ester, a stereoisomer, a tautomer, a polymorph, a solvate, a metabolite, an isotopically labeled compound or a prodrug thereof, wherein X, Y, and Z are each independently CH, CF, or N.

In some embodiments, the present invention provides a compound of formula (I), formula (II), or formula (III), or a pharmaceutically acceptable salt, an ester, a stereoisomer, a tautomer, a polymorph, a solvate, a metabolite, an isotopically labeled compound or a prodrug thereof, wherein $R^1$ is selected from $C_{1-6}$ alkyl, $C_{3-6}$ cyclohydrocarbyl, 3- to 10-membered heterocyclyl, $C_{6-10}$ aryl, and 5- to 14-membered heteroaryl;

the alkyl, cyclohydrocarbyl, heterocyclyl, aryl, and heteroaryl are each optionally substituted with one or more substituents independently selected from: halogen, -OH, -CN, $C_{1-6}$ alkyl, $C_{3-6}$ cyclohydrocarbyl, 3- to 10-membered heterocyclyl, $C_{6-10}$ aryl, 5- to 14-membered heteroaryl, $-C(=O)R^c$, $-OC(=O)R^c$, $-C(=O)OR^c$, - $OR^c$, $-S(=O)_2R^c$, $-NR^cR^d$, $-C(=O)NR^cR^d$, $-NR^c-C(=O)R^d$, $-NR^c-C(=O)OR^d$, $-NR^c-S(=O)_2-R^d$, and $-NR^c-C(=O)-NR^cR^d$; the alkyl, cyclohydrocarbyl, heterocyclyl, aryl, and heteroaryl described above are further optionally substituted with one or more substituents independently selected from: halogen (e.g., fluoro), - OH, =O, $C_{1-6}$ alkyl (e.g., methyl), halogenated $C_{1-6}$ alkyl (e.g., trifluoromethyl), $-OC_{1-6}$ alkyl (e.g., methoxy), and -O-halogenated $C_{1-6}$ alkyl (e.g., trifluoromethoxy).

In some embodiments, the present invention provides a compound of formula (I), formula (II), or formula (III), or a pharmaceutically acceptable salt, an ester, a stereoisomer, a tautomer, a polymorph, a solvate, a metabolite, an isotopically labeled compound or a prodrug thereof, wherein $R^c$ and $R^d$, at each occurrence, are each independently selected from H, $C_{1-6}$ alkyl (e.g., methyl, ethyl, or *tert-butyl),* $C_{3-10}$ cyclohydrocarbyl (e.g., cyclopropyl), 3- to 10-membered heterocyclyl (e.g., pyrrolidinyl, morpholinyl, or piperidinyl optionally substituted with F), $C_{6-10}$ aryl (phenyl optionally substituted with F), and 5- to 14-membered heteroaryl (e.g., pyridinyl); the alkyl, cyclohydrocarbyl, heterocyclyl, aryl, and heteroaryl are further optionally substituted with one or more substituents independently selected from: halogen (e.g., F), -OH, halogenated $C_{1-6}$ alkyl (e.g., trifluoromethyl), and $C_{3-6}$ cyclohydrocarbyl (e.g., cyclopropyl).

In some embodiments, the present invention provides a compound of formula (I), formula (II), or formula (III), or a pharmaceutically acceptable salt, an ester, a stereoisomer, a tautomer, a polymorph, a solvate, a metabolite, an isotopically labeled compound or a prodrug thereof, wherein $R^1$ is selected from methyl, cyclopropyl, cyclohexyl, tetrahydropyrrolyl, tetrahydropyranyl, piperidinyl, morpholinyl, phenyl, pyrazolyl, pyridinyl, pyrimidinyl, pyridazinyl, and imidazopyridinyl; the groups are each optionally substituted with one or more substituents independently selected from: -F,

-Cl, -OH, -CN, $-NH_2$, $-CH_3$, $-CF_3$, $-CH_2CF_2CF_3$, - $NHCH_2CF_3$,

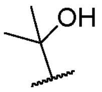 , 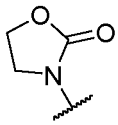 , 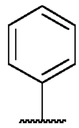 , 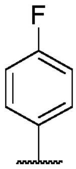 , 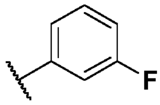 , 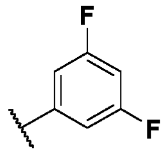 , 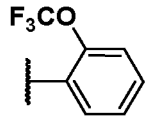 ,

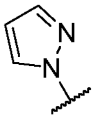 , 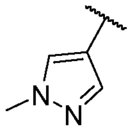 , 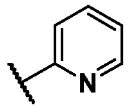 , 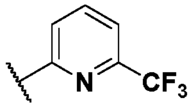 , 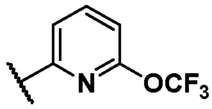 , 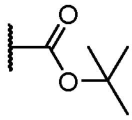 , 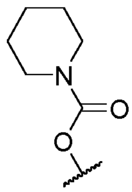 ,

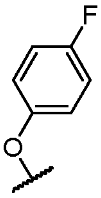 , 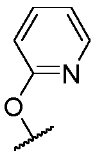 , 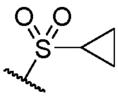 , 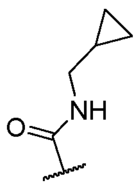 , 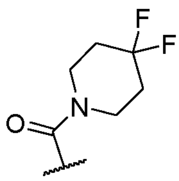 , 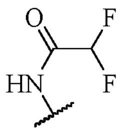 , 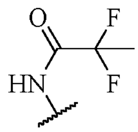 , 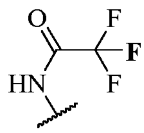 ,

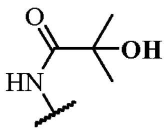 , 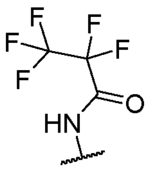 , 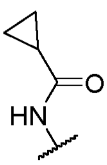 , 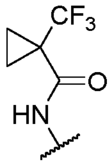 , 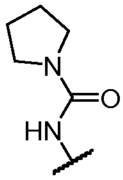 , 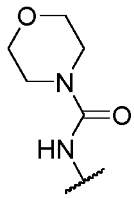 , 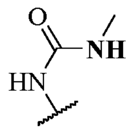 ,

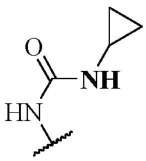 , 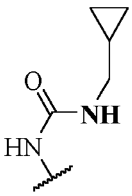 , 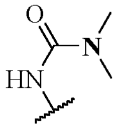 , 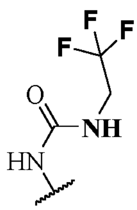 , 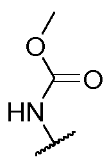 , 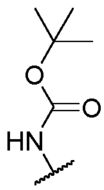 , 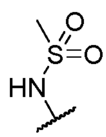 , 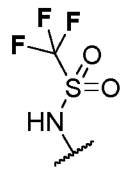 ,

and

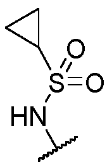 .

In some embodiments, the present invention provides a compound of formula (I), formula (II), or formula (III), or a pharmaceutically acceptable salt, an ester, a stereoisomer, a tautomer, a polymorph, a solvate, a metabolite, an isotopically labeled compound or a prodrug thereof, wherein $R^1$ has the following structure:

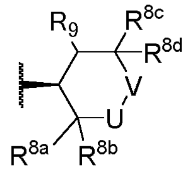

wherein:

U and V are each independently $CR^{8e}R^{8f}$, $NR^{8g}$, or O;

$R^{8a}$, $R^{8b}$, $R^{8c}$, $R^{8d}$, $R^{8e}$, $R^{8f}$, and $R^{8g}$ are each independently H or halogen (e.g., F);

$R^9$ is $-OR^c$, $-NR^c-C(=O)R^d$, $-NR^cR^d$, $-NR^c-C(=O)OR^d$, $-NR^c-S(=O)_2-R^d$, $-NR^c-C(=O)-NR^cR^d$, or $-OC(=O)R^c$.

In some embodiments, the present invention provides a compound of formula (I), formula (II), or formula (III), or a pharmaceutically acceptable salt, an ester, a stereoisomer, a tautomer, a polymorph, a solvate, a metabolite, an isotopically labeled compound, or a prodrug thereof, wherein $R^1$ is selected from methyl,

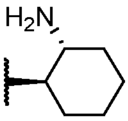 , 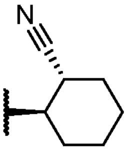 , 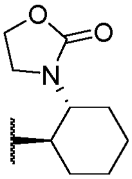 , 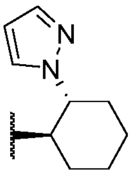 , 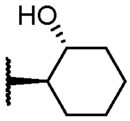 , 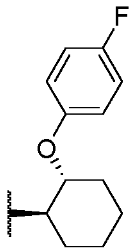 , 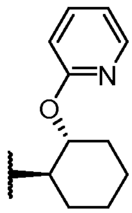 ,

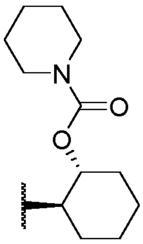 , 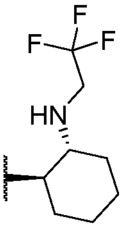 , 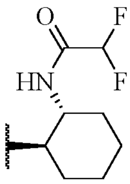 , 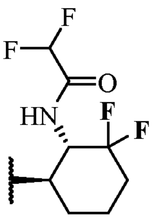 , 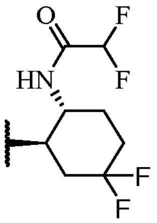 , 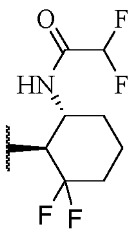 , 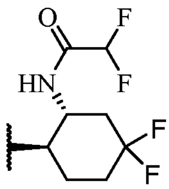 ,

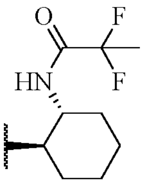 , 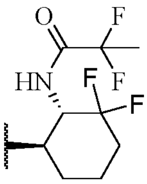 , 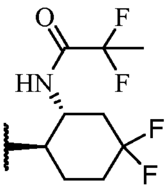 , 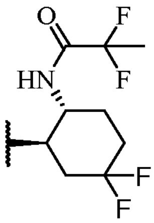 , 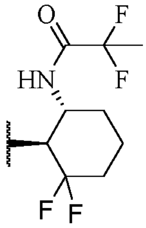 , 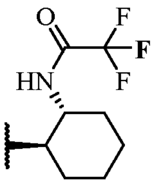 ,

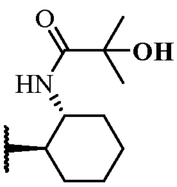 , 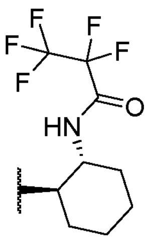 , 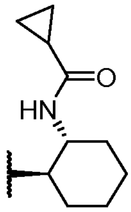 , 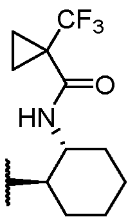 , 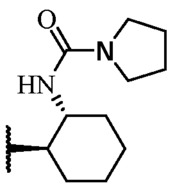 , 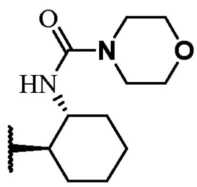 ,

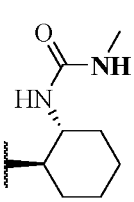, 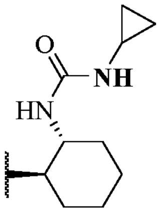, 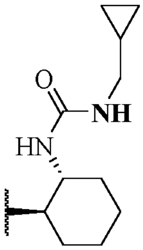, 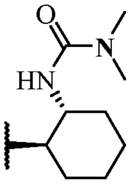, 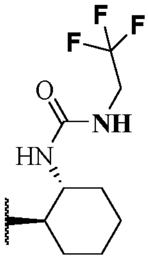, 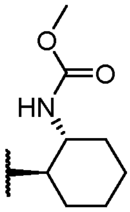, 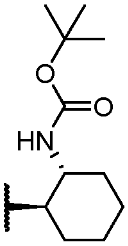,

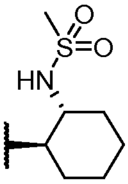, 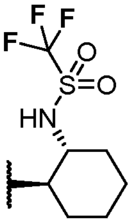, 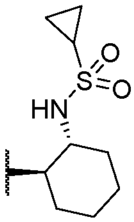, 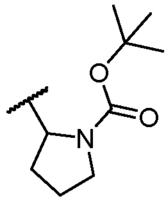, 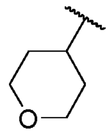, 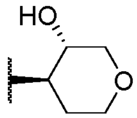, 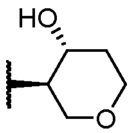,

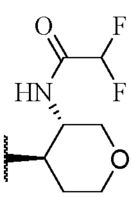, 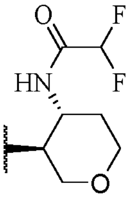, 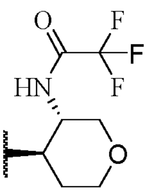, 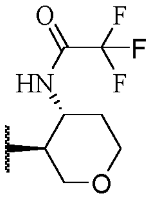, 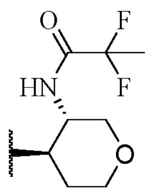, 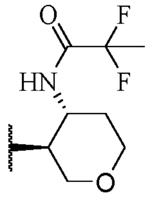, 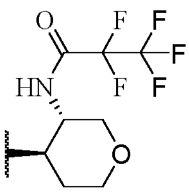,

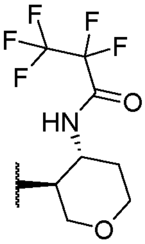, 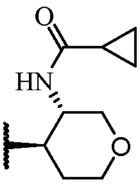, 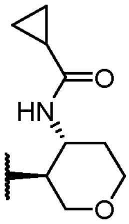, 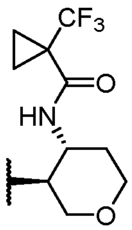, 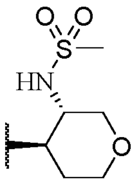, 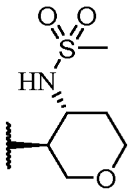, 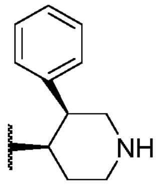,

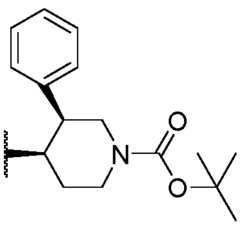, 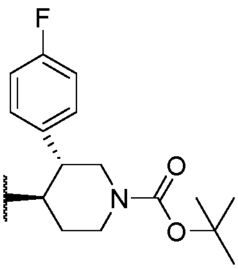, 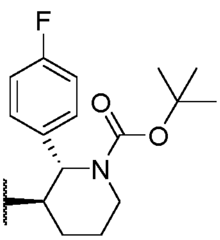, 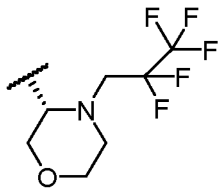, 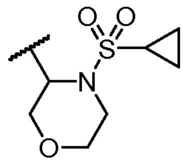,

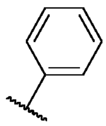, 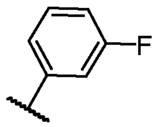, 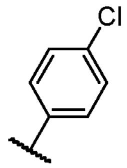, 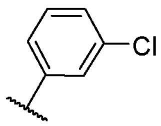, 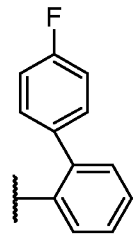, 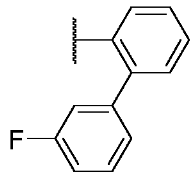, 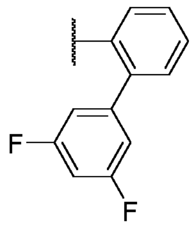,

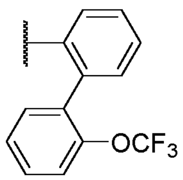, 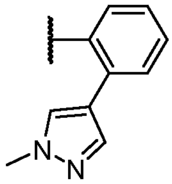, 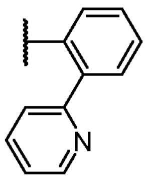, 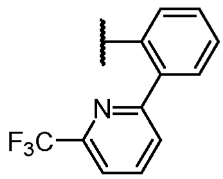, 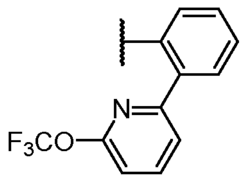, 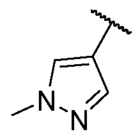,

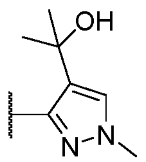, 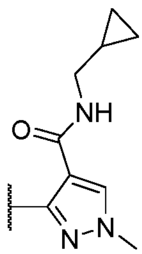, 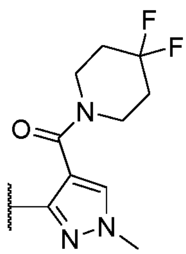, 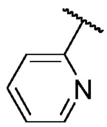, 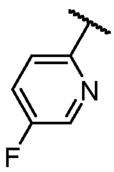, 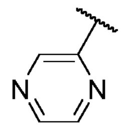, 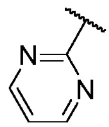,

and

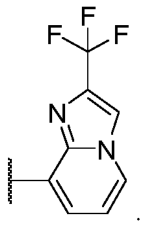.

In some embodiments, the present invention provides a compound of formula (I), formula (II), or formula (III), or a pharmaceutically acceptable salt, an ester, a stereoisomer, a tautomer, a polymorph, a solvate, a metabolite, an isotopically labeled compound or a prodrug thereof, wherein $R^2$ and $R^3$ are H or $C_{1-6}$ alkyl, preferably H or methyl; or $R^2$ and $R^3$ together form oxo (=O); or $R^2$ and $R^3$, together with the carbon atom to which they are attached, form $C_{3-6}$ cyclohydrocarbyl, preferably cyclopropyl.

In some embodiments, the present invention provides a compound of formula (I), formula (II), or formula (III), or a pharmaceutically acceptable salt, an ester, a stereoisomer, a tautomer, a polymorph, a solvate, a metabolite, an isotopically labeled compound or a prodrug thereof, wherein $R^4$, $R^5$, and $R^6$ are each independently H or halogen; preferably, $R^4$, $R^5$, and $R^6$ are each independently H or F

The present invention encompasses the compounds resulting from any combination of the various embodiments.

In a preferred embodiment, the present invention provides a compound, or a pharmaceutically acceptable salt, an ester, a stereoisomer, a tautomer, a polymorph, a solvate, a metabolite, an isotopically labeled compound or a prodrug thereof, wherein the compound is selected from:

| No. | Structure |
|---|---|
| 1. | 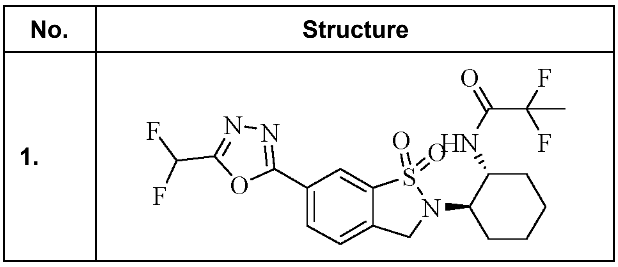 |

(continued)

| No. | Structure |
|---|---|
| 2. | 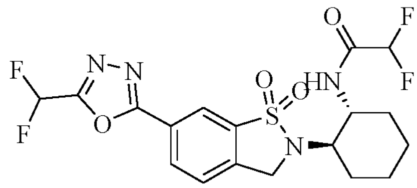 |
| 3. | 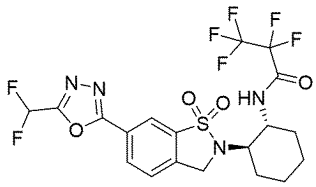 |
| 4. | 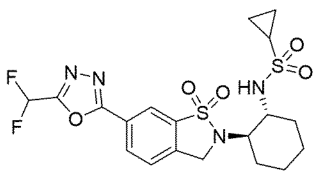 |
| 5. | 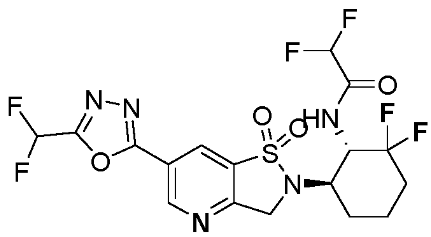 |
| 6. | 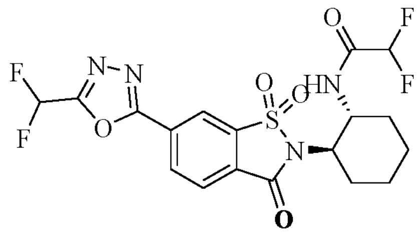 |
| 7. | 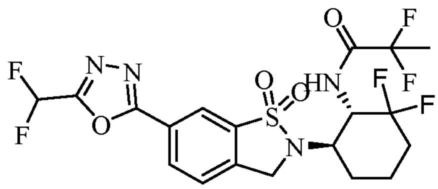 |
| 8. | 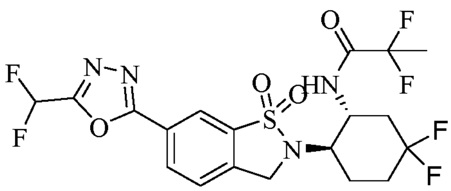 |

(continued)

| No. | Structure |
|---|---|
| 9. | 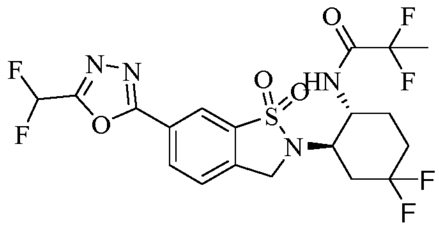 |
| 10. | 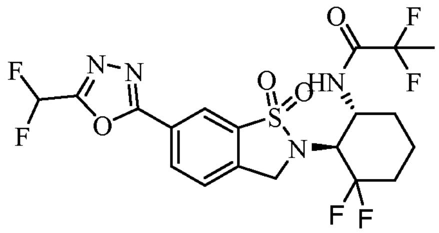 |
| 11. | 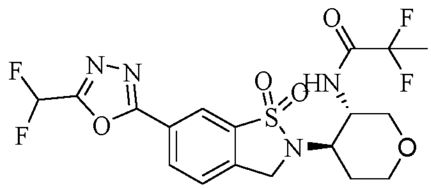 |
| 12. | 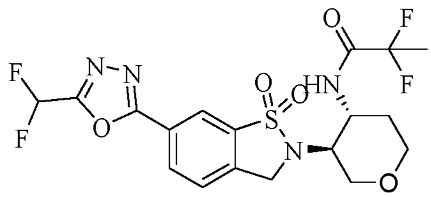 |
| 13. | 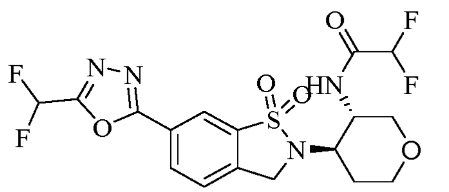 |
| 14. | 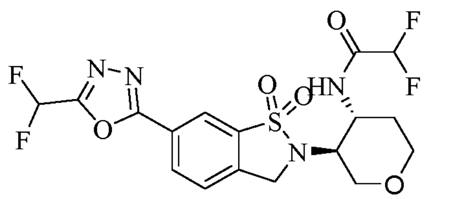 |
| 15. | 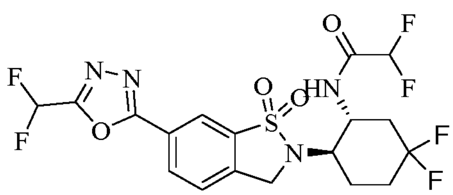 |

(continued)

| No. | Structure |
|---|---|
| 16. | 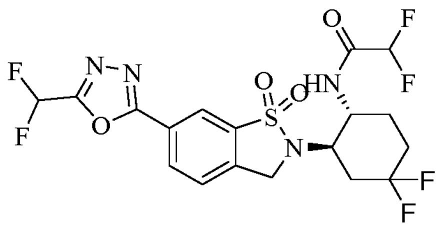 |
| 17. | 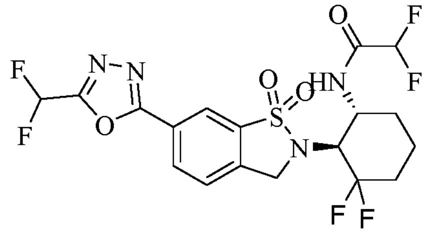 |
| 18. | 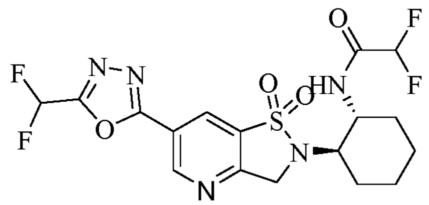 |
| 19. | 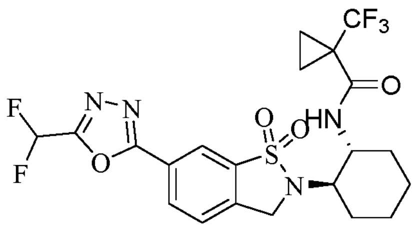 |
| 20. | 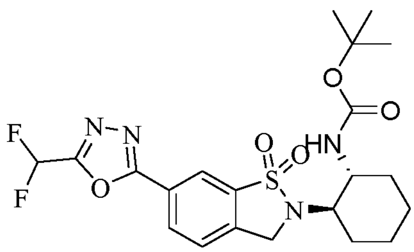 |
| 21. | 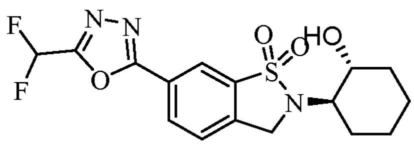 |
| 22. | 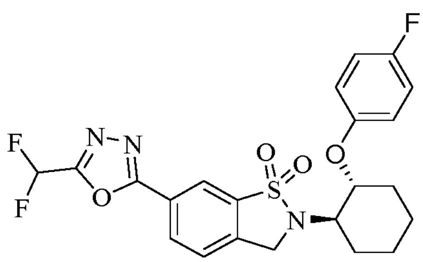 |

(continued)

| No. | Structure |
|---|---|
| 23. | 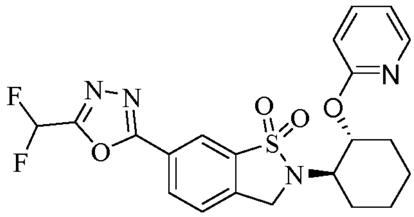 |
| 24. | 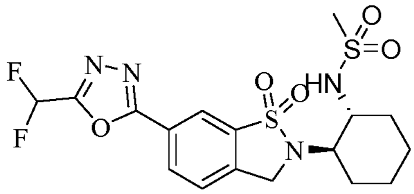 |
| 25. | 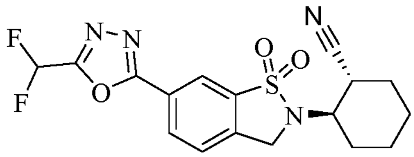 |
| 26. | 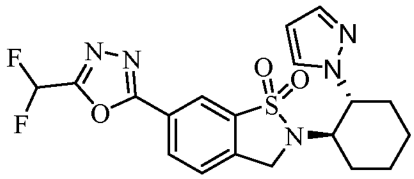 |
| 27. | 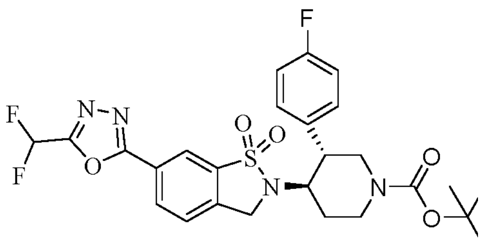 |
| 28. | 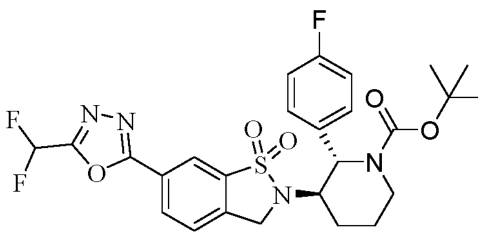 |
| 29. | 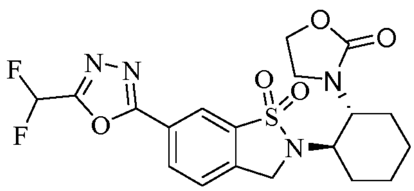 |

(continued)

| No. | Structure |
|---|---|
| 30. | 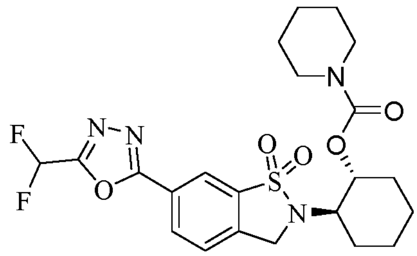 |
| 31. | 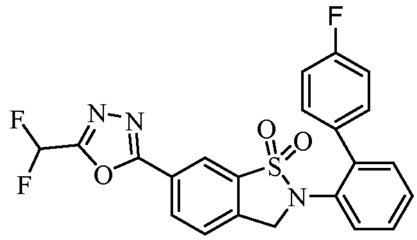 |
| 32. | 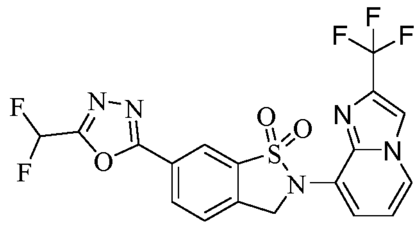 |
| 33. | 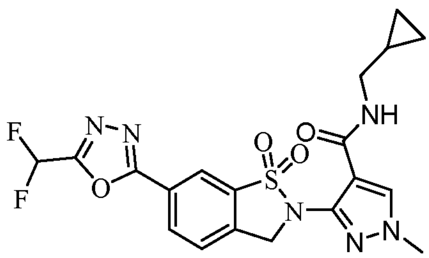 |
| 34. | 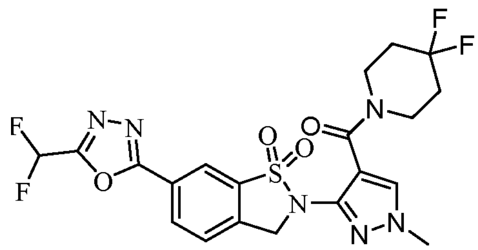 |
| 35. | 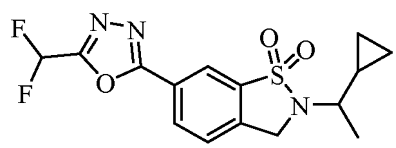 |
| 36. | 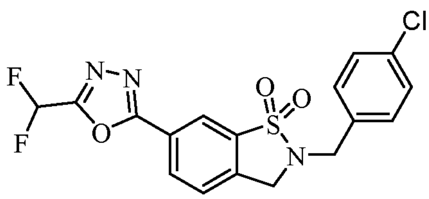 |

(continued)

| No. | Structure |
|---|---|
| 37. | 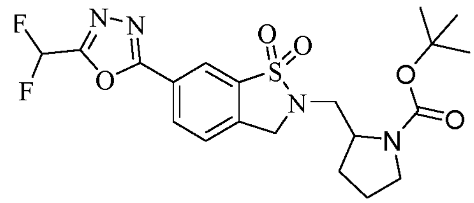 |
| 38. | 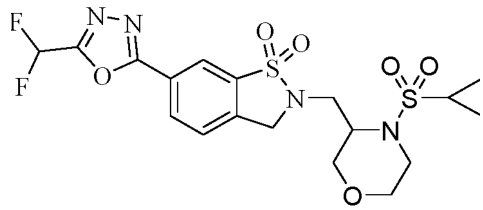 |
| 39. | 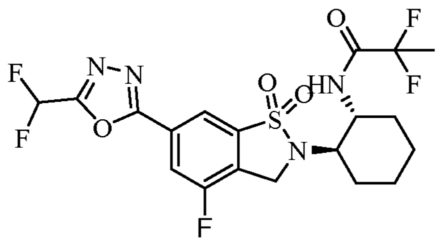 |
| 40. | 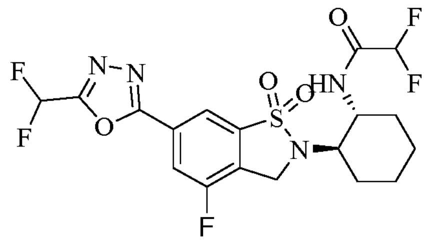 |
| 41. | 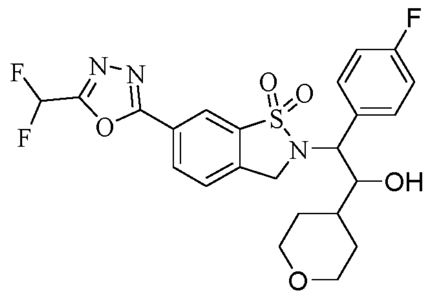 |
| 42. | 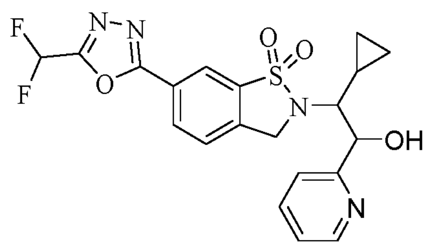 |

(continued)

| No. | Structure |
|---|---|
| 43. | 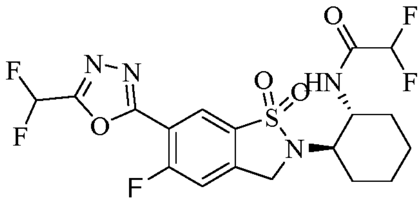 |
| 44. | 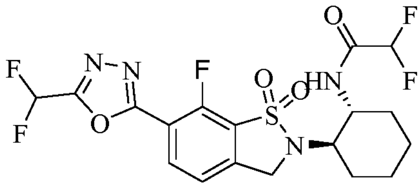 |
| 45. | 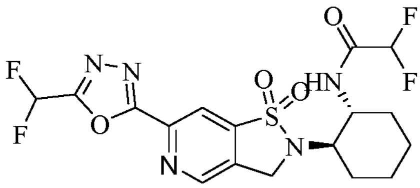 |
| 46. | 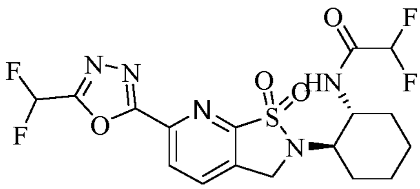 |
| 47. | 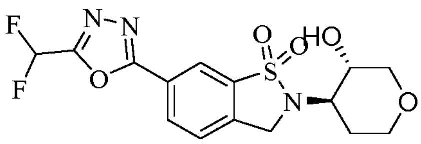 |
| 48. | 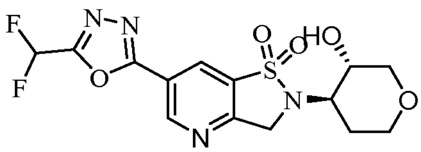 |
| 49. | 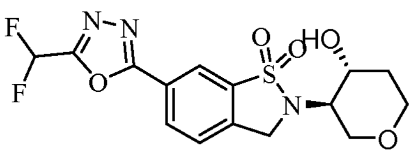 |
| 50. | 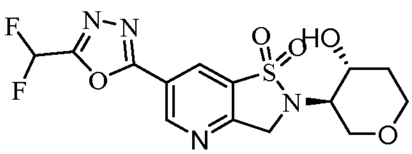 |

(continued)

| No. | Structure |
|---|---|
| 51. | 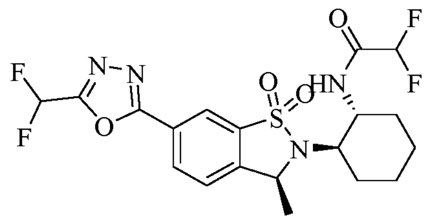 |
| 52. | 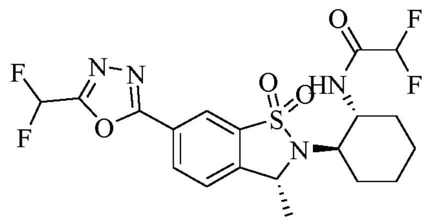 |
| 53. | 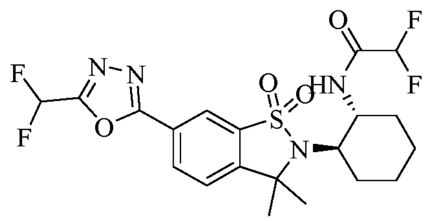 |
| 54. | 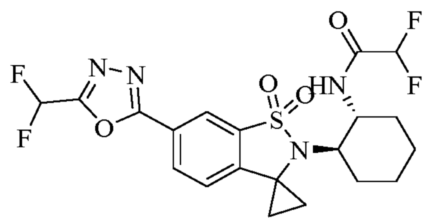 |
| 55. | 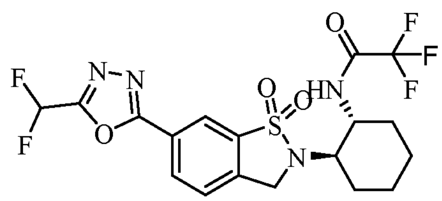 |
| 56. | 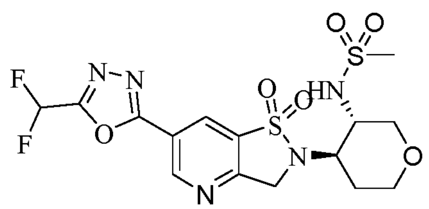 |
| 57. | 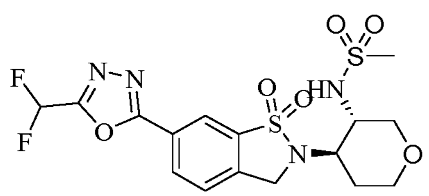 |

(continued)

| No. | Structure |
|---|---|
| 58. | 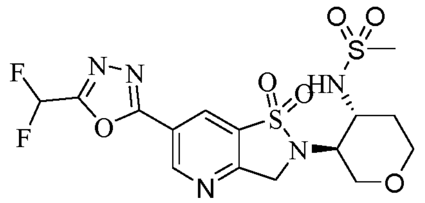 |
| 59. | 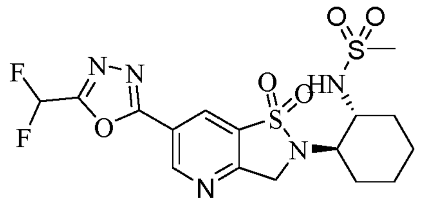 |
| 60. | 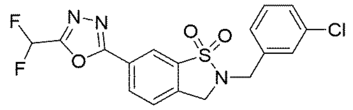 |
| 61. | 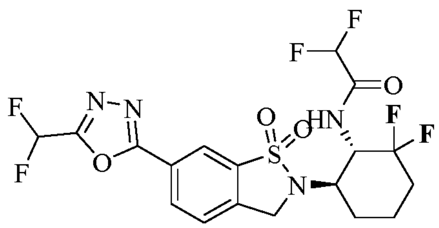 |
| 62. | 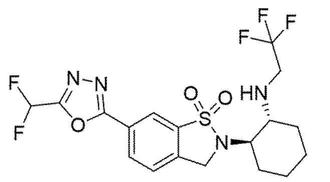 |
| 63. | 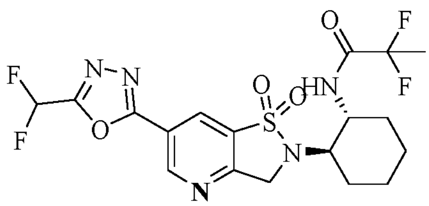 |
| 64. | 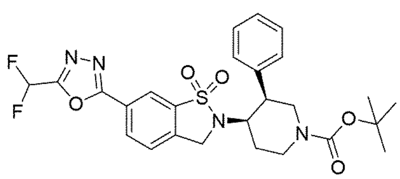 |
| 65. | 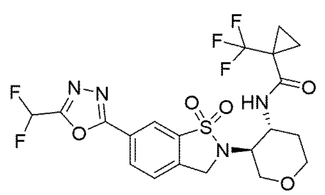 |

(continued)

| No. | Structure |
|---|---|
| 66. | 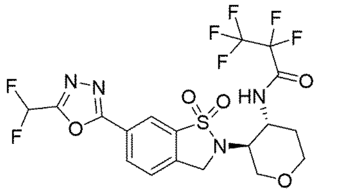 |
| 67. | 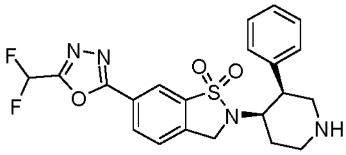 |
| 68. | 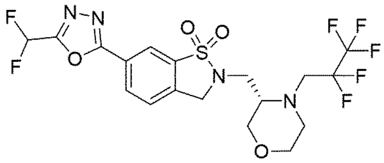 |
| 69. | 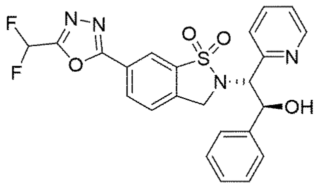 |
| 70. | 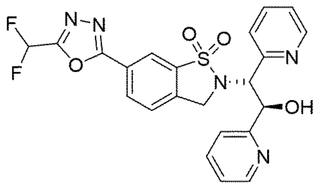 |
| 71. | 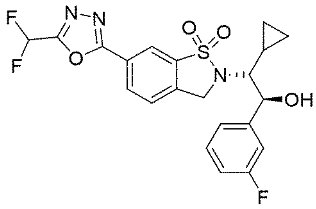 |
| 72. | 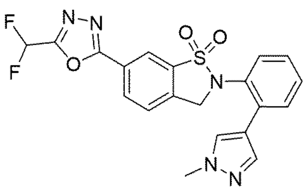 |
| 73. | 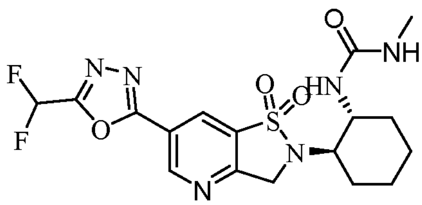 |

(continued)

| No. | Structure |
|---|---|
| 74. | 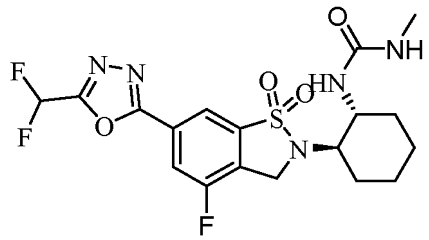 |
| 75. | 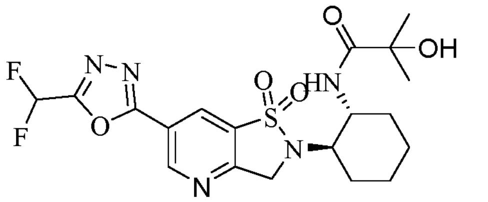 |
| 76. | 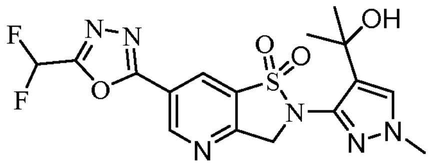 |
| 77. | 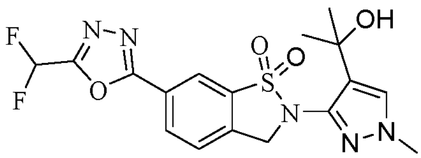 |
| 78. | 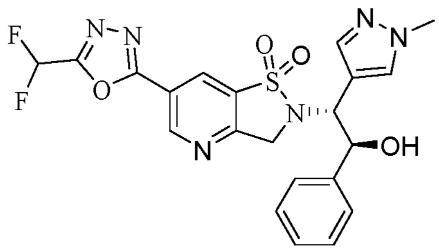 |
| 79. | 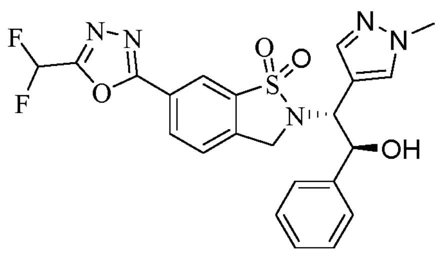 |
| 80. | 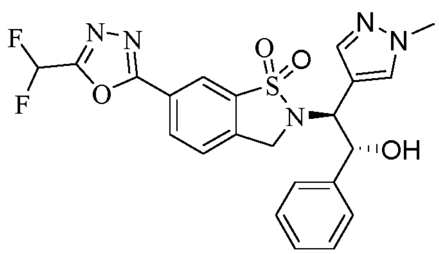 |

(continued)

| No. | Structure |
|---|---|
| 81. | 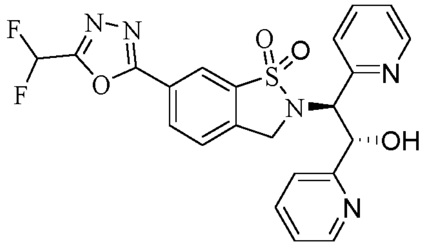 |
| 82. | 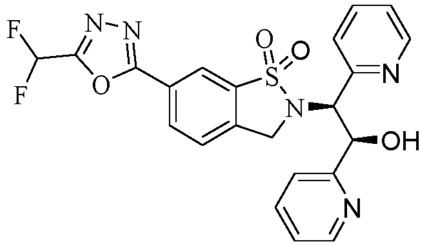 |
| 83. | 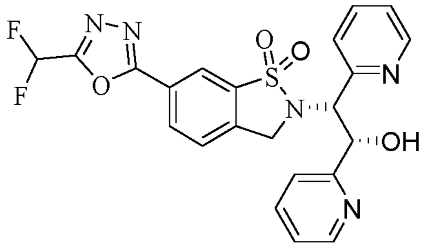 |
| 84. | 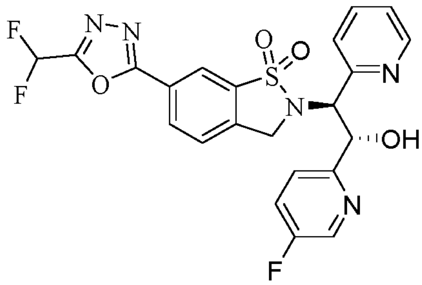 |
| 85. | 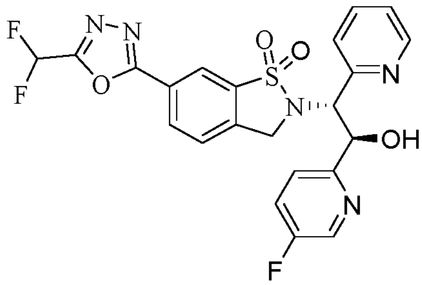 |
| 86. | 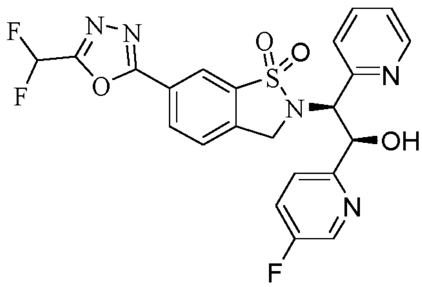 |

(continued)

| No. | Structure |
|---|---|
| 87. | 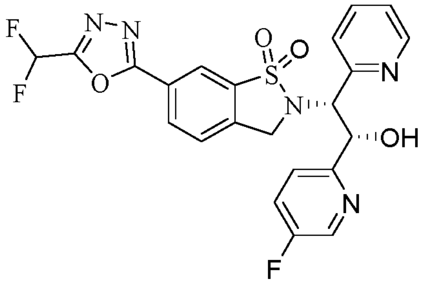 |
| 88. | 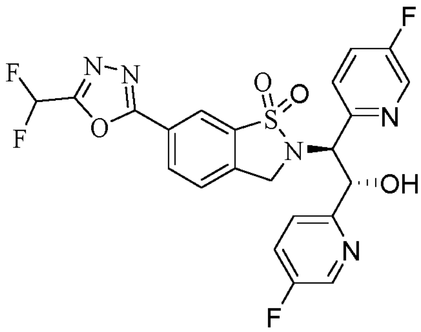 |
| 89. | 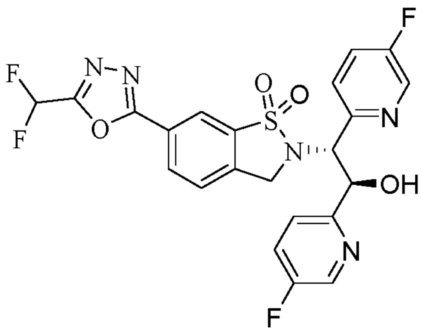 |
| 90. | 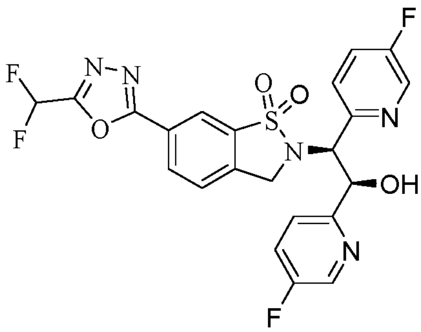 |
| 91. | 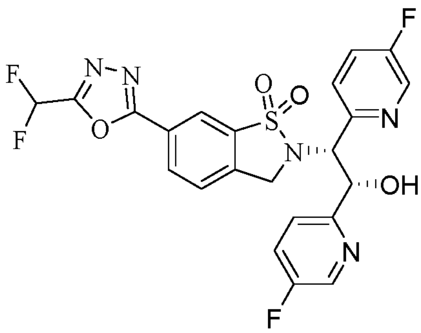 |

(continued)

| No. | Structure |
|---|---|
| 92. | 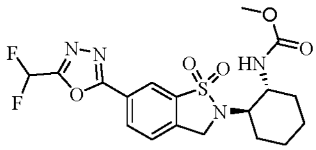 |
| 93. | 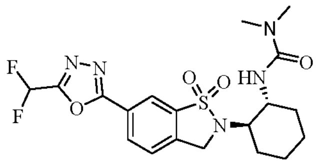 |
| 94. | 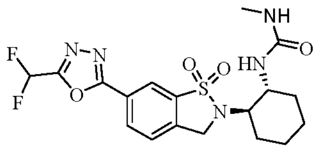 |
| 95. | 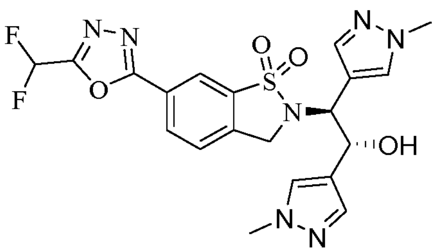 |
| 96. | 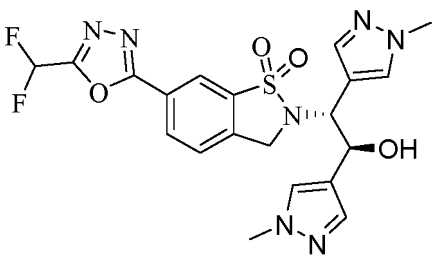 |
| 97. | 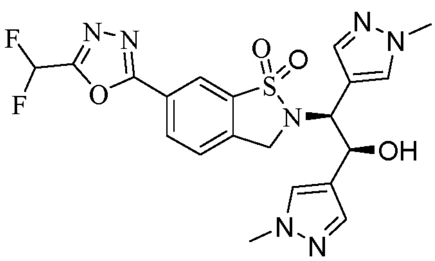 |
| 98. | 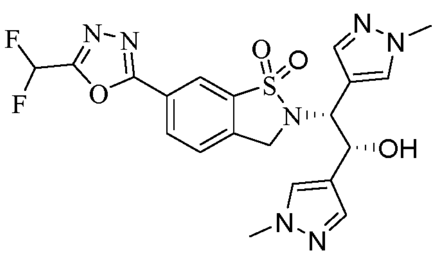 |

(continued)

| No. | Structure |
|---|---|
| **99.** | 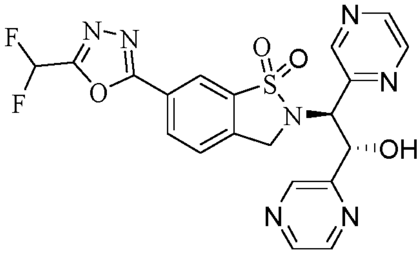 |
| **100.** | 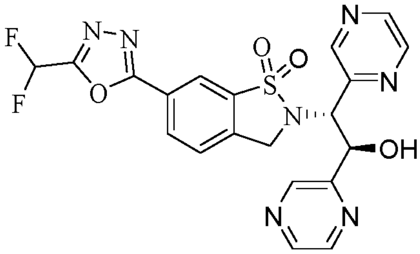 |
| **101.** | 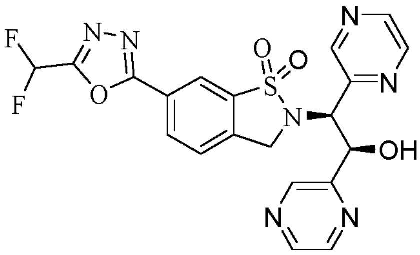 |
| **102.** | 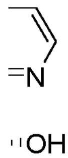 |
| **103.** | |
| **104.** | |
| **105.** | |
| **106.** | |
| **107.** | 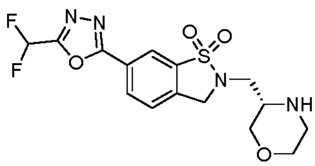 |
| **108.** | 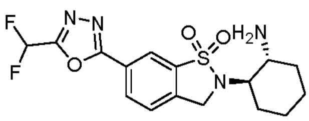 |
| **109.** | 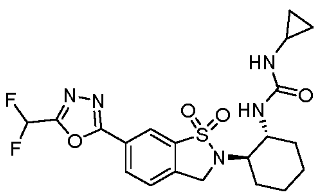 |

(continued)

| No. | Structure |
| --- | --- |
| 110. | 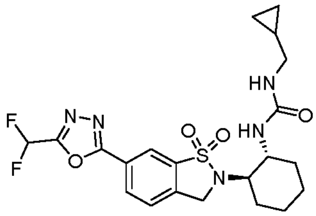 |
| 111. | 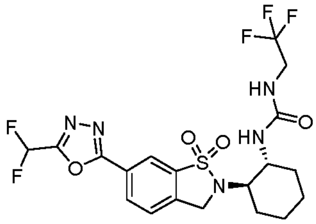 |
| 112. | 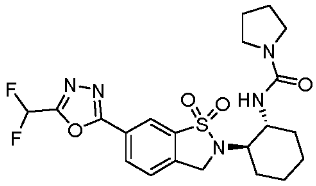 |
| 113. | 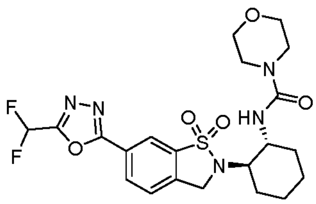 |
| 114. | 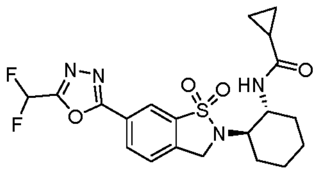 |
| 115. | 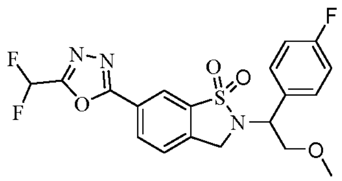 |
| 116. | 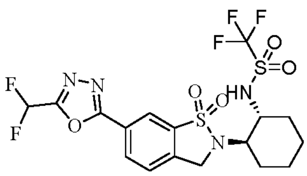 |
| 117. | 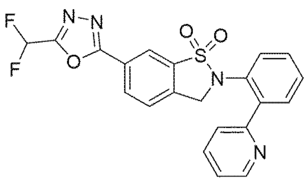 |

(continued)

| No. | Structure |
|---|---|
| **118.** | 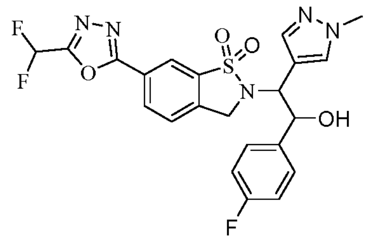 |
| **119.** | 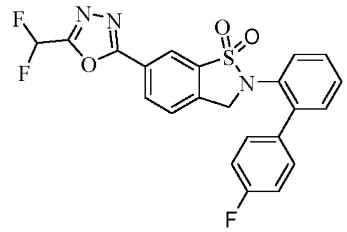 |
| **120.** | 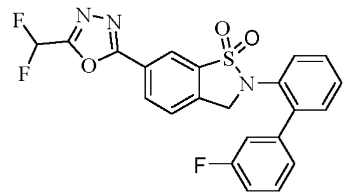 |
| **121.** | 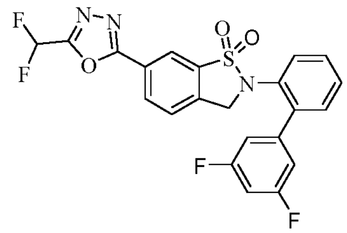 |
| **122.** | 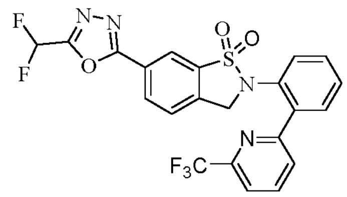 |
| **123.** | 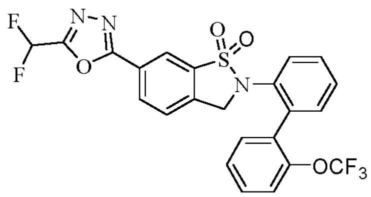 |
| **124.** | 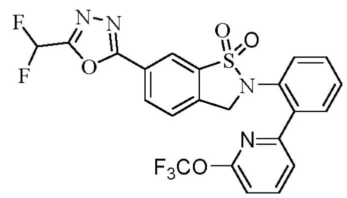 |

(continued)

| No. | Structure |
|---|---|
| 125. | 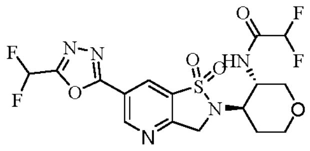 |
| 126. | 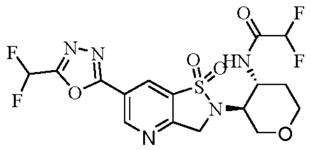 |
| 127. | 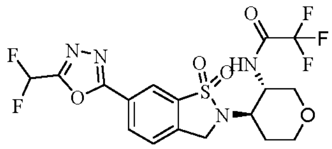 |
| 128. | 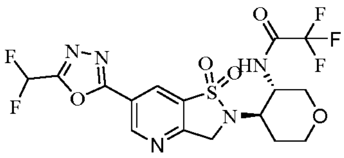 |
| 129. | 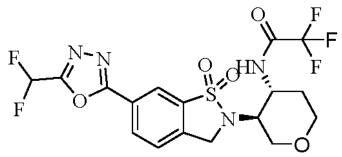 |
| 130. | 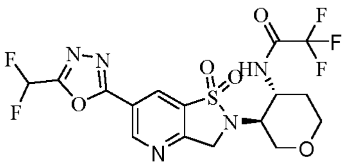 |
| 131. | 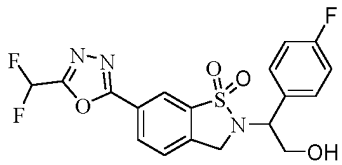 |
| 132. | 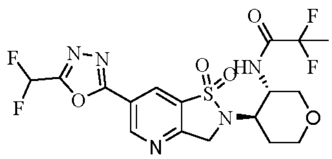 |
| 133. | 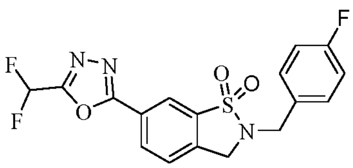 |

(continued)

| No. | Structure |
|---|---|
| 134. | 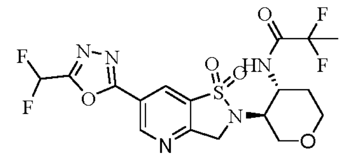 |
| 135. | 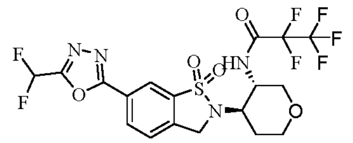 |
| 136. | 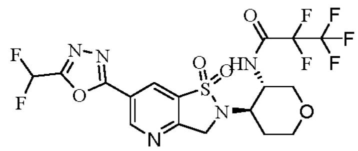 |
| 137. | 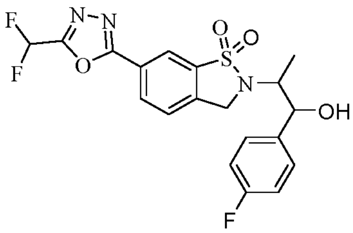 |
| 138. | 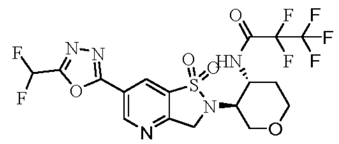 |
| 139. | 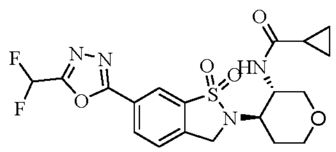 |
| 140. | 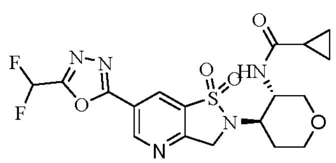 |
| 141. | 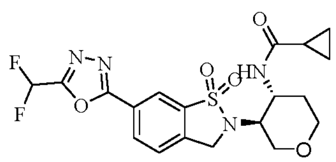 |
| 142. | 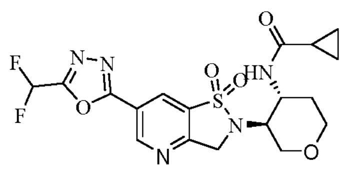 |

(continued)

| No. | Structure |
|---|---|
| 143. | 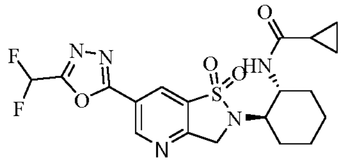 |
| 144. | 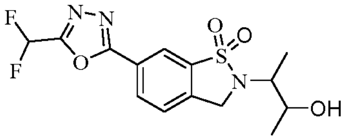 |
| 145. | 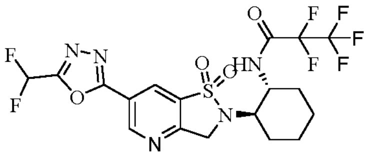 |
| 146. | 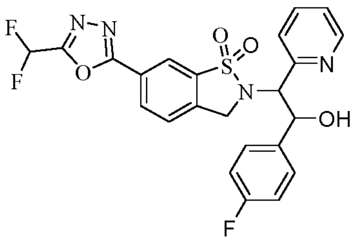 |
| 147. | 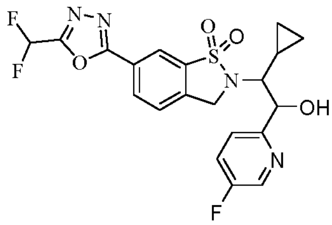 |
| 148. | 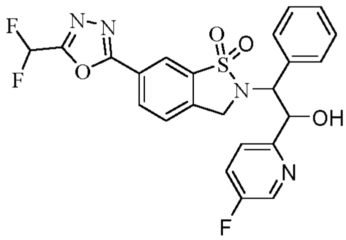 |
| 149. | 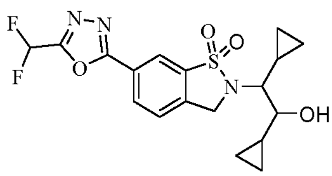 |
| 150. | 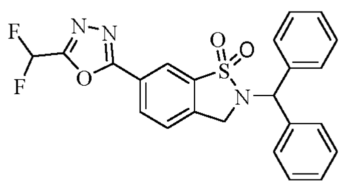 |

(continued)

| No. | Structure |
|---|---|
| 151. | 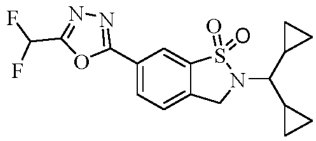 |
| 152. | 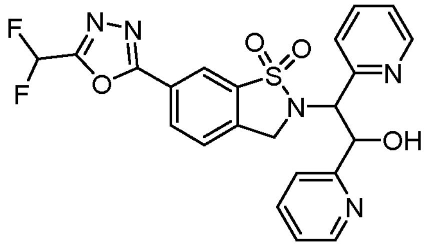 |
| 153. | 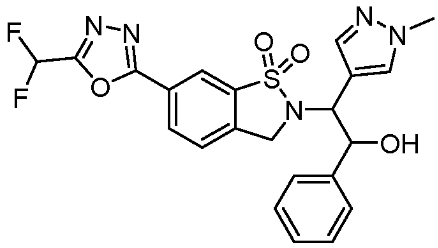 |
| 154. | 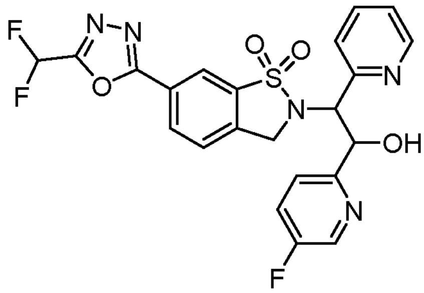 |
| 155. | 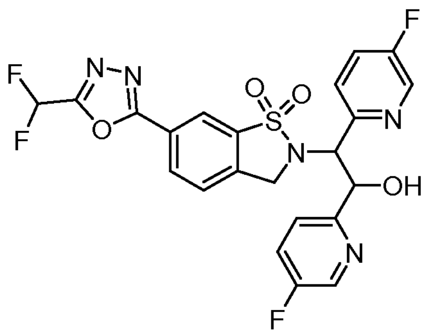 |
| 156. | 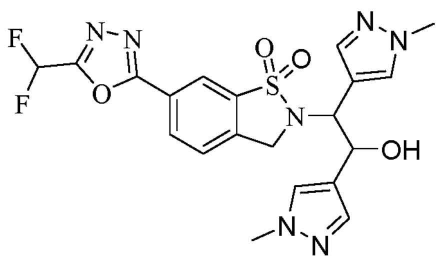 |

(continued)

| No. | Structure |
|---|---|
| 157. | 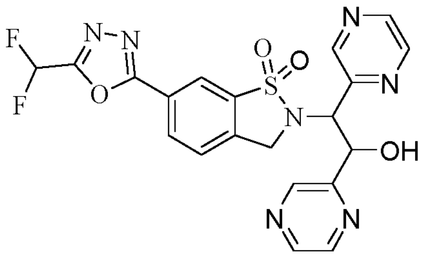 |
| 158. | 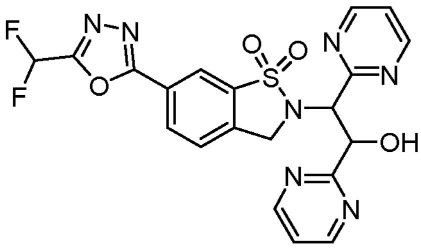 |
| 159. | 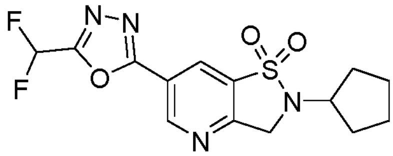 |

## Pharmaceutical Composition and Treatment Method

In some embodiments, the present invention provides a pharmaceutical composition comprising a prophylactically or therapeutically effective amount of the compound, or the pharmaceutically acceptable salt, the ester, the stereoisomer, the tautomer, the polymorph, the solvate, the metabolite, the isotopically labeled compound or the prodrug thereof of the present invention, and one or more pharmaceutically acceptable carriers. The pharmaceutical composition is preferably a solid formulation, a liquid formulation, or a transdermal formulation.

In some embodiments, the present invention provides use of the compound, or the pharmaceutically acceptable salt, the ester, the stereoisomer, the tautomer, the polymorph, the solvate, the metabolite, the isotopically labeled compound or the prodrug thereof of the present invention, or the pharmaceutical composition of the present invention in the preparation of a medicament for preventing or treating an HDAC6-related disease.

In some embodiments, the present invention provides the compound, or the pharmaceutically acceptable salt, the ester, the stereoisomer, the tautomer, the polymorph, the solvate, the metabolite, the isotopically labeled compound or the prodrug thereof of the present invention, or the pharmaceutical composition of the present invention, for use in preventing or treating an HDAC6-related disease.

In some embodiments, the present invention provides a method for preventing or treating an HDAC6-related disease, comprising administering to a subject in need thereof an effective amount of the compound, or the pharmaceutically acceptable salt, the ester, the stereoisomer, the tautomer, the polymorph, the solvate, the metabolite, the isotopically labeled compound or the prodrug thereof of the present invention, or the pharmaceutical composition of the present invention.

The HDAC6-related disease includes, but is not limited to, cancer or proliferative diseases (e.g., lung cancer, colon cancer, breast cancer, prostate cancer, liver cancer, brain cancer, kidney cancer, ovarian cancer, stomach cancer, skin cancer, bone cancer, pancreatic cancer, glioma, glioblastoma, hepatocellular carcinoma, papillary renal carcinoma, head and neck squamous cell carcinoma, leukemia, lymphoma, myeloma, multiple myeloma, and solid tumors); Wilson's disease, spinocerebellar ataxia, prion disease, Parkinson's disease, Huntington's disease, amyotrophic lateral sclerosis, amyloidosis, Alzheimer's disease, Alexander's disease, alcoholic liver disease, cystic fibrosis, Pick's disease, spinal muscular atrophy, or Lewy body dementia; rheumatoid arthritis, osteoarthritis; rheumatoid spondylitis; psoriasis; inflammatory bowel disease; chronic inflammatory lung disease, eczema, asthma, ischemia/reperfusion injury, ulcerative colitis, acute respiratory distress syndrome, psoriatic arthritis, infectious arthritis, progressive chronic arthritis, arthritis deformans, osteoarthritis, traumatic arthritis, gouty arthritis, Reiter's syndrome, polychondritis, acute synovitis and spondylitis, glomerulonephritis, hemolytic anemia, aplastic anemia, idiopathic thrombocytopenic purpura, neutropenia, ulcerative

colitis, Crohn's disease, host-versus-graft disease, graft-versus-host disease, allograft rejection, chronic thyroiditis, Graves' disease, scleroderma, diabetes, active hepatitis, primary biliary cirrhosis, myasthenia gravis, multiple sclerosis (MS), systemic lupus erythematosus, atopic dermatitis, contact dermatitis, sunburn of the skin, chronic renal insufficiency, Stevens-Johnson syndrome, idiopathic steatorrhea, sarcoidosis, Guillain-Barre syndrome, uveitis, conjunctivitis, keratoconjunctivitis, otitis media, periodontal disease, pulmonary interstitial fibrosis, asthma, bronchitis, rhinitis, sinusitis, pneumoconiosis, pulmonary insufficiency syndrome, emphysema, pulmonary fibrosis, or silicosis.

The term "pharmaceutically acceptable carrier" refers to a diluent, an adjuvant, an excipient, or a vehicle administered together with a therapeutic agent, which is suitable, within the scope of sound medical judgment, for contact with the tissues of humans and/or other animals without undue toxicity, irritation, allergic response, or other problems or complications commensurate with a reasonable benefit/risk ratio. Unless otherwise stated, the term "treat", "treating", or "treatment" as used herein means reversing, alleviating, or inhibiting the progression of a disorder or condition to which such term applies or one or more symptoms of such a disorder or condition, or preventing such a disorder or condition or one or more symptoms of such a disorder or condition.

As used herein, an "individual" includes a human or non-human animal. Exemplary human individuals include human individuals with a disease (e.g., a disease described herein), which are referred to as patients, or normal individuals. In the present invention, "non-human animals" include all vertebrates, e.g., non-mammals (e.g., birds, amphibians, reptiles) and mammals, e.g., non-human primates, livestock, and/or domesticated animals (e.g., sheep, dogs, cats, cows, pigs, etc.).

In another embodiment, the pharmaceutical composition of the present invention may further comprise one or more additional therapeutic or prophylactic agents.

**Preparation Method**

In some embodiments, the compounds of the present application are synthesized by the following reaction routes:

Route I

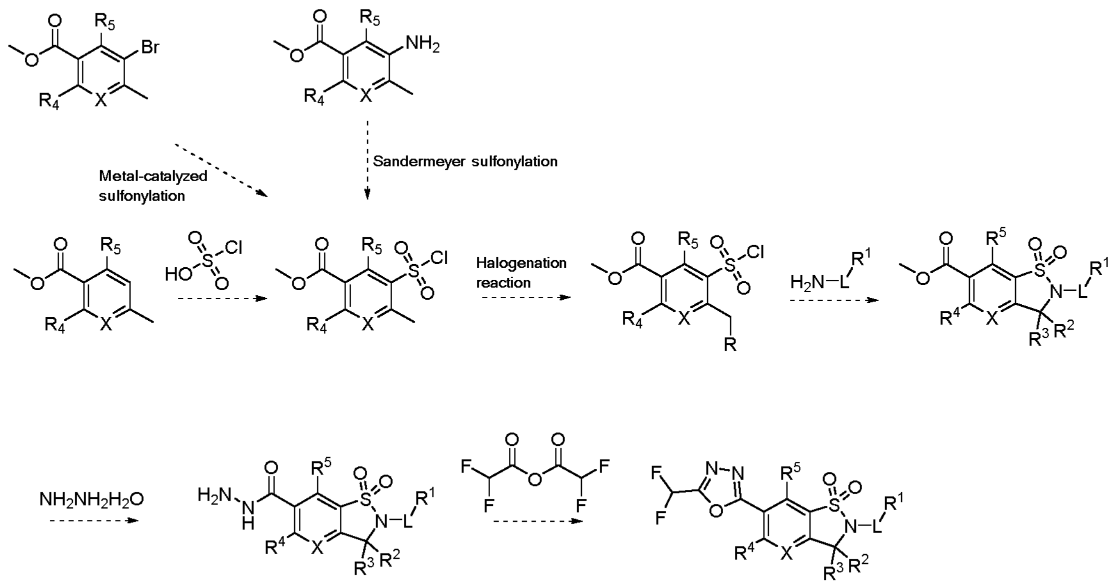


wherein R is halogen and the other groups are as defined herein.

Route II

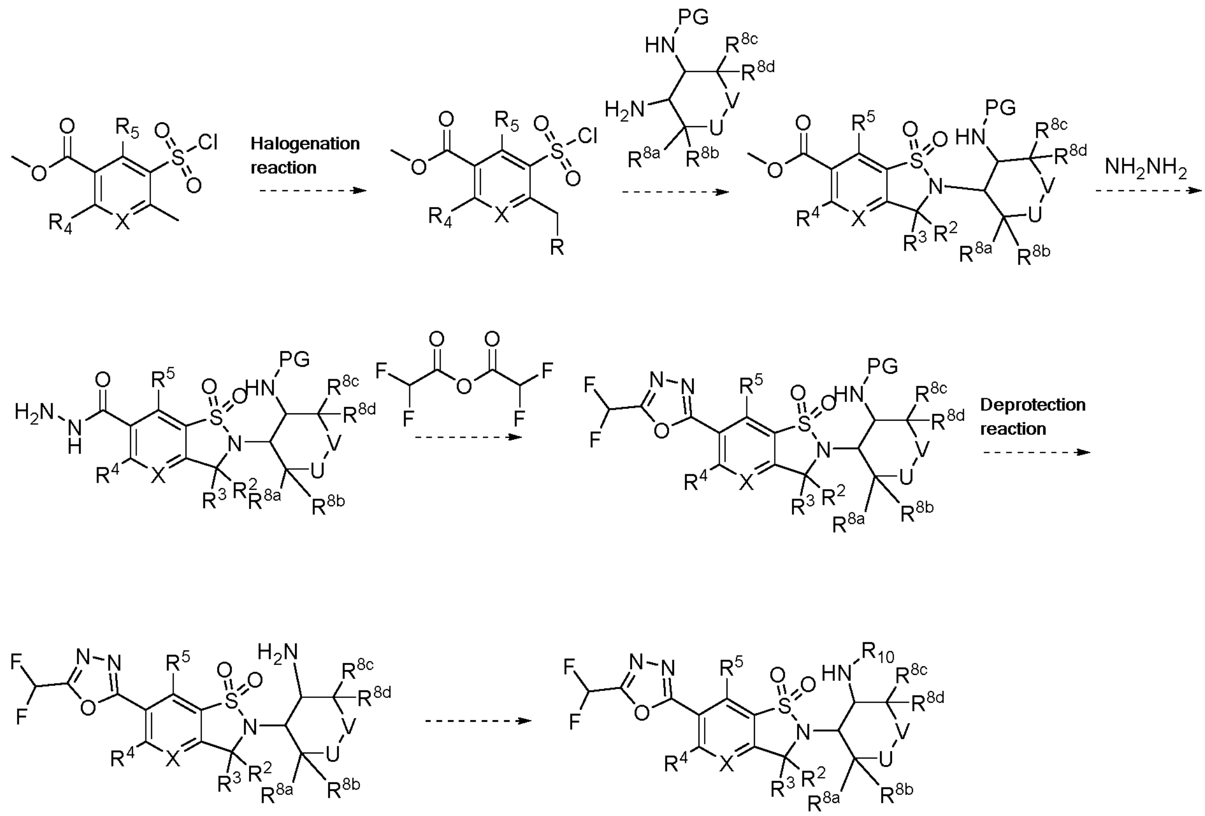


wherein R is halogen; PG is an amino protecting group; $R^{10}$ is -C(=O)$R^d$, -C(=O)O$R^d$, -S(=O)$_2$-$R^d$, or C(=O)-N$R^cR^d$; the other groups are as defined herein.

Route III

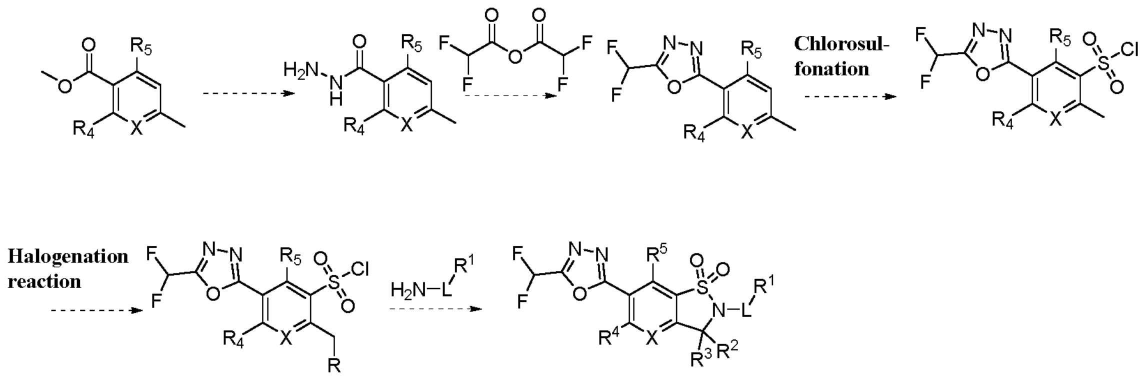


wherein R is halogen and the other groups are as defined herein.

Route IV

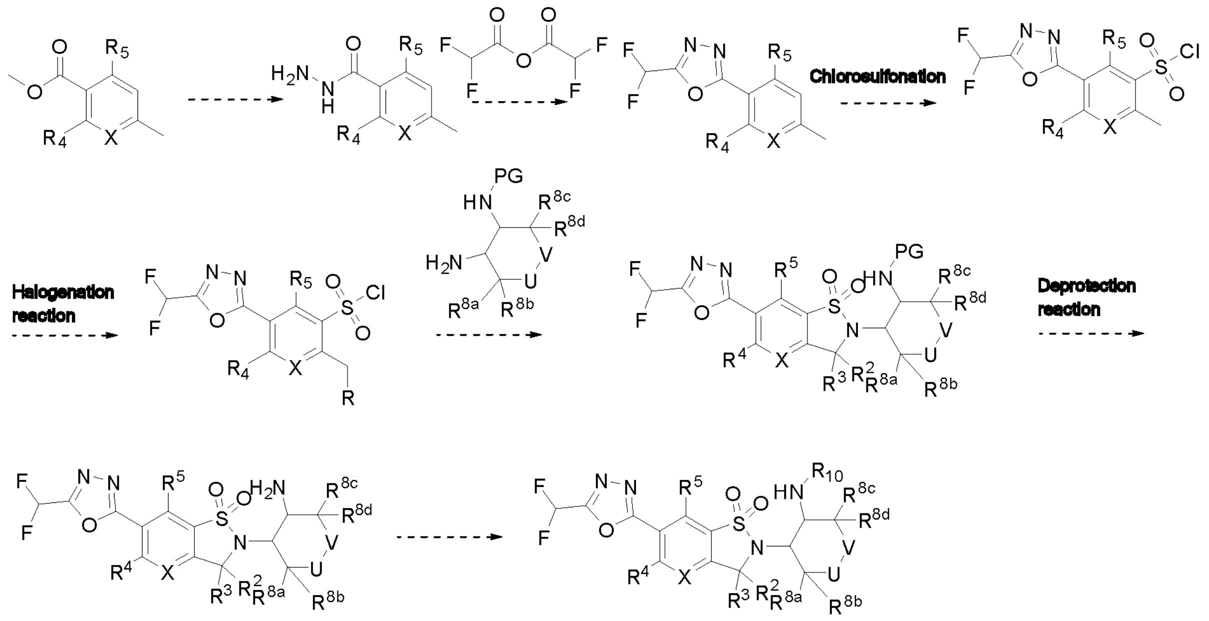


wherein R is halogen; PG is an amino protecting group; $R^{10}$ is -C(=O)$R^d$, -C(=O)O$R^d$, -S(=O)$_2$-$R^d$, or C(=O)-N$R^cR^d$; the other groups are as defined herein.

Route V

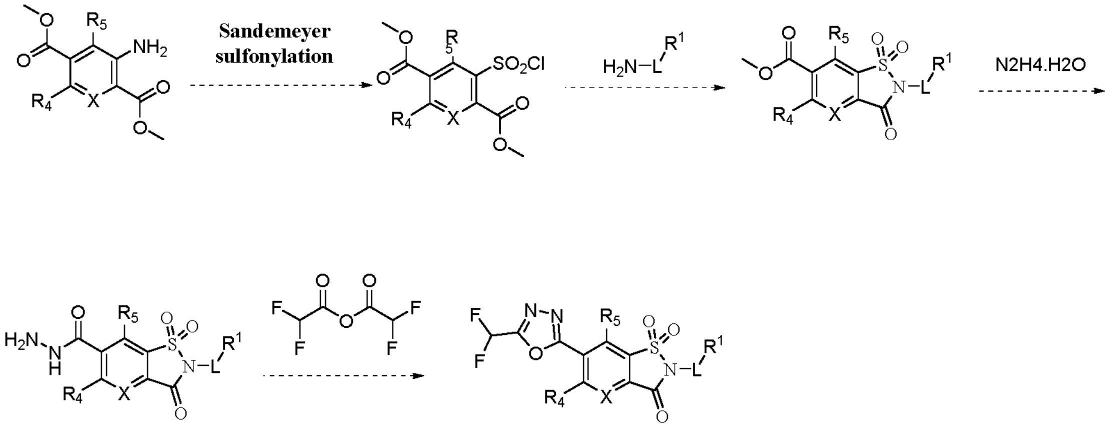


wherein the groups are as defined herein.

Route VI

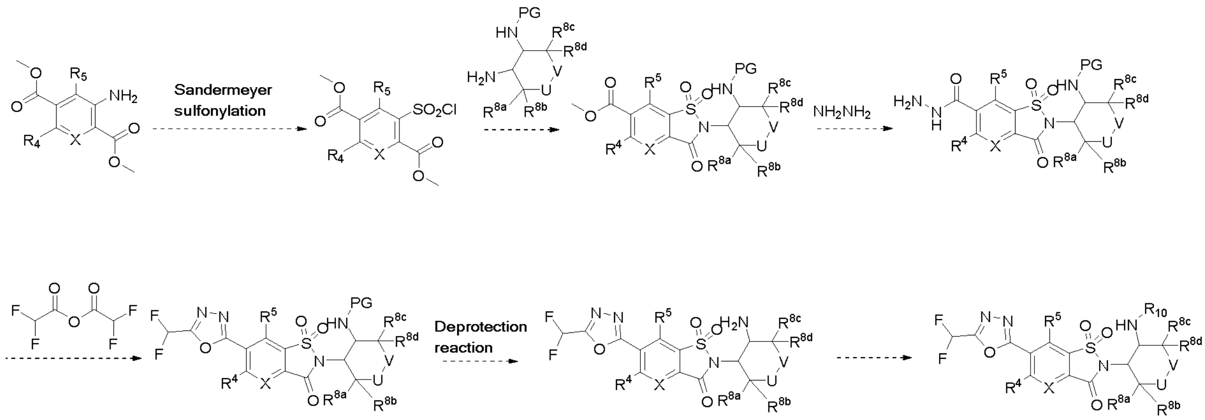


wherein PG is an amino protecting group; $R^{10}$ is $-C(=O)R^d$, $-C(=O)OR^d$, $-S(=O)_2-R^d$, or $C(=O)-NR^cR^d$; the other groups are as defined herein.

**Example**

The present invention is further described below with reference to examples, but these examples are not intended to limit the scope of the present invention.

The abbreviations herein have the following meanings:

| **Abbreviation** | **Full version** |
|---|---|
| ACN/MeCN | Acetonitrile |
| AcOH | Acetic acid |
| AIBN | Azobisisobutyronitrile |
| BnSH | Benzyl mercaptan |
| $CDCl_3$ | Deuterated chloroform |
| $Cs_2CO_3$ | Cesium carbonate |
| DAST | Diethylaminosulfur trifluoride |
| DCE | Dichloroethane |
| DCM | Dichloromethane |
| DIPEA/DIEA | *N,N*-diisopropylethylamine |
| DMF | *N,N*-dimethylfonnamide |
| DMSO | Dimethyl sulfoxide |
| EtOAc | Ethyl acetate |
| EtOH | Ethanol |
| HATU | O-(7-azabenzotriazol-l-yl)-*N*,*N*,*N*',*N*'-tetramethyluronium hexafluorophosphate |
| LC-MS | Liquid chromatography-mass spectrometry |
| MeCN | Acetonitrile |
| MeOH | Methanol |
| NBS | *N*-bromosuccinimide |
| NCS | *N*-chlorosuccinimide |
| n-BuLi | *n*-Butyllithium |
| NMR | Nuclear magnetic resonance |
| $Pd_2(dba)_3$ | Tris(dibenzylideneacetone)dipalladium |

(continued)

| Abbreviation | Full version |
|---|---|
| Pd(dppf)$Cl_2$ | 1,1'-Bis(diphenylphosphino)ferrocenepalladium dichloride |
| Pre-HPLC | Preparative high-performance liquid chromatography |
| SFC | Chiral resolution |
| TEA | Triethylamine |
| TFA | Trifluoroacetic acid |
| THF | Tetrahydrofuran |
| TLC | Thin-layer chromatography |
| TsCl | *p*-Toluenesulfonyl chloride |
| Xantphos | 4,5-Bis(diphenylphosphino)-9,9-dimethylxanthene |
| XPhosPd-G3 | Methanesulfonato(2-dicyclohexylphosphino-2',4',6'-triisopropyl-1,1'-biphenyl)(2'-amino-1,1'-biphenyl-2-yl)palladium(II) |

**Example 1: (Compound 1)**

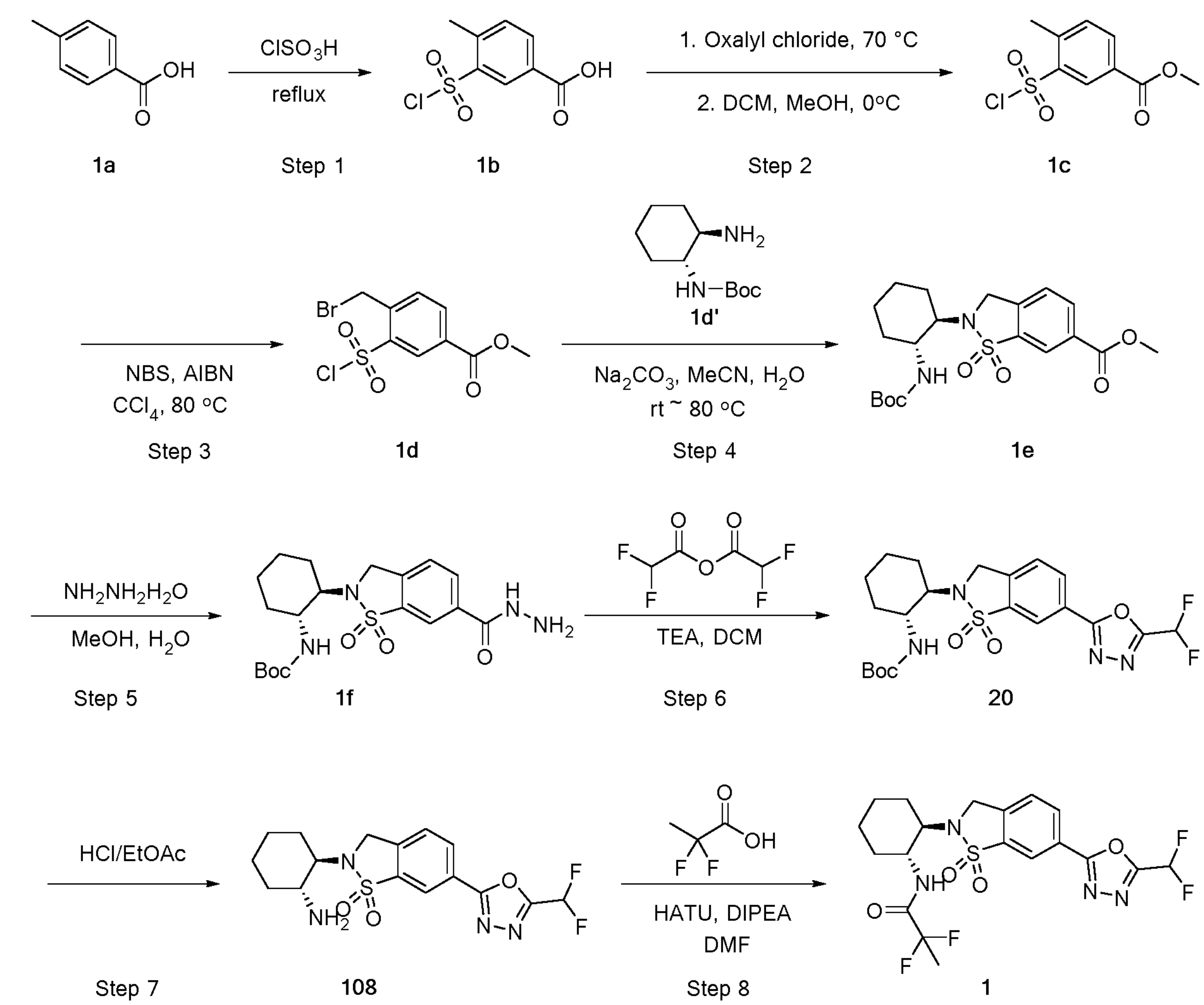

1a 1b 1c 1d 1e 1f 20 108 1

Step 1: (compound **1b**)

Compound **1a** (5 g, 36.72 mmol) was dissolved in chlorosulfonic acid (17.11 g, 146.88 mmol). The mixture was stirred at 120 °C for 4 h under nitrogen atmosphere. After the reaction was completed as detected by LC-MS, the reaction solution was slowly poured into crushed ice, and the resulting mixture was filtered to give a solid, which was dried under vacuum to give **1b** (7.3 g, 75%) as a white solid.
$^1$H NMR (400 MHz, DMSO-$d_6$) δ 8.35 (d, J = 1.9 Hz, 1H), 7.80 (dd, J = 7.8, 2.0 Hz, 1H), 7.29 (d, J =7.8 Hz, 1H), 2.61 (s, 3H).

Step 2: (compound **1c**)

Compound **1b** (6.3 g, 26.85 mmol) was dissolved in oxalyl chloride (30 g, 236.35 mmol). The mixture was stirred at 70 °C for 2 h under nitrogen atmosphere. After the reaction was completed as detected by TLC, the reaction was quenched with methanol, and the reaction solution was concentrated under reduced pressure to remove excess oxalyl chloride. The residue was dissolved in dichloromethane (20 mL). The mixture was cooled to 0 °C, and methanol (10 mL) was slowly added dropwise. After the addition, the mixture was stirred at 0 °C for 2 h. After the reaction was completed as detected by TLC, the reaction solution was concentrated and purified by column chromatography to give **1c** (5.5 g, 82%) as a white solid.
$^1$HNMR (400MHz, $CDCl_3$) δ 8.76 (d, J = 1.8Hz, 1H), 8.30 (dd, J = 7.9, 1.8Hz, 1H), 7.57 (d, J = 8.0Hz, 1H), 4.01 (s, 3H), 2.90 (s, 3H).

Step 3: (compound **1d**)

To carbon tetrachloride (30 mL) were added compound **1c** (2 g, 8.04 mmol), *N*-bromosuccinimide (1.57 g, 8.84 mmol), and azobisisobutyronitrile (132.02 mg, 0.80 mmol). The mixture was stirred at 80 °C for 8 h under nitrogen atmosphere. After the reaction was completed as monitored by TLC, the reaction solution was diluted with ethyl acetate (100 mL) and water (30 mL). The separated organic phase was washed with saturated brine (30 mL × 2), dried over anhydrous sodium sulfate, filtered, concentrated, and purified by column chromatography to give **1d** (1.6 g, 35%) as a colorless oil.
$^1$HNMR (400MHz, $CDCl_3$) δ 8.80 - 8.76 (m, 1H), 8.41 (dd, J = 8.1, 1.8Hz, 1H), 7.90 (d, J = 8.1Hz, 1H), 5.06 (s, 2H), 4.03 (s, 3H).

Step 4: (compound **1e**)

To a mixed solvent of acetonitrile (30 mL) and water (6 mL) were added compound **1d** (1.6 g, 2.78 mmol), **1d'** (0.6, 2.78 mmol), and sodium carbonate (0.69 g, 5.56 mmol). The mixture was stirred at 25 °C for 30 min, and then heated to 80 °C and stirred for 4 h. The reaction solution was diluted with ethyl acetate (60 mL) and water (20 mL). The separated organic phase was washed with saturated brine (15 mL × 2), dried over anhydrous sodium sulfate, filtered, concentrated, and purified by column chromatography to give **1e** (1.02 g, 86%) as a colorless oil.

ESI m/z $[M+H]^+$ = 425.1.
$^1$HNMR (400 MHz, DMSO-$d_6$) δ 8.24 (d, J = 9.2Hz, 2H), 7.81 (d, J = 8.0Hz, 1H), 6.72 (d, J = 9.2Hz, 1H), 4.79 (d, J = 15.2Hz, 1H), 4.58 (d, J = 15.1Hz, 1H), 3.92 (s, 3H), 3.55-3.41 (m, 2H), 1.88-1.66 (m, 5H), 1.51 (d, J = 12.1Hz, 1H), 1.27 (d, J = 9.6Hz, 2H), 1.10 (s, 9H).

Step 5: (compound **1f**)

To methanol (6 mL) were added compound **1e** (500 mg, 1.18 mmol) and hydrazine hydrate (2.06 g, 32.92 mmol, 80%). The mixture was stirred in a sealed tube at 80 °C for 2 h. After the reaction was completed as detected by LC-MS, the reaction solution was concentrated under reduced pressure to remove excess methanol, filtered, and dried under vacuum to give **1f** (495 mg, 99%) as a white solid.
ESI m/z $[M+H]^+$ = 425.2.

Step 6: (compound **20)**

To dichloromethane (20 mL) were sequentially added compound **1f** (495 mg, 1.17 mmol), triethylamine (0.53 g, 5.23 mmol), and difluoroacetic anhydride (0.45 g, 2.57 mmol). The mixture was stirred at 25 °C for 16 h. After the reaction was completed as detected by LC-MS, the reaction solution was concentrated and purified by column chromatography to

give **20** (330 mg, 58%) as a white solid.

ESI m/z $[M+H]^+$ = 485.1. $^1H$ NMR (400 MHz, DMSO-$d_6$) δ 8.40 (d, J = 1.5 Hz, 1H), 8.36 (dd, J = 8.1, 1.6 Hz, 1H), 7.93 (d, J =8.1 Hz, 1H), 7.59 (t, J = 51.3 Hz, 1H), 6.76 (d, J = 9.1 Hz, 1H), 4.83 (d, J = 15.2 Hz, 1H), 4.62 (d, J =15.2 Hz, 1H), 3.54 - 3.41 (m, 2H), 1.84 (d, J = 11.8 Hz, 2H), 1.73 (d, J = 17.5 Hz, 3H), 1.52 (q, J = 12.3, 11.5 Hz, 1H), 1.31 (d, J = 12.2 Hz, 2H), 1.12 (s, 9H).

Step 7: (compound **108)**

Compound **20** (150 mg, 0.31 mmol) was dissolved in a 4 M solution of hydrogen chloride in ethyl acetate (20 mL). The solution was stirred at 25 °C for 2 h. After the reaction was completed as detected by LC-MS, the reaction solution was concentrated to give **108** (130 mg, 100%) as a white solid.

ESI m/z $[M+H]^+$ = 385.1. $^1H$ NMR (400 MHz, DMSO-$d_6$) δ 8.40 (d, J = 1.5 Hz, 1H), 8.36 (dd, J = 8.1, 1.6 Hz, 1H), 7.93 (d, J = 8.1 Hz, 1H), 7.59 (t, J = 51.3 Hz, 1H), 6.76 (d, J = 9.1 Hz, 1H), 4.83 (d, J = 15.2 Hz, 1H), 4.62 (d, J =15.2 Hz, 1H), 3.47 (dtd, J = 21.6, 10.8, 5.2 Hz, 2H), 1.84 (d, J = 11.5 Hz, 2H), 1.73 (d, J = 17.5 Hz, 3H), 1.52 (d, J = 12.2 Hz, 1H), 1.31 (d, J = 12.6 Hz, 2H).

Step 8: (compound 1)

To N,N-dimethylformamide (3 mL) were sequentially added compound **108** (65 mg, 0.15 mmol), difluoropropionic acid (20 mg, 0.18 mmol), N,N-diisopropylethylamine (39 mg, 0.3 mmol), and HATU (74 mg, 0.20 mmol). The mixture was stirred at 25 °C for 2 h. After the reaction was completed as detected by LC-MS, the reaction solution was diluted with ethyl acetate (40 mL) and water (15 mL). The separated organic phase was washed with saturated brine (15 mL × 2), dried over anhydrous sodium sulfate, filtered, concentrated, and purified by column chromatography to give 1 (26 mg, 36%) as a white solid.

ESI m/z $[M+H]^+$ = 477.1. $^1H$ NMR (400 MHz, DMSO-$d_6$) δ 7.97 (d, J = 9.3 Hz, 1H), 7.77 (d, J = 1.5 Hz, 1H), 7.74 (dd, J = 8.1, 1.6 Hz, 1H), 7.28 (d, J = 8.1 Hz, 1H), 6.95 (t, J = 51.3 Hz, 1H), 4.14 - 3.91 (m, 2H), 3.36 - 3.21 (m, 1H), 3.01 (td, J = 11.0, 4.2 Hz, 1H), 1.28 - 1.03 (m, 6H), 0.87 (t, J = 19.4 Hz, 3H), 0.71 (d, J = 11.2 Hz, 2H).

**Example 2: (Compound 2)**

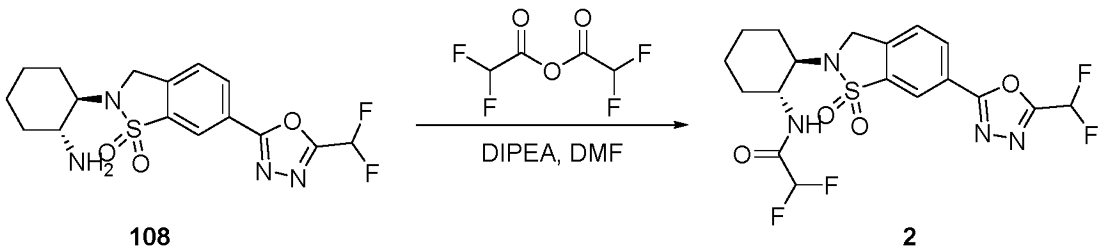


108 2

To a solution of compound **108** (71 mg, 0.17 mmol) and *N*,*N*-diisopropylethylamine (0.11 g, 0.85 mmol) in *N*,*N*-dimethylformamide (3 mL) was added dropwise difluoroacetic anhydride (59 mg, 0.34 mmol) at 25 °C. After the dropwise addition, the mixture was stirred for 1 h. After the reaction was completed as detected by TLC, the reaction solution was diluted with ethyl acetate (40 mL) and water (15 mL). The separated organic phase was washed with saturated brine (15 mL × 2), dried over anhydrous sodium sulfate, filtered, concentrated, and purified by column chromatography to give 2 (36.2 mg, 46%) as a white solid.

ESI m/z $[M+H]^+$ = 463.0. $^1H$ NMR (400 MHz, DMSO-$d_6$) δ 8.76 (d, J = 9.3 Hz, 1H), 8.44-8.31 (m, 2H), 7.92 (d, J = 8.1 Hz, 1H), 7.60 (t, J = 51.3 Hz, 1H), 6.03 (t, J = 53.8 Hz, 1H), 4.76 -4.62 (m, 2H), 3.99-3.87 (m, 1H), 3.61 (td, J =10.8, 4.5 Hz, 1H), 1.94-1.57 (m, 6H), 1.32 (d, J = 25.9 Hz, 2H).

**Example 3: (Compound 3)**

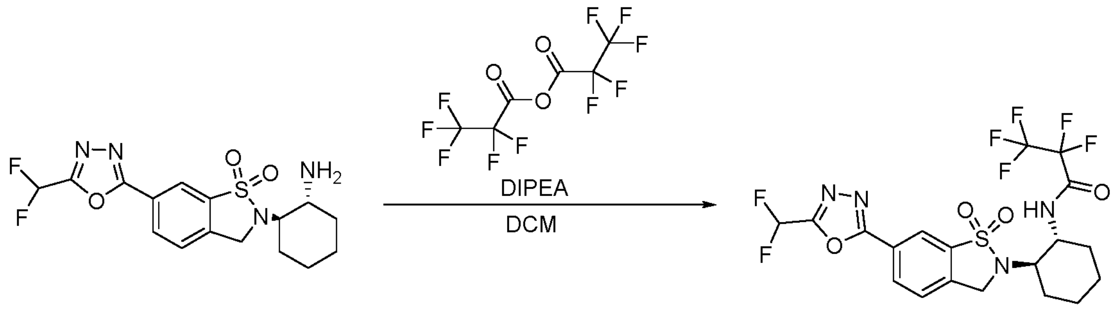


108 3

Compound **108** (200 mg, 0.48 mmol) was dissolved in dichloromethane (4 mL), and *N,N*-diisopropylethylamine (156.27 mg, 0.50 mmol) and pentafluoropropionic anhydride (189.21 mg, 1.46 mmol) were added. The mixture was stirred at 10 °C for 14 h. After the reaction was completed as detected by LCMS, the reaction solution was poured into water, and the resulting mixture was extracted with dichloromethane. The organic phase was washed once with saturated brine, dried over anhydrous sodium sulfate, filtered, concentrated, and subjected to column chromatography to give **3** (23 mg, 9%) as a white solid.

ESI m/z $[M-H]^-$ = 529.1. $^1$H NMR (400 MHz, DMSO-$d_6$) δ 9.44 (d, *J* = 9.1 Hz, 1H), 8.39 (d, *J* = 1.5 Hz, 1H), 8.35 (dd, *J* = 8.1, 1.6 Hz, 1H), 7.89 (d, *J* = 8.1 Hz, 1H), 7.56 (t, *J* = 51.3 Hz, 1H), 4.69 (d, *J* = 15.4 Hz, 1H), 4.56 (d, *J* = 15.4 Hz, 1H), 4.09 - 3.90 (m, 1H), 3.63 (td, *J* = 11.0, 4.2 Hz, 1H), 1.78 (ddd, *J* = 33.3, 16.2, 6.8 Hz, 6H), 1.34 (d, *J* = 9.4 Hz, 2H).

**Example 4: (Compound 4)**

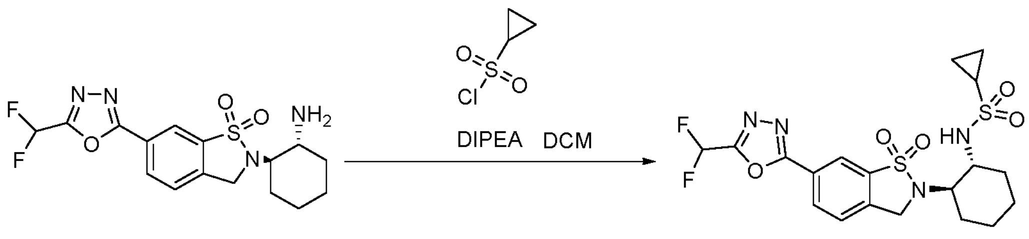


108 4

Compound **108** (200 mg, 0.48 mmol) was dissolved in dichloromethane (4 mL), and *N,N*-diisopropylethylamine (186.11 mg, 1.44 mmol) and cyclopropylsulfonyl chloride (80.98 mg, 0.58 mmol) were added. The mixture was stirred at 10 °C for 14 h. After the reaction was completed as detected by LCMS, the reaction solution was poured into water, and the resulting mixture was extracted with dichloromethane. The organic phase was washed once with saturated brine, dried over anhydrous sodium sulfate, filtered, concentrated, and purified by prep-TLC to give 4 (27 mg, 12%) as a white solid.

ESI m/z $[M-H]^-$ = 487.2. $^1$H NMR (400 MHz, DMSO-$d_6$) δ 8.41 (d, *J* = 1.5 Hz, 1H), 8.36 (dd, *J* = 8.1, 1.6 Hz, 1H), 7.89 (d, *J* = 8.1 Hz, 1H), 7.57 (t, *J* = 51.3 Hz, 1H), 7.11 (d, *J* = 8.7 Hz, 1H), 4.71 (d, *J* = 2.5 Hz, 2H), 3.52 = 3.37 (m, 2H), 2.18 (d, *J* = 12.9 Hz, 1H), 1.92 (d, *J* = 12.6 Hz, 1H), 1.72 (q, *J* = 13.2, 12.2 Hz, 3H), 1.58 - 1.44 (m, 1H), 1.37 - 1.22 (m, 3H), 0.92 - 0.75 (m, 4H).

**Example 5: (Compound 6)**

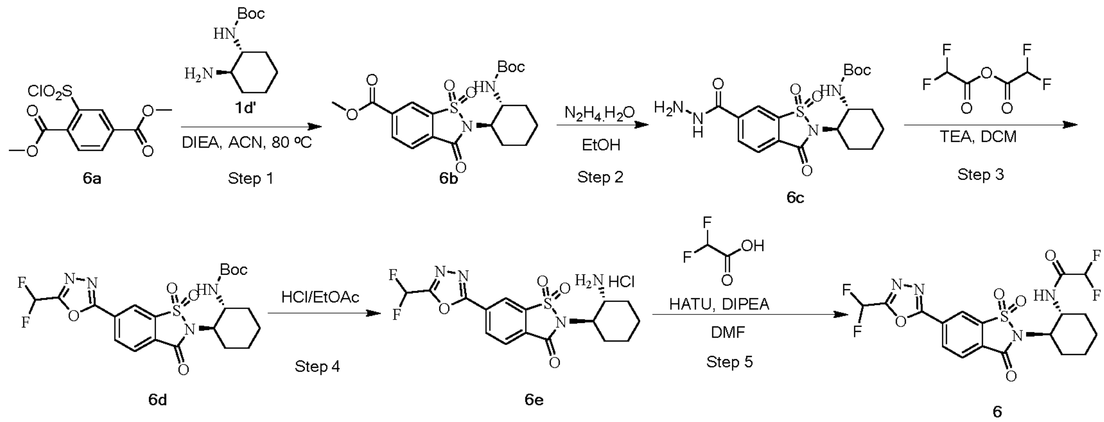


6a 6b 6c 6d 6e 6

Step 1 (compound **6b)**

Compound **6a** (1 g, 3.41 mmol) was dissolved in acetonitrile (20 mL), and diisopropylethylamine (1.1 g, 8.53 mmol) and compound **1d'** (730 mg, 3.41 mmol) were added. The mixture was stirred at room temperature for 30 min and then at 80 °C overnight. The reaction solution was washed with water, extracted with ethyl acetate, concentrated, and separated by column chromatography to give compound **6b** (1.2 g, 82%).

Steps 2-5 (compound **6)**

Referring to steps 5-8 of the synthetic route of Example 1, **1e** was replaced with compound **6b** in step 5 and difluoropropionic acid was replaced with difluoroacetic acid in step 8 to give **6** as a while solid.

ESI m/z $[M+H]^+$ = 477.0. $^1$H NMR (400 MHz, $CDCl_3$) δ 8.54 (s, 1H), 8.50 (d, *J*=8.0 Hz, 1 H), 8.17 (d, *J*=8.0 Hz, 1 H), 6.89 (t, *J=51.6* Hz, 1 H), 6.29 (d, *J=8.4* Hz, 1 H), 5.61 (t, J=54.4 Hz, 1 H), 4.62-4.60 (m, 1 H), 3.77-3.74 (m, 1 H), 2.50-2.41 (m, 1 H), 2.22-2.16 (m, 1 H), 2.04-2.01 (m, 1 H), 1.93-1.87 (m, 1 H), 1.81-1.78 (m, 1 H), 1.47-1.40 (m, 1 H), 1.37-1.28 (m, 2 H).

**Example 6: (Compound 7)**

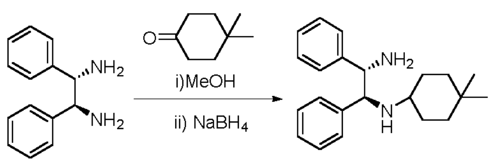


7a 7b

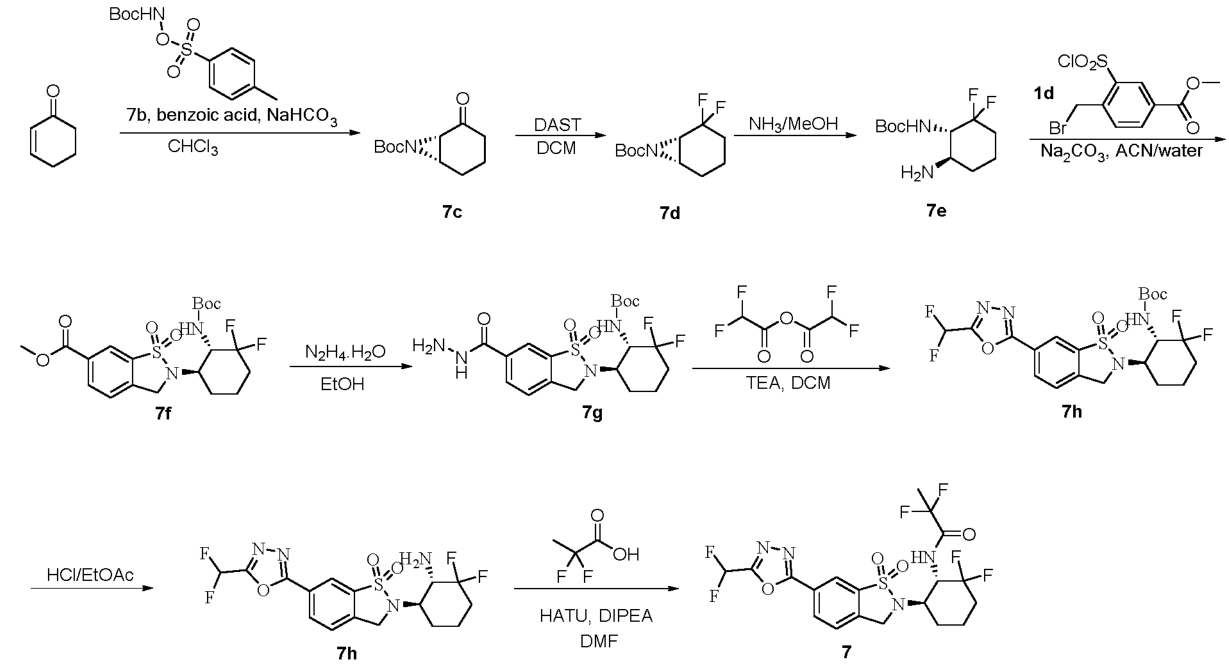


1. (Compound **7b)**

A solution of compound 7a (5.0 g, 23.6 mmol) and 4,4-dimethylcyclohexanone (5.9 g, 47.2 mmol) in methanol (100 mL) was stirred at room temperature for 1 h. The mixture was cooled to 0 °C, and sodium borohydride (3.6 g, 94.3 mmol) was added. The mixture was stirred at room temperature for 2 h. The reaction was quenched with water, and the reaction solution was extracted with ethyl acetate. The organic phase was dried, concentrated, and stirred in hydrogen chloride (4 M in dioxane, 20 mL) for 1 h. The mixture was filtered, and the filter cake was dissolved in water, neutralized with sodium bicarbonate, and extracted with ethyl acetate. The organic phase was dried and concentrated to give 7b (5 g, 66%) as a pale yellow oil.

$^1H$ NMR (400 MHz, $CDCl_3$) δ 7.21-7.12 (m, 10H), 4.01 (d, *J*= 7.2 Hz, 1H), 3.84 (d, *J*= 7.6 Hz,1H), 2.23-2.16 (m, 1H), 2.04 (s, 3H), 1.74-1.64 (m, 1H), 1.531.45 (m, 1H), 1.28-1.21 (m, 3H), 1.17-0.96 (m, 3H), 0.82 (d, *J*= 14.4 Hz, 6H).

2. (Compound **7c)**

To chloroform (200 mL) were added compound 7b (3.6 g, 11.1 mmol), N-Boc-O-p-toluenesulfonyl hydroxylamine (16.0 g, 55.7 mmol), sodium bicarbonate (23.4 g, 278.7 mmol), and benzoic acid (6.8 g, 55.7 mmol). The mixture was stirred at room temperature for 1 h, and cyclohexenone (16.0 g, 167.2 mmol) was added. The mixture was stirred at room temperature overnight. The reaction was quenched with water, and the reaction solution was extracted with dichloromethane. The organic phase was dried, concentrated, and separated by column chromatography to give **7c** (8.5 g, 64%) as a yellow oil.

$^1H$ NMR (400 MHz, $CDCl_3$) δ 3.10-3.05 (m, 1H), 2.89 (d, *J* = 5.6 Hz, 1H), 2.54-2.46 (m, 1H), 2.27-2.19 (m, 1H), 2.10-1.91 (m, 2H), 1.83-1.74 (m, 1H), 1.68-1.60 (m, 1H), 1.45 (s, 9H).

3. (Compound **7d**)

To a solution of compound 7c (2.5 g, 11.8 mmol) in dichloromethane (30 mL) was added DAST (12.3 g, 59.2 mmol) at 0 °C. The mixture was stirred at room temperature for 16 h. The reaction solution was poured into ice water and extracted with dichloromethane. The organic phase was dried, concentrated, and separated by column chromatography to give **7d** (5.0 g, 63%) as a yellow oil.

$^1H$ NMR (400 MHz, $CDCl_3$) δ 2.88-2.81 (m, 2H), 1.99-1.64 (m, 4H), 1.62-1.55 (m, 2H), 1.45 (s, 9H).

4. (Compound **7e**)

A solution of compound 7e (5.0 g, 21.5 mmol) in methanol/ammonia (100 mL) was stirred at room temperature for 3 days. The reaction solution was directly concentrated and separated by column chromatography to give **7e** (1.8 g, 33%) as a white solid.

ESI m/z $[M+H]^+$ = 251.1. $^1H$ NMR (400 MHz, DMSO-$d_6$) δ 6.89 (d, *J*=9.6 Hz, 1H), 3.50-3.35 (m, 1H), 2.62-2.53 (m, 1H), 2.06-1.93 (m, 1H), 1.85-1.59 (m, 3 H), 1.48 (s, 2H), 1.39 (s, 9H), 1.36-1.15 (m, 2H).

5. (Compound **7)**

Referring to the synthetic route for compound 1 in Example 1, **1d'** was replaced with compound **7e** to give **7** as a white solid.

ESI m/z $[M+H]^+$ = 512.9. $^1H$ NMR (400 MHz, $CDCl_3$) δ 8.50 (s, 1H), 8.38 (d, *J*=7.6 Hz, 1 H), 7.43 (d, *J*=8.4 Hz, 1 H), 7.06-6.78 (m, 2 H), 4.69 (d, *J*=14.4 Hz, 1 H), 4.50 (d, *J=14.0* Hz, 1 H), 4.44-4.33 (m, 1 H), 3.96-3.90 (m, 1 H), 2.38-2.34 (m, 1 H), 2.24-2.19 (m, 1 H), 1.97-1.92 (m, 1 H), 1.88-1.67 (m, 3 H), 1.56 (t,J=18.8 Hz, 3 H).

**Example 7: (Compound 10)**

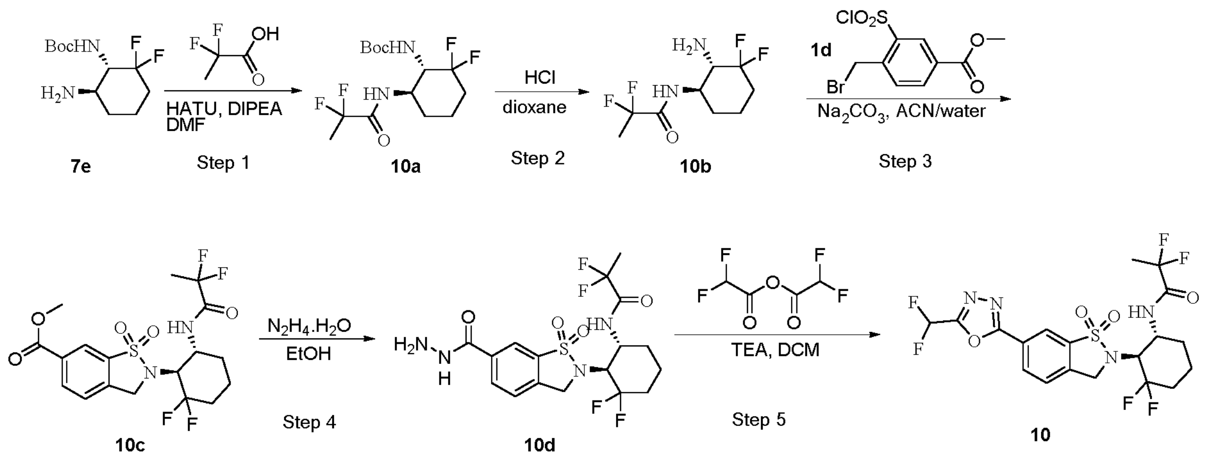


1. (Compound **10a)**

To a solution of 7e (0.2 g, 0.8 mmol) in DMF (1 mL) were added HATU (0.3 g, 0.8 mmol) and *N,N*-diisopropylethylamine (0.31 g, 2.4 mmol). The mixture was stirred at 25 °C for 30 min, and difluoropropionic acid (0.11 g, 0.96mmol) was added. The mixture was stirred at 20 °C for 16 h. Water was added to precipitate a solid, and the mixture was filtered to give 10a (0.2 g, 73%) as a white solid.

ESI m/z $[M+H]^+$ = 343.1.

2. (Compound **10b**)

To a solution of compound **10a** (150 mg, 0.44 mmol) in dioxane (5 mL) was added a solution of hydrogen chloride in dioxane (1 mL, 4 M). The mixture was stirred at room temperature for 3 h. The reaction solution was directly concentrated to give **10b** (0.1 g, 94%) as a yellow oil.

ESI m/z $[M+H]^+$ = 243.1.

5. (Compound **10)**

Referring to the synthetic route for compound **20** in Example 1, **1d'** was replaced with compound **10b** in step 4 to give **10** as a white solid.

ESI m/z $[M-H]^-$ = 511.1. $^1H$ NMR (400 MHz, DMSO-$d_6$) δ 8.70 (d, *J* = 9.2 Hz, 1H), 8.48 (d, *J* = 1.5 Hz, 1H), 8.40 (dd, *J* = 8.1, 1.6 Hz, 1H), 7.95 (d, *J* = 8.1 Hz, 1H), 7.60 (t, *J* = 51.3 Hz, 1H), 4.88 - 4.73 (m, 2H), 4.30 - 4.19 (m, 1H), 4.18 -4.04 (m, 1H), 2.25 (s, 1H), 2.02 - 1.83 (m, 3H), 1.54 (d, *J* = 13.9 Hz, 2H), 1.38 (d, *J* = 19.2 Hz, 3H).

**Example 8: (Compound 21)**

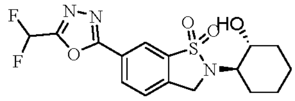

21

Referring to the synthetic route for compound 20 in Example 1, **1d'** was replaced with (1R,2R)-2-aminocyclohexan-1-ol in step 4 to give 21 as a white solid.

ESI m/z $[M-H]^-$ = 384.1. $^1H$ NMR (400 MHz, DMSO-$d_6$) δ 8.40 - 8.33 (m, 2H), 7.88 (d, *J* = 8.1 Hz, 1H), 7.56 (t, *J* = 51.3 Hz, 1H), 4.84 (d, *J* = 5.0 Hz, 1H), 4.72 - 4.59 (m, 2H), 3.59 - 3.49 (m, 2H), 1.94 (dd, *J* = 27.5, 11.9 Hz, 2H), 1.65 (d, *J* = 15.7 Hz, 2H), 1.37 - 1.14 (m, 4H).

**Example 9: (Compound 24)**

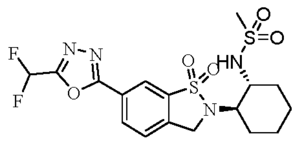

24

Referring to the synthetic method for compound **4** in Example 4, cyclopropylsulfonyl chloride was replaced with methanesulfonyl chloride to give **24** as a white solid.

ESI m/z $[M+H]^+$ = 463.2. $^1H$ NMR (400 MHz, DMSO-$d_6$) δ 8.44 (d, *J* = 1.5 Hz, 1H), 8.39 (dd, *J* = 8.1, 1.6 Hz, 1H), 7.92 (d, *J* = 8.2 Hz, 1H), 7.60 (t, *J* = 51.3 Hz, 1H), 7.13 (d, *J* = 8.3 Hz, 1H), 4.72 (s, 2H), 3.52 - 3.39 (m, 2H), 2.84 (s, 3H), 2.17 - 2.06 (m, 1H), 1.99 - 1.63 (m, 4H), 1.47 (dd, *J* = 18.2, 7.6 Hz, 1H), 1.31 (q, *J* = 16.0, 13.4 Hz, 2H).

**Example 10: (Compound 39)**

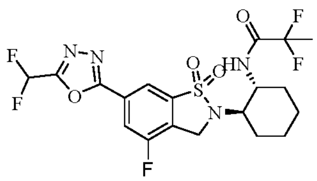

39

Referring to the synthetic method for compound **1** in Example 1, *p*-methylbenzoic acid was replaced with methyl 3-fluoro-4-methylbenzoate in step 1 to give **39** as a white solid.

ESI m/z $[M+H]^+$ = 494.9. $^1H$ NMR (400 MHz, DMSO-$d_6$) δ 8.58 (d, *J*=9.6 Hz, 1 H), 8.30 (d, *J*=0.8 Hz, 1 H), 8.25-8.22 (m, 1 H), 7.56 (d, *J*=51.2 Hz, 1 H), 4.77-4.66 (m, 2 H), 3.96-3.93 (m, 1 H), 3.65-3.61 (m, 1 H), 1.86-1.70 (m, 6 H), 1.54-1.44 (m, 3 H), 1.34-1.23 (m, 2 H).

**Example 11: (Compound 40)**

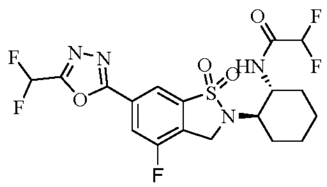

40

Referring to the synthetic method for compound **1** in Example 1, p-methylbenzoic acid was replaced with methyl

3-fluoro-4-methylbenzoate in step 1 and difluoropropionic acid was replaced with difluoroacetic acid in step 8 to give **40** as a white solid.

ESI m/z $[M+H_2O]^+$ = 498.0. $^1H$ NMR (400 MHz, DMSO-$d_6$) δ 8.73 (d, *J*=9.2 Hz, 1 H), 8.29 (d, *J*=0.8 Hz, 1 H), 8.23 (d, *J*=9.2 Hz, 1 H), 7.57 (d, *J=51.2* Hz, 1 H), 6.00 (d, *J*=53.6 Hz, 1 H), 4.78-4.67 (m, 2 H), 3.98-3.93 (m, 1 H), 3.61-3.55 (m, 1 H), 1.87-1.63 (m, 6 H), 1.35-1.23 (m, 2 H).

**Example 12: (Compound 47)**

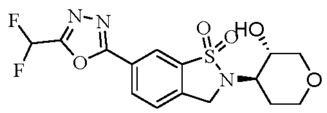

**47**

Referring to the synthetic method for compound **20** in Example 1, **1d'** was replaced with (3*R*,4*R*)-4-amino-3-hydroxytetrahydropyran in step 4 to give **47** as a white solid.

ESI m/z $[M+H]^+$ = 387.9. $^1H$ NMR (400 MHz, DMSO-$d_6$) δ 8.40 (s, 1H), 8.38-8.36 (m, 1 H), 7.89 (d, *J*=8.4 Hz, 1 H), 7.70-7.44 (m, 1 H), 5.23 (d, *J*=5.2 Hz, 1 H), 4.77 (d, *J*=15.6 Hz, 1 H), 4.65 (d, *J*=15.6 Hz, 1 H), 3.91-3.82 (m, 2 H), 3.69-3.57 (m, 2 H), 3.42-3.34 (m, 1 H), 3.15-3.09 (m, 1 H), 1.94-1.90 (m, 2 H).

**Example 13: (Compound 49)**

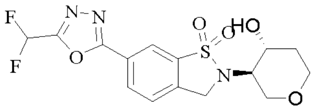

**49**

Referring to the synthetic method for compound **20** in Example 1, **1d'** was replaced with (3R,4R)-3-amino-4-hydroxytetrahydropyran in step 4 to give **49** as a white solid.

ESI m/z $[M+H]^+$ = 387.9. $^1H$ NMR (400 MHz, DMSO-$d_6$) δ 8.40 (s, 1H), 8.38-8.36 (m, 1 H), 7.89 (d, *J*=8.4 Hz, 1 H), 7.70-7.44 (m, 1 H), 5.23 (d, *J*=5.2 Hz, 1 H), 4.77 (d, *J*=15.6 Hz, 1 H), 4.65 (d, *J*=15.6 Hz, 1 H), 3.91-3.82 (m, 2 H), 3.69-3.57 (m, 2 H), 3.42-3.34 (m, 1 H), 3.15-3.09 (m, 1 H), 1.94-1.90 (m, 2 H).

**Example 14: (Compound 55)**

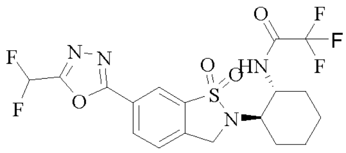

**55**

Referring to the synthetic method for compound2 in Example 2, difluoroacetic anhydride was replaced with trifluoroacetic anhydride to give **55** as a white solid.

ESI m/z $[M-H]^-$ = 479.2. $^1H$ NMR (400 MHz, DMSO-$d_6$) δ 9.36 (d, J = 9.1 Hz, 1H), 8.46 - 8.33 (m, 2H), 7.93 (d, J = 8.1 Hz, 1H), 7.60 (t, J = 51.3 Hz, 1H), 4.73 (d, J = 15.5 Hz, 1H), 4.63 (d, J = 15.4 Hz, 1H), 4.03 - 3.90 (m,1H), 3.69 - 3.59 (m, 1H), 1.82 (d, J = 17.0 Hz, 4H), 1.73 (d, J = 11.0 Hz, 2H), 1.36 (d, J = 10.4 Hz, 2H).

**Example 15: (Compound 60)**

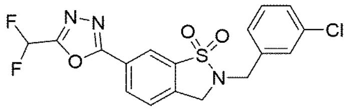

Referring to the synthetic method for compound 20 in Example 1, **1d'** was replaced with 3-chlorobenzylamine to give **60** as a white solid.

ESI m/z [M-H]$^-$ = 410.0. $^1$H NMR (400 MHz, DMSO-$d_6$) δ 8.51 (d, *J* = 1.5 Hz, 1H), 8.41 (dd, *J* = 8.1, 1.6 Hz, 1H), 7.90 - 7.86 (m, 1H), 7.75 - 7.45 (m, 5H), 4.54 (s, 2H), 4.53 (s, 2H).

**Example 16: (Compound 61)**

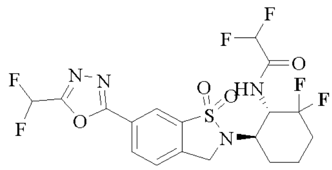

**61**

Referring to the synthetic method for compound 1 in Example 1, **1d'** was replaced with **7e** in step 4 and difluoropropionic acid was replaced with difluoroacetic acid in step 8 to give**61** as a white solid.

ESI m/z [M+H]$^+$ = 499.1. $^1$H NMR (400 MHz, DMSO-$d_6$) δ 9.28 (d, *J* = 9.7 Hz, 1H), 8.44 (d, *J* = 1.5 Hz, 1H), 8.39 (dd, *J* = 8.1, 1.6 Hz, 1H), 7.94 (d, *J* = 8.2 Hz, 1H), 7.60 (t, *J* = 51.3 Hz, 1H), 6.19 (t, *J* = 53.7 Hz, 1H), 4.78 (d, *J* = 15.6 Hz, 1H), 4.74-4.62 (m, 1H), 4.57 (d, *J* = 15.5 Hz, 1H), 3.77 (td, *J* = 11.7, 3.9 Hz, 1H), 2.26 - 1.99 (m, 3H), 1.87 (d, *J* = 13.1 Hz, 2H), 1.64 - 1.48 (m, 1H).

**Example 17: (Compound 62)**

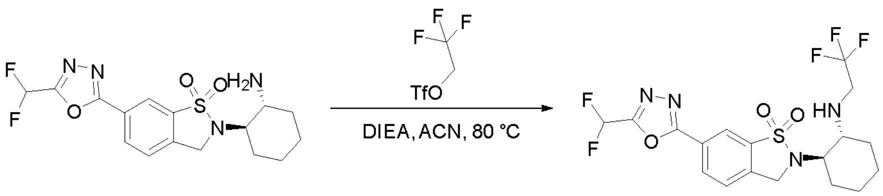


**108** **62**

To a solution of compound **108** (100 mg, 0.65 mol) in acetonitrile (2 mL) were added *N,N*-diisopropylethylamine (100 mg, 0.78 mmol) and 2,2,2-trifluoroethyl trifluoromethanesulfonate (91 mg, 0.39 mmol) at 25 °C under stirring. Then the mixture was reacted at 80 °C for 16 h. The reaction solution was subjected to reversed-phase column chromatography to give **62** (50 mg, 41%) as a white solid.

ESI m/z [M+H]$^+$ = 467.2. $^1$H NMR (400 MHz, DMSO-$d_6$) δ 8.45 (d, J = 1.5 Hz, 1H), 8.40 (dd, J = 8.1, 1.6 Hz, 1H), 7.91 (d, J = 8.1 Hz, 1H), 7.60 (t, J = 51.3 Hz, 1H), 4.75- 4.61 (m, 2H), 3.48 (t, J = 9.0 Hz, 1H), 3.28 (s, 1H), 2.79 - 2.66 (m, 1H), 2.48 - 2.34 (m, 1H), 2.14- 2.02 (m, 1H), 1.91 (d, J = 11.6 Hz, 1H), 1.72 (s, 3H), 1.38 (d, J = 12.2 Hz, 1H), 1.23 (d, J = 9.4 Hz, 1H), 1.03 (d, J = 6.4 Hz, 1H).

**Example 18: (Compounds 70i-A, 70i-B, 70i-B-A, and 70i-B-B)**

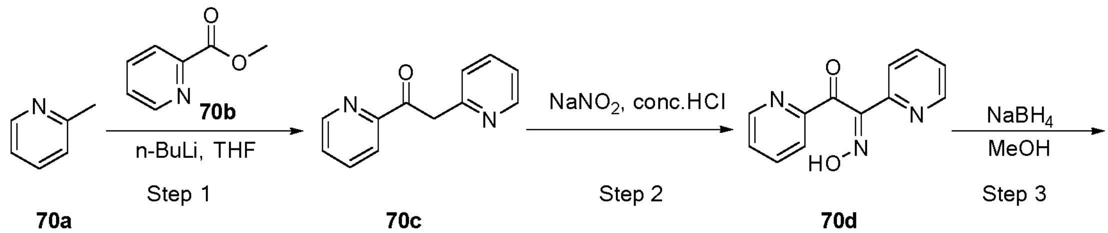


70a 70c 70d

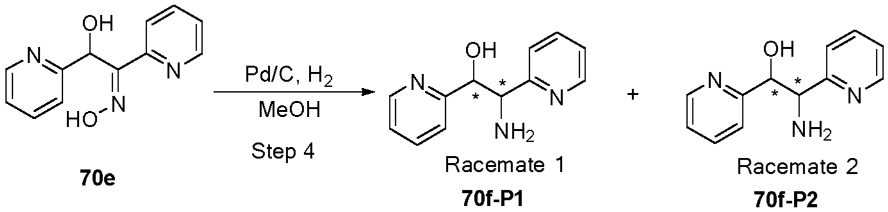


70e 70f-P1 70f-P2

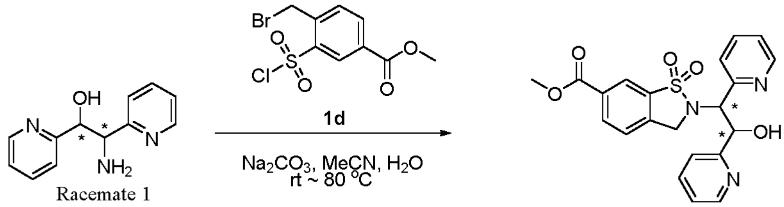


70f-P1 70g-A

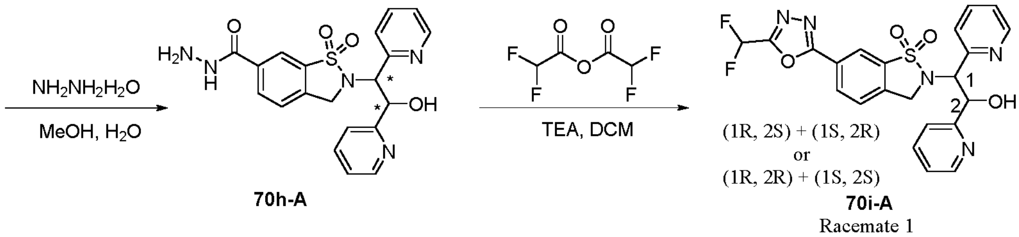


70h-A 70i-A

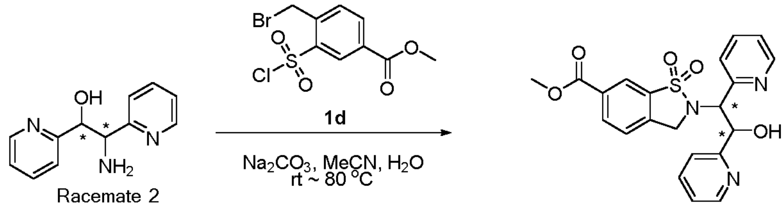


70f-P2 70g-B

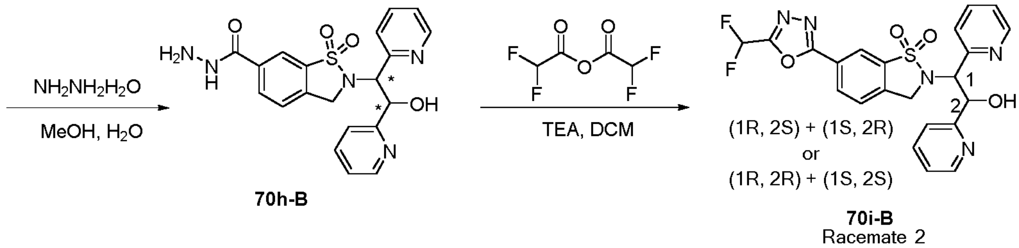


70h-B 70i-B

70i-B SFC 70i-B-A + 70i-B-B

1. (Compound **70c**)

To a solution of compound **70a** (2.34 g, 25.25 mmol) in tetrahydrofuran (40 mL) was added *n*-butyllithium (16.8 mL, 1.5 M in THF) at -78 °C. The mixture was stirred at this temperature for 1 h. Then a solution of compound **70b** (3.46 g, 25.25 mmol) in tetrahydrofuran (20 mL) was slowly added dropwise. The mixture was stirred at this temperature for 3 h, then heated to room temperature and stirred overnight. The reaction was quenched with dilute hydrochloric acid, and the reaction solution was extracted with dichloromethane, concentrated, and separated by column chromatography to give compound **70c** (2.0g, 40%) as a white solid.

2. (Compound **70d**)

To hydrochloric acid (6 M, 20 mL) was added compound **70c** (1.94 g, 9.78 mmol) at 0 °C, and an aqueous solution (5 mL) of sodium nitrite (1.69 g, 24.5 mmol) was slowly added. The mixture was stirred at room temperature for 2 h. The reaction solution was extracted with dichloromethane, concentrated, and separated by column chromatography to give **70d** (1.51 g, 68%) as a yellow solid.

3. (Compound **70c**)

To a solution of compound **70d** (1.51 g, 6.68 mmol) in methanol (40 mL) was added sodium borohydride (304 mg, 8 mmol) at 0 °C. The mixture was stirred for 2 h. The reaction solution was washed with water, extracted with dichloromethane, and concentrated to give **70e** (1.5 g, 98%) as a yellow solid.

4. (Compounds **70f-P1** and **70f-P2)**

A solution of compound **70e** (1.5 g, 6.5 mmol) and Pd/C (750 mg, 10%) in methanol (200 mL) was stirred at 70 °C under hydrogen atmosphere (4.0 MPa) for 2 days. The reaction solution was filtered, and the filtrate was separated by column chromatography to give **70f-P1** (400 mg, 27%) and **70f-P2** (400 mg, 27%) as yellow oils.

**70f-P1:** $^1$H NMR (400 MHz, DMSO-$d_6$): δ 8.51-8.47 (m, 2 H), 7.79-7.68 (m, 2 H), 7.46 (d, *J*=10.4 Hz, 1 H), 4.79 (d, *J*= 10.4 Hz, 1 H), 7.26-7.20 (m, 2 H), 5.47 (s, 1 H), 4.91 (d, *J*=4.8 Hz, 1 H), 4.25 (d, *J*=5.2 Hz, 1 H), 1.94 (s, 2 H).
**70f-P2:** $^1$H NMR (400 MHz, DMSO-$d_6$): δ 8.48-8.41 (m, 2 H), 7.68-7.59 (m, 2 H), 7.23-7.15 (m, 4 H), 5.46 (s, 1 H), 4.79 (d, *J=6.0* Hz, 1 H), 4.19 (d, *J*=6.4 Hz, 1 H), 2.04 (s, 2 H).

5. (Compound **70i-A**)

Referring to the synthetic route for compound **20** in Example 1, **1d'** was replaced with compound **70f-P1** to give 70i-A as a white solid, which was a racemate.
ESI m/z [M+H]$^+$ = 485.9. $^1$H NMR (400 MHz, DMSO-$d_6$) δ 8.51-8.47 (m, 2 H), 8.35-8.33 (m, 2 H), 7.93 (d, *J*=8.4 Hz, 1 H), 7.70-7.43 (m, 4 H), 7.24-7.18 (m, 3 H), 5.96 (d, *J*=5.6 Hz, 1 H), 5.61-5.58 (m, 1 H), 5.44 (d, *J*=6.0 Hz, 1 H), 5.14-5.01 (m, 2 H).

6. (Compound **70i-B**)

Referring to the synthetic route for compound **20** in Example 1, **1d'** was replaced with compound **70f-P2** to give **70i-B** as a white solid, which was a racemate.
ESI m/z [M+H]$^+$ = 485.9. $^1$H NMR (400 MHz, DMSO-$d_6$) δ 8.58 (d, *J*=4.0 Hz, 1 H), 8.38 (d, *J*=4.4 Hz, 1 H), 8.30-8.28 (m, 1 H), 8.24 (s, 1 H), 7.89 (d, *J*=8.4 Hz, 1 H), 7.80-7.74 (m, 2 H), 7.66-7.40 (m, 3 H), 7.37-7.31 (m, 1 H), 7.22-7.19 (m, 1 H), 6.99 (d, *J*=6.0 Hz, 1 H), 5.47-5.44 (m, 1 H), 5.18 (d, *J*=8.4 Hz, 1 H), 5.14-5.02 (m, 2 H).

7. (Compounds **70i-B-A** and **70i-B-B**)

Compound **70i-B** (240 mg, 0.49 mmol) was subjected to chiral resolution (column: IC, mobile phase: Hex/-EtOH/TFA 50/50/0.3, flow rate: 25 mL/min, and detection wavelength: 254 nM) to give compound **70i-B-A** (retention time: 21.264 min, 50 mg, 21%) and compound **70i-B-B** (retention time: 29.823 min, 50 mg, 21%) as white solids, both of which were chirally pure compounds.

**70i-B-A:** ESI m/z [M+H]$^+$ = 486.0. $^1$H NMR (400 MHz, DMSO-$d_6$) δ 8.59 (s, 1 H), 8.40 (s, 1 H), 8.29 (d, *J*=8.0 Hz, 1 H), 8.25 (s, 1 H), 7.89(d, J=8.4 Hz, 1 H), 7.80-7.74(m, 2 H), 7.66-7.63(m, 1 H), 7.53-7.41(m, 2 H), 7.33(m, 1 H), 7.23(m, 1

H), 6.03(s, 1 H), 5.47 (d, *J*=7.2 Hz, 1 H), 5.19-5.00 (m, 3 H).

**70i-B-B:** ESI m/z $[M+H]^+$ = 485.9. $^1H$ NMR (400 MHz, DMSO-$d_6$) δ 8.58 (d, *J*=4.0 Hz, 1 H), 8.38 (d, *J*=4.4 Hz, 1 H), 8.30-8.28 (m, 1 H), 8.25 (s, 1 H), 7.89(d, *J*=8.0 Hz, 1 H), 7.80-7.75(m, 2 H), 7.66-7.41(m, 3 H), 7.34-7.31(m, 1 H), 7.23-7.20(m, 1 H), 6.01(s, 1 H), 5.46(d, *J*=8.0 Hz, 1 H), 5.20-5.02 (m, 3 H).

**Example 19: (Compound 72)**

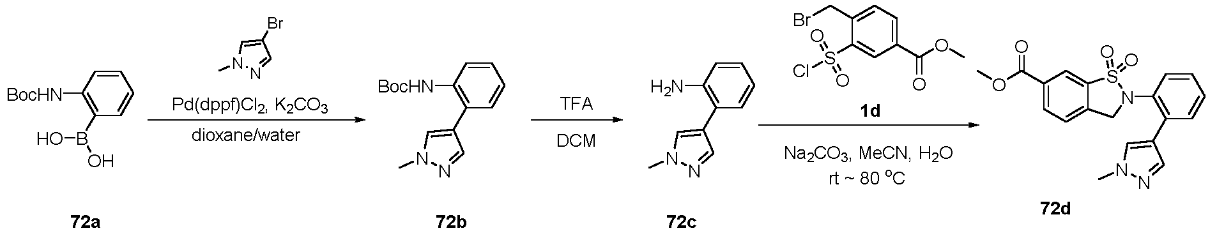


**72a** **72b** **72c** **72d**

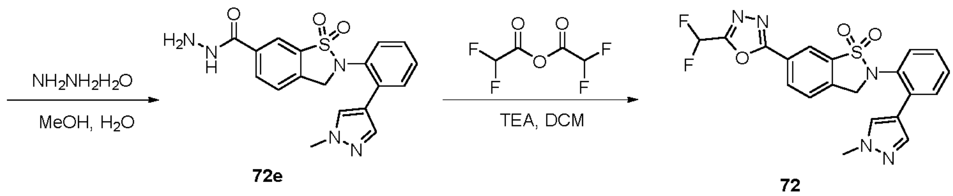


**72e** **72**

1. (Compound **72b**)

Compound **72a** (1 g, 6.21 mmol), 4-bromo-1-methyl-1H-pyrazole (1.98 g, 6.21 mmol), potassium carbonate (2.57 g, 18.6 mmol), and 1,1'-bis(diphenylphosphino)ferrocenepalladium dichloride (0.55 g, 0.75 mmol) were added to a mixed solution of dioxane (5 mL) and water (1 mL). The mixture was reacted with stirring at 80 °C for 16 h. The reaction solution was poured into water, extracted with dichloromethane, concentrated, and purified by column chromatography to give **72b** (900 mg, 53%) as a white solid.

ESI m/z $[M+H]^+$ = 274.1.

2. (Compound **72c)**

Compound **72b** (500 mg, 2.12 mmol) was added to a dichloromethane solution (5 mL), and trifluoroacetic acid (2 mL) was added with stirring. The mixture was reacted for 2 h. The reaction solution was concentrated to give **72c** (300 mg, 95%) as a yellow oil.

3. (Compound **72**)

Referring to the synthetic route for compound **20** in Example 1, **1d'** was replaced with compound **72c** for synthesis over three steps to give **72** as a white solid.

ESI m/z $[M+H]^+$ = 444.2. $^1H$ NMR (400 MHz, DMSO-$d_6$) δ 8.61 - 8.57 (m, 1H), 8.46 (dd,J = 8.1, 1.6 Hz, 1H), 8.06 (s, 1H), 7.91 (d,J = 8.2 Hz, 1H), 7.79 (s, 1H), 7.77 - 7.61 (m, 3H), 7.54 (td,J = 7.6, 1.4 Hz, 1H), 7.43 (td,J = 7.6, 1.6 Hz, 1H), 4.83 (s, 2H), 3.81 (s, 3H).

**Example 20: (Compounds 79i, 79i-A, and 79i-B)**

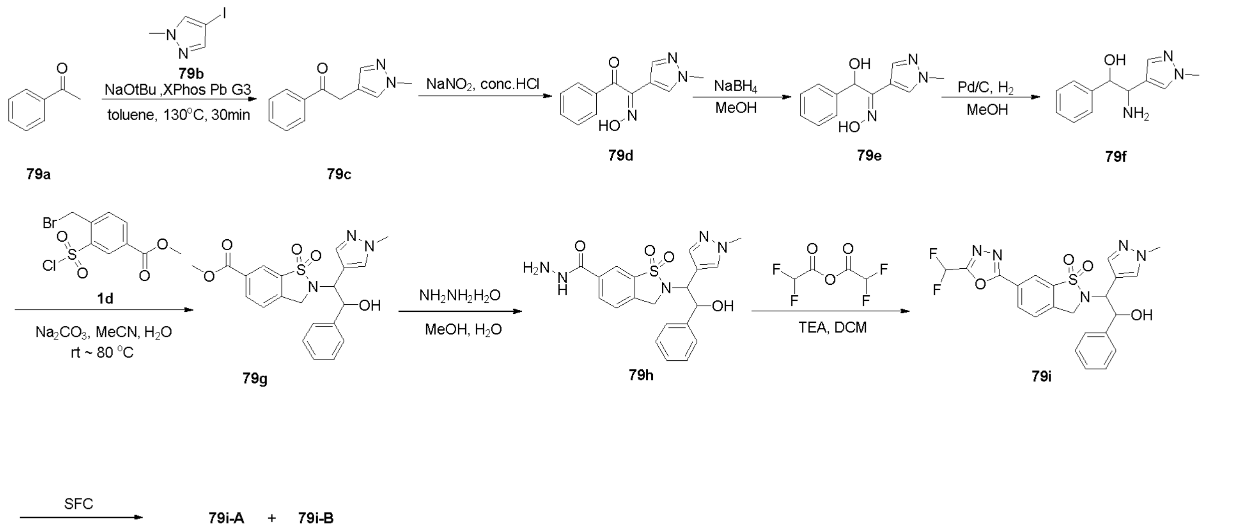


1. (Compound **79c)**

A mixture of compound **79a** (2 g, 16.6 mmol), sodium tert-butoxide (3.8 g, 39.6 mmol), and XPhosPd-G3 (1.41 g, 1.66 mmol) in toluene (20 mL) was stirred under nitrogen atmosphere for 30 min, and then compound **79b** (3.8 g, 18.2 mmol) was added. The mixture was reacted at 130 °C under microwave irradiation for 30 min. The reaction solution was washed with water and extracted with ethyl acetate. The organic phase was concentrated and separated by column chromatography to give **79c** (1.05 g, 32%) as a white solid.

ESI m/z $[M+H]^+$ = 201.0.

2. (Compound **79f**)

Referring to the synthetic route for 70f in Example 18, **70c** was replaced with **79c** for synthesis over three steps to give 79f. The isomers of 79f were not separated, and the mixture was used in the next step.

3. (Compounds **79i, 79i-A,** and **79i-B)**

Referring to the synthesis conditions for compound 70i-A in Example 18, **70f-P1** was replaced with **79f for** synthesis over 3 steps to give a mixture **79i,** which was subjected to chiral resolution (column: IBN, mobile phase: Hex/EtOH 50/50, flow rate: 25 mL/min, and detection wavelength: 254 nM) to give **79i-A** (isomer A, retention time: 15.494 min) and **79i-B** (isomer B, retention time: 18.437 min) as white solids, both of which were chirally pure compounds. Meanwhile, the other two isomers 79i-C (retention time: 29.632 min) and 79i-D (retention time: 35.885 min) with lower yields were obtained.

**79i-A** (isomer A): ESI m/z $[M+H]^+$ = 488.0. $^1$H NMR (400 MHz, DMSO-$d_6$) δ 8.38 (s, 1 H), 8.32(d, *J*=8.0 Hz, 1 H), 7.84(d, *J*=8.4 Hz, 1 H), 7.72 (s, 1 H), 7.69-7.44(m, 2 H), 7.35-7.33(m, 2 H), 7.28-7.25(m, 2 H), 7.19-7.18(m, 1 H), 6.01(d, *J*=4.8 Hz, 1 H), 5.25-5.23(m, 1 H), 4.98(d, *J*=4.0 Hz, 1 H), 4.89(d, *J*=16.0 Hz, 1 H), 4.37(d, *J*=16.4 Hz, 1 H), 3.74 (s, 3 H).
**79i-B** (isomer B): ESI m/z $[M+H]^+$ = 488.0. $^1$H NMR (400 MHz, DMSO-$d_6$) δ 8.38 (s, 1 H), 8.33-8.31(m, 1 H), 7.84(d, *J*=8.4 Hz, 1 H), 7.72 (s, 1 H), 7.69-7.44(m, 2 H), 7.35-7.34(m, 2 H), 7.28-7.25(m, 2 H), 7.19-7.18(m, 1 H), 6.02(d, J=4.4 Hz, 1 H), 5.26-5.23(m, 1 H), 4.98(d, *J*=4.4 Hz, 1 H), 4.89(d, *J*=16.0 Hz, 1 H), 4.37(d, *J*=16.0 Hz, 1 H), 3.74 (s, 3 H).

**Example 21: (Compounds 84j-A, 84j-B, 84j-A-A, and 84j-A-B)**

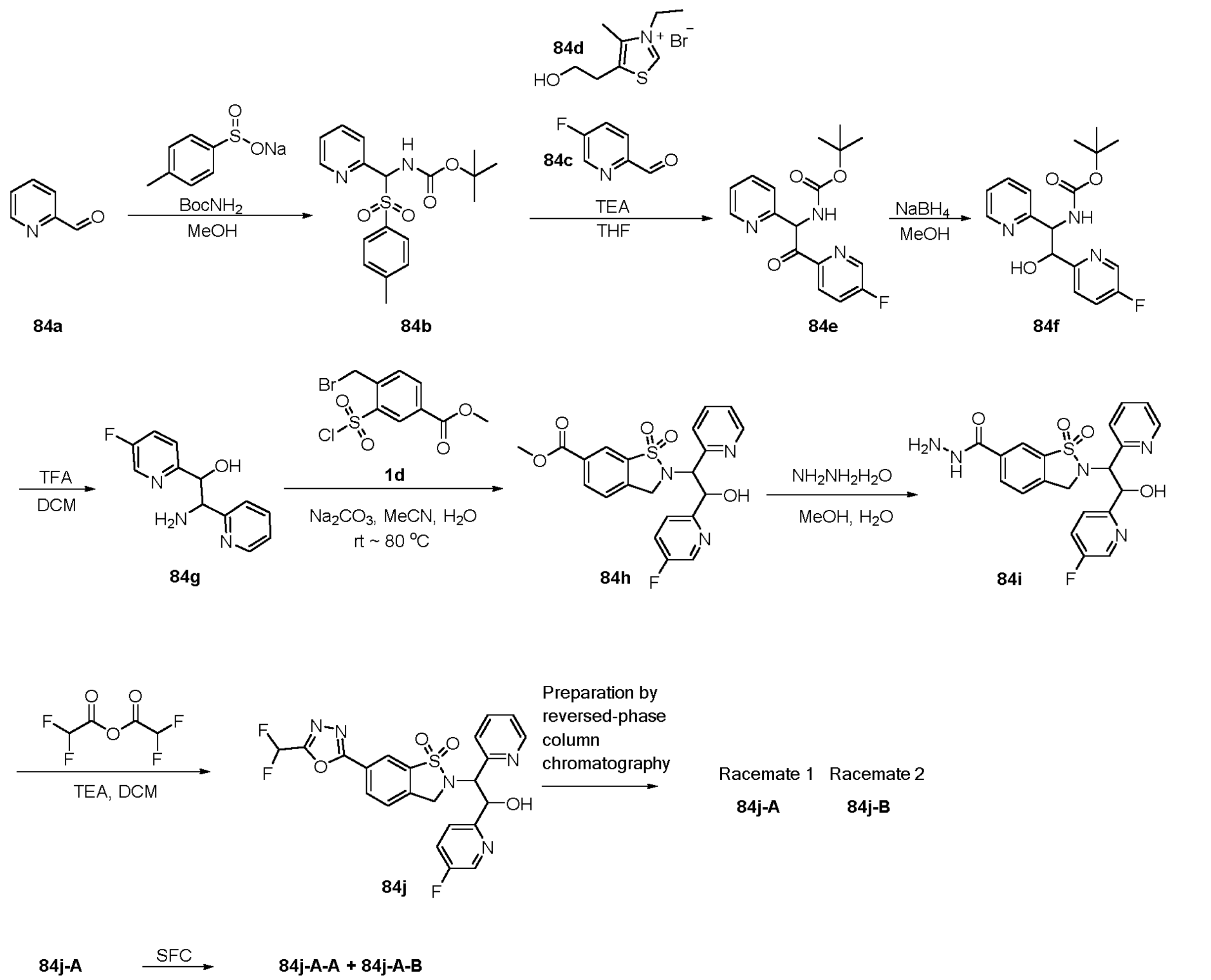


1. (Compound **84b)**

To an aqueous solution (14 mL) of *tert*-butyl carbamate (1.09 g, 9.3 mmol) and sodium p-toluenesulfonate (2.5 g, 14.0 mmol) was slowly added a solution of compound 84a (1.0 g, 9.3 mmol) in methanol (7 mL). The mixture was stirred at room temperature for half an hour, and formic acid (0.72 mL) was added. The mixture was stirred at room temperature for 3 days. The reaction solution was directly filtered, and the filter cake was washed with water and dried to give **84b** (1.7 g, 50%) as a white solid.

2. (Compound **84e)**

To a solution of compound **84c** (125 mg, 4.47 mmol), compound **84b** (1.7 g, 4.7 mmol), and compound **84d** (226 mg, 0.89 mmol) in tetrahydrofuran (20 mL) was added triethylamine (7.1 g, 70.3 mmol). The mixture was stirred at 60 °C under nitrogen atmosphere for 16 h. The reaction solution was cooled to 0 °C, treated with saturated ammonium chloride, extracted with ethyl acetate, dried over sodium sulfate, and separated by column chromatography to give **84e** (1.05 g, 70%) as a white solid.
ESI m/z $[M+H]^+$ = 332.1.

3. (Compound **84f**)

To a solution of compound **84e** (1.84 g, 5.55 mmol) in methanol (40 mL) was slowly added sodium borohydride (253 mg, 6.67 mmol). The mixture was stirred at 0 °C for 1 h. A small amount of water was added to quench excess sodium borohydride, and the reaction solution was directly concentrated and separated by column chromatography to give **84f** (1.75 g, 94%) as a yellow solid.
ESI m/z $[M+H]^+$ = 334.1.

4. (Compound **84g)**

To a solution of compound **84f** (750 mg, 2.25 mmol) in dichloromethane (10 mL) was slowly added trifluoroacetic acid (5 mL). The mixture was stirred at room temperature for 1 h. The reaction solution was directly concentrated to give **84g** (781 mg, trifluoroacetate) as a yellow oil.
ESI m/z $[M+H]^+$ = 234.1.

5. (Compounds **84j-A** and **84j-B**)

Referring to the synthetic route for compound **20** in Example 1, **1d'** was replaced with compound **84g** for synthesis to give 84j, which was separated by reversed-phase preparative chromatography to give diastereoisomers **84j-A** and **84j-B** as white solids.

**84j-A** (isomer A): ESI m/z $[M+H]^+$ = 504.0. $^1$H NMR (400 MHz, DMSO-$d_6$) δ 8.60 (d, *J*=4.0 Hz, 1 H), 8.36 (s, 1 H), 8.30-8.28 (m, 1 H), 8.25 (s, 1 H), 7.89 (d, *J*=8.0 Hz, 1 H), 7.82-7.78 (m, 1 H), 7.72-7.70 (m, 2 H), 7.66-7.36 (m, 2 H), 7.34-7.32 (m, 1 H), 6.03 (d, *J*=6.0 Hz, 1 H), 5.52-5.48 (m, 1 H), 5.12 (d, *J*=8.8 Hz, 1 H), 5.09-4.98 (m, 2 H).
**84j-B** (isomer B): ESI m/z $[M+H]^+$ = 503.9. $^1$H NMR (400 MHz, DMSO-$d_6$) δ 8.53-8.47 (m, 2 H), 8.35-8.33 (m, 2 H), 7.93-7.91 (m, 1 H), 7.71-7.43 (m, 4 H), 7.27-7.21 (m, 2 H), 6.08 (d, *J*=5.2 Hz, 1 H), 5.67-5.64 (m, 1 H), 5.38 (d, *J*=5.6 Hz, 1 H), 5.10-4.99 (m, 2 H).

6. (Compounds **84j-A-A** and **84j-A-B**)

Compound **84j-A** (100 mg) was subjected to chiral resolution (column: IC, mobile phase: Hex/EtOH/TFA 40/60/0.3, flow rate: 25 mL/min, and detection wavelength: 254 nM) to give compound **84j-A-A** (retention time: 15.293 min, 30 mg, 30%) and compound **84j-A-B** (retention time: 21.500 min, 30 mg, 30%) as white solids, both of which were chirally pure compounds.

**84j-A-A** (isomer A-A): ESI m/z $[M+H]^+$ = 504.0. $^1$H NMR (400 MHz, DMSO-$d_6$) δ 8.60 (d, *J*=4.0 Hz, 1 H), 8.36 (s, 1 H), 8.30-8.28 (m, 1 H), 8.25 (s, 1 H), 7.88 (d, *J*=8.0 Hz, 1 H), 7.82-7.77 (m, 1 H), 7.72-7.70 (m, 2 H), 7.66-7.41 (m, 2 H), 7.35-7.32 (m, 1 H), 6.03 (d, *J*=6.0 Hz, 1 H), 5.52-5.48 (m, 1 H), 5.11 (d, *J*=8.4 Hz, 1 H), 5.09-4.98 (m, 2 H).
**84j-A-B** (isomer A-B): ESI m/z $[M+H]^+$ = 504.0. $^1$H NMR (400 MHz, DMSO-$d_6$) δ 8.60 (d, *J*=4.0 Hz, 1 H), 8.36 (s, 1 H), 8.30-8.28 (m, 1 H), 8.25 (s, 1 H), 7.89 (d, *J*=8.0 Hz, 1 H), 7.82-7.78 (m, 1 H), 7.72-7.70 (m, 2 H), 7.66-7.41 (m, 2 H), 7.35-7.32 (m, 1 H), 6.03 (d, *J*=6.0 Hz, 1 H), 5.52-5.48 (m, 1 H), 5.13-4.98 (m, 3 H).

**Example 22: (Compounds 88j-A, 88j-B, 88j-A-A, and 88j-A-B)**

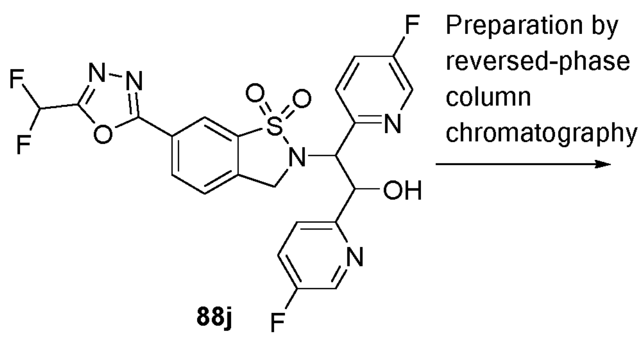


88j

Racemate 1 Racemate 2

**88j-A** **88j-B**

**88j-A** SFC **88j-A-A + 88j-A-B**

1. (Compounds **88j-A** and **88j-B**)

Referring to the synthetic route for compound 84j in Example 21, 84a was replaced with the compound 5-fluoropyridine-2-formaldehyde, and finally, the product was separated by reversed-phase preparative chromatography to

give diastereoisomers **88j-A** and **88j-B** as white solids.

**88j-A** (isomer A): ESI m/z $[M+H]^+$ = 521.9. $^1$H NMR (400 MHz, DMSO-$d_6$) δ 8.59 (d, *J*=3.2 Hz, 1 H), 8.37 (d, *J*=1.6 Hz, 1 H), 8.31-8.29 (m, 1 H), 8.26 (s, 1 H), 7.88 (d, *J*=8.4 Hz, 1 H), 7.77-7.70 (m, 3 H), 7.67-7.41(m, 2 H), 6.02 (d, *J*=5.6 Hz, 1 H), 5.49-5.45 (m, 1 H), 5.17 (d, *J*=8.8 Hz, 1 H), 5.07-4.96 (m, 2 H).

**88j-B** (isomer B): ESI m/z $[M+H]^+$ = 522.1. $^1$H NMR (400 MHz, DMSO-$d_6$) δ 8.51 (d, *J*=3.2 Hz, 1 H), 8.47 (d, *J*=2.8 Hz, 1 H), 8.36-8.34 (m, 2 H), 7.94 (d, *J*=8.4 Hz, 1 H), 7.69-7.43 (m, 4 H), 7.35-7.32 (m, 1 H), 6.10 (d, *J*=5.2 Hz, 1 H), 5.62-5.59 (m, 1 H), 5.42 (d, *J*=6.0 Hz, 1 H), 5.09-4.99 (m, 2 H).

2. (Compounds **88j-A-A** and **88j-A-B**)

Compound **88j-A** (100 mg) was subjected to chiral resolution (column: IC, mobile phase: Hex/EtOH/TFA 40/60/0.3, flow rate: 25 mL/min, and detection wavelength: 254 nM) to give compounds **88j-A-A** (retention time: 10.727 min, 30 mg, 30%) and **88j-A-B** (retention time: 17.608 min, 20 mg, 20%) as white solids, both of which were chirally pure compounds.

**88j-A-A** (isomer A-A): ESI m/z $[M+H]^+$ = 521.9. $^1$H NMR (400 MHz, DMSO-$d_6$) δ 8.59 (d, *J*=2.8 Hz, 1 H), 8.37 (d, *J*=1.6 Hz, 1 H), 8.31-8.29 (m, 1 H), 8.25 (s, 1 H), 7.88 (d, *J*=8.4 Hz, 1 H), 7.76-7.69 (m, 3 H), 7.66-7.41(m, 2 H), 6.01 (d, *J*=5.6 Hz, 1 H), 5.48-5.44 (m, 1 H), 5.16 (d, *J*=8.4 Hz, 1 H), 5.06-4.95 (m, 2 H).

**88j-A-B** (isomer A-B): ESI m/z $[M+H]^+$ = 521.9. $^1$H NMR (400 MHz, DMSO-$d_6$) δ 8.59 (d, *J*=2.4 Hz, 1 H), 8.37 (s, 1 H), 8.31-8.29 (m, 1 H), 8.25 (s, 1 H), 7.88 (d, *J*=8.0 Hz, 1 H), 7.76-7.69 (m, 3 H), 7.66-7.41(m, 2 H), 6.02 (d, *J*=6.0 Hz, 1 H), 5.48-5.44 (m, 1 H), 5.17 (d, *J*=8.4 Hz, 1 H), 5.06-4.95 (m, 2 H).

**Example 23: (Compound 92)**

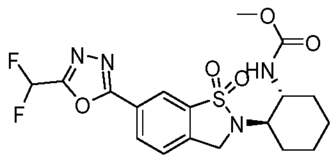

Referring to the synthetic method for compound **4** in Example 4, cyclopropylsulfonyl chloride was replaced with methyl chloroformate to give **92** as a white solid.

ESI m/z $[M+H]^+$ = 443.1. $^1$H NMR (400 MHz, DMSO-$d_6$) δ 8.38-8.34 (m, 2H), 7.98 (d, *J* = 4Hz, 1H), 7.57 (t, 1H), 7.03 (d, *J* = 4.2Hz, 1H), 4.68 (s, 1H), 3.50-3.45 (m, 2H) 3.31 (s, 3H), 1.89-1.70 (m, 5H), 1.51-1.49 (m, 1H), 1.28-1.24 (m, 2H).

**Example 24: (Compound 93)**

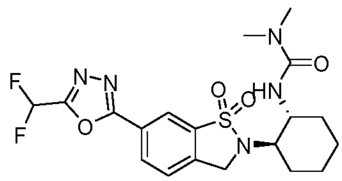

Referring to the synthetic method for compound **4** in Example 4, cyclopropylsulfonyl chloride was replaced with dimethylcarbamoyl chloride to give **93** as a white solid.

ESI m/z $[M+H]^+$ = 456.1. $^1$H NMR (400 MHz, DMSO-$d_6$) δ 8.37-8.33 (m, 2H), 7.87 (d, *J*=4Hz, 1H), 7.57 (t, 1H), 5.99 (d, *J*=3.6Hz, 1H), 4.65 (s, 1H), 3.76-3.73 (m, 1H), 3.57-3.56 (m, 1H), 2.63 (s, 6H), 1.85-1.70 (m, 5H), 1.55-1.52(m, 1H), 1.30-1.24 (m, 2H).

**Example 25: (Compound 94)**

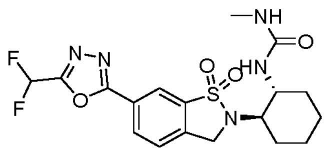

Referring to the synthetic method for compound **4** in Example 4, cyclopropylsulfonyl chloride was replaced with methylcarbamoyl chloride to give **94** as a white solid.

ESI m/z $[M+H]^+$ = 442.1. $^1H$ NMR (400 MHz, DMSO-$d_6$) δ 8.39-8.35 (m, 2H), 7.91 (d, *J*=8Hz, 1H), 7.59 (t, 1H), 5.77 (d, *J*=12Hz, 1H), 4.64 (d, *J*=5.2Hz, 1H), 4.71 (dd, *J*=10Hz, 2H), 3.76-3.73 (m, 1H), 3.45-3.37 (m, 1H), 2.39 (d, *J*=6Hz, 3H), 1.91-1.88 (m, 2H), 1.75-1.72 (m, 3H), 1.34-1.30 (m, 3H).

**Example 26: (Compounds 95j-A, 95j-B, 95j-B-A, and 95j-B-B)**

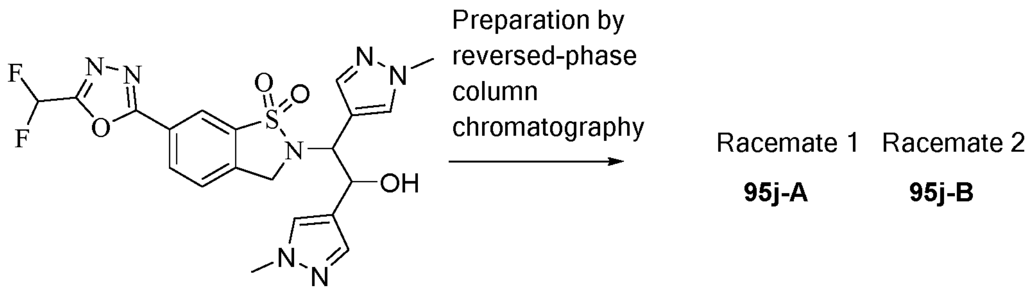


**95j**

**95j-B** SFC **95j-B-A + 95j-B-B**

1. (Compounds **95j-A** and **95j-B**)

Referring to the synthetic route for compound 84j in Example 21, **84a** and **84c** were replaced with 1-methylpyrazole 4-formaldehyde for synthesis to give **95j,** which was separated by reversed-phase preparative chromatography to give diastereoisomers **95j-A** and **95j-B** as white solids.

**95j-A** (isomer A): ESI m/z $[M+H]^+$ = 492.0. $^1H$ NMR (400 MHz, DMSO-$d_6$) δ 8.44 (s, 1 H), 8.34 (dd, $J_1$=1.2 Hz, $J_2$=8.0 Hz, 1 H), 7.73 (d, *J*=8.0 Hz, 1 H), 7.70 (s, 1 H), 7.60 (s, 1 H), 7.56 (s, 1 H), 7.46 (s, 1 H), 7.36-7.10 (m, 1 H), 5.52 (d, *J*=6.0 Hz, 1 H), 5.04 (d, *J*=6.0 Hz, 1 H), 4.83 (d, *J*=6.0 Hz, 1 H), 4.45 (d, *J*=15.6 Hz, 1 H), 3.81 (s, 6 H).

**95j-B** (isomer B): ESI m/z $[M+H]^+$ = 492.0. $^1H$ NMR (400 MHz, DMSO-$d_6$) δ 8.44 (s, 1 H), 8.34 (dd, $J_1$=1.2 Hz, $J_2$=8.0 Hz, 1 H), 7.78 (s, 1 H), 7.72 (d, *J*=8.0 Hz, 1 H), 7.64 (s, 1 H), 7.49 (s, 1 H), 7.36 (s, 1 H), 7.35-7.10 (m, 1 H), 5.34 (d, *J*=5.2 Hz, 1 H), 5.03 (d, *J*=5.2 Hz, 1 H), 4.77 (d, *J*=5.2 Hz, 1 H), 4.39 (d, *J*=15.6 Hz, 1 H), 3.84 (s, 3 H), 3.79 (s, 3 H).

2. (Compounds **95j-B-A** and **95j-B-B**)

Compound **95j-B** (40 mg) was subjected to chiral resolution (column: IE, mobile phase: MeOH/EtOH 50/50, flow rate: 25 mL/min, and detection wavelength: 214 nM) to give compound **95j-B-A** (retention time: 12.459 min, 20 mg, 50%) and compound **95j-B-B** (retention time: 15.560 min, 20 mg, 50%) as white solids, both of which were chirally pure compounds.

**95j-B-A** (isomer B-A): ESI m/z $[M+H]^+$ = 492.0. $^1H$ NMR (400 MHz, DMSO-$d_6$) δ 8.44 (s, 1 H), 8.34 (dd, $J_1$=1.6 Hz,

$J_2$=8.4 Hz, 1 H), 7.78 (s, 1 H), 7.72 (d, *J*=8.0 Hz, 1 H), 7.64 (s, 1 H), 7.49 (s, 1 H), 7.36 (s, 1 H), 7.35-7.10 (m, 1 H), 5.34 (d, *J*=4.8 Hz, 1 H), 5.04 (d, *J*=4.8 Hz, 1 H), 4.80 (d, *J*=4.8 Hz, 1 H), 4.39 (d, *J*=15.6 Hz, 1 H), 3.85 (s, 3 H), 3.79 (s, 3 H).
**95j-B-B** (isomer B-B): ESI m/z $[M+H]^+$ = 492.0. $^1$H NMR (400 MHz, DMSO-$d_6$) δ 8.44 (s, 1 H), 8.34 (dd, $J_1$=*1.6* Hz, $J_2$=8.4 Hz, 1 H), 7.78 (s, 1 H), 7.72 (d, *J*=8.4 Hz, 1 H), 7.64 (s, 1 H), 7.49 (s, 1 H), 7.36 (s, 1 H), 7.35-7.10 (m, 1 H), 5.34 (d, *J*=4.8 Hz, 1 H), 5.03 (d, *J*=4.8 Hz, 1 H), 4.78 (d, *J*=4.8 Hz, 1 H), 4.40 (d, *J*=15.6 Hz, 1 H), 3.85 (s, 3 H), 3.79 (s, 3 H).

**Example 27: (Compounds 99j-A, 99j-B, 99j-A-A, and 99j-A-B)**

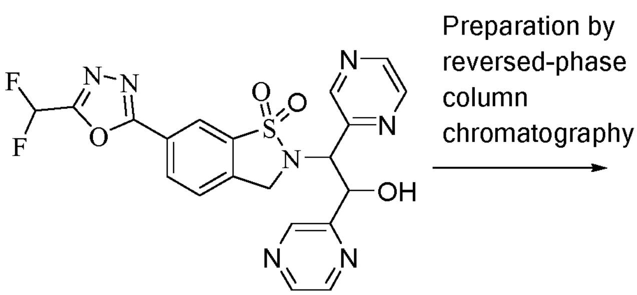


Racemate 1 Racemate 2

**99j-A** **99j-B**

**99j**

**99j-A** SFC **99j-A-A + 99j-A-B**

1. (Compounds **99j-A** and **99j-B**)

Referring to the synthetic route for compound 84j in Example 21, **84a and 84c** were replaced with 2-pyrazine formaldehyde, and finally, the product was separated by reversed-phase preparative chromatography to give diastereoisomers **99j-A** and **99j-B** as white solids.

**99j-A** (isomer A): ESI m/z $[M+H]^+$ = 488.1. $^1$H NMR (400 MHz, DMSO-$d_6$) δ 8.92 (s, 1 H), 8.83 (d, *J*=1.2 Hz, 1 H), 8.70 (s, 1 H), 8.63 (d, *J*=2.4 Hz, 1 H), 8.52-8.48 (m, 2 H), 8.32-8.30 (m, 1 H), 8.26 (s, 1 H), 7.88 (d, *J*=8.4 Hz, 1 H), 7.66-7.41 (m, 1 H), 6.24 (d, *J*=5.6 Hz, 1 H), 5.54-5.50 (m, 1 H), 5.36 (d, *J*=9.2 Hz, 1 H), 5.03 (s, 2 H).
**99j-B** (isomer B): ESI m/z $[M+H]^+$ = 488.1. $^1$H NMR (400 MHz, DMSO-$d_6$) δ 8.81 (s, *J*=1.2 Hz, 1 H), 8.64 (d, *J*=1.2 Hz, 1 H), 8.59-8.50 (m, 4 H), 8.37-8.35 (m, 2 H), 7.93 (d, *J*=8.4 Hz, 1 H), 7.68-7.43 (m, 1 H), 6.39 (d, *J*=5.2 Hz, 1 H), 5.73-5.70 (m, 1 H), 5.56 (d, *J*=5.6 Hz, 1 H), 5.15-5.05 (s, 2 H).

2. (Compounds **99j-A-A** and **99j-A-B**)

Compound **99j-A** (100 mg) was subjected to chiral resolution (column: IH, mobile phase: MeOH/EtOH/TFA 50/50/0.3, flow rate: 25 mL/min, and detection wavelength: 254 nM) to give compound **99j-A-A** (retention time: 8.371 min, 35 mg, 35%) and compound **99j-A-B** (retention time: 11.741 min, 35 mg, 35%) as white solids, both of which were chirally pure compounds.

**99j-A-A** (isomer A-A): ESI m/z $[M+H]^+$ = 488.0. $^1$H NMR (400 MHz, DMSO-$d_6$) δ 8.92 (d, *J*=1.2 Hz, 1 H), 8.83 (d, *J*=1.2 Hz, 1 H), 8.70 (s, 1 H), 8.63 (d, *J*=2.4 Hz, 1 H), 8.52-8.48 (m, 2 H), 8.32-8.26 (m, 2 H), 7.88 (d, *J*=8.0 Hz, 1 H), 7.66-7.41 (m, 1 H), 6.24 (d, *J*=6.0 Hz, 1 H), 5.54-5.51 (m, 1 H), 5.36 (d, *J*=9.2 Hz, 1 H), 5.03 (s, 2 H).
**99j-A-B** (isomer A-B): ESI m/z $[M+H]^+$ = 488.0. $^1$H NMR (400 MHz, DMSO-$d_6$) δ 8.93 (s, 1 H), 8.83 (d, *J*=0.8 Hz, 1 H), 8.70 (d, *J*=2.0 Hz, 1 H), 8.63 (d, *J*=2.4 Hz, 1 H), 8.52-8.48 (m, 2 H), 8.32-8.26 (m, 2 H), 7.88 (d, *J*=8.4 Hz, 1 H), 7.66-7.41 (m, 1 H), 6.25 (d, *J*=6.0 Hz, 1 H), 5.55-5.51 (m, 1 H), 5.36 (d, *J*=9.2 Hz, 1 H), 5.04 (s, 2 H).

**Example 28: (Compounds 103j-A, 103j-B, 103j-C, and 103j-D)**

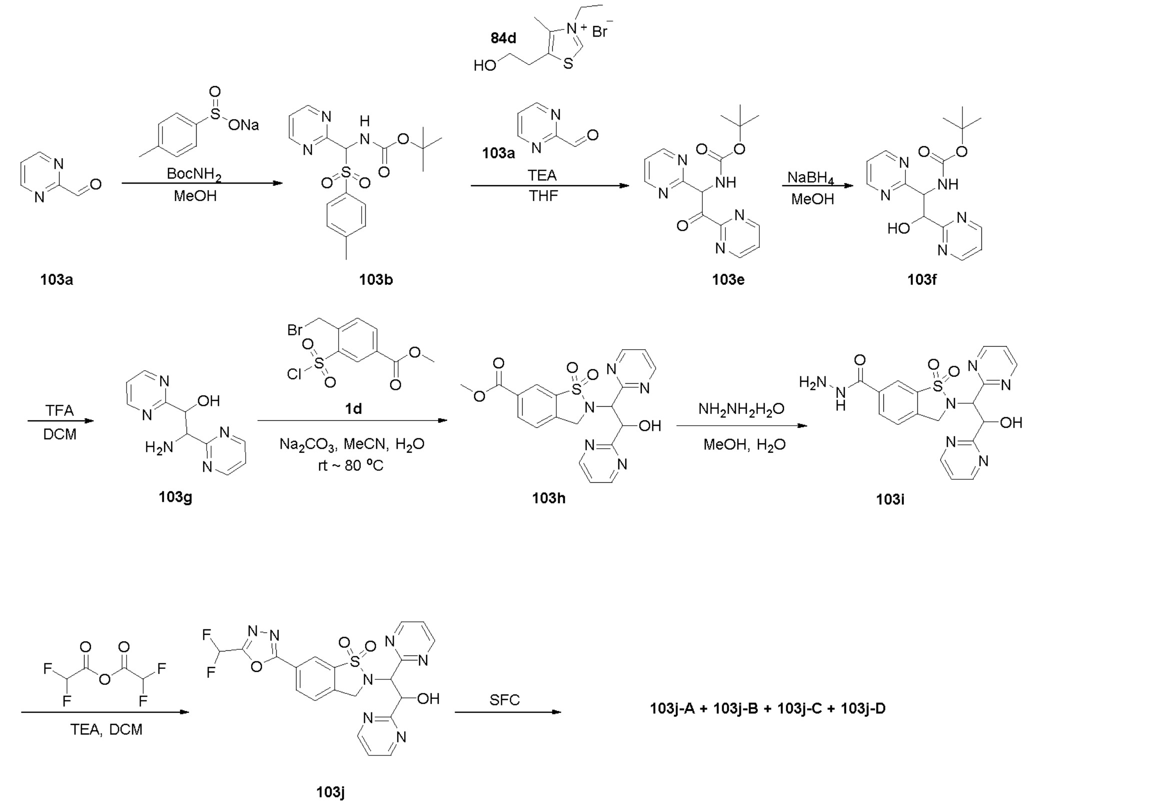


Referring to the synthetic route for compound 84j in Example 21, **84a** and **84c** were replaced with 2-pyrimidinecarboxaldehyde, and finally, the resulting mixture 103j was subjected to chiral resolution (column: IBN, mobile phase: MeOH/DCM 90/10, flow rate: 25 mL/min, and detection wavelength: 254 nM) to give compounds **103j-A** (retention time: 9.214 min), **103j-B** (retention time: 12.041 min), **103j-C** (retention time: 13.807 min), and **103j-D** (retention time: 18.648 min) as white solids, all of which were chirally pure compounds.

**103j-A** (isomer A): ESI m/z $[M+H]^+$ = 488.0. $^1H$ NMR (400 MHz, DMSO-$d_6$) δ 8.78-8.75 (m, 4 H), 8.36-8.34 (m, 1 H), 8.31 (s, 1 H), 7.97 (d, *J*=8.4 Hz, 1 H), 7.68-7.43 (m, 1 H), 7.41-7.36 (m, 2 H), 5.94 (d, *J*=4.8 Hz, 1 H), 5.73-5.71 (m, 1 H), 5.53-5.48 (m, 2 H), 5.14 (d, *J*=16.4 Hz, 1 H).
**103j-B** (isomer B): ESI m/z $[M+H]^+$ = 488.0. $^1H$ NMR (400 MHz, DMSO-$d_6$) δ 8.82-8.76 (m, 4 H), 8.32-8.30 (m, 1 H), 8.25 (s, 1 H), 7.94 (d, *J*=8.0 Hz, 1 H), 7.67-7.38 (m, 3 H), 5.86 (d, *J*=6.0 Hz, 1 H), 5.51-5.42 (m, 2 H), 5.33 (d, *J*=16.0 Hz, 1 H), 5.06 (d, *J*=15.6 Hz, 1 H).
103j-C (isomer C): ESI m/z $[M+H]^+$ = 487.9. $^1H$ NMR (400 MHz, DMSO-$d_6$) δ 8.82-8.77 (m, 4 H), 8.31 (d, *J*=8.4 Hz, 1 H), 8.25 (s, 1 H), 7.94 (d, *J*=8.0 Hz, 1 H), 7.67-7.38 (m, 3 H), 5.86 (d, *J*=6.4 Hz, 1 H), 5.51-5.42 (m, 2 H), 5.33 (d, *J*=16.0 Hz, 1 H), 5.06 (d, *J*=15.6 Hz, 1 H).
**103j-D** (isomer D): ESI m/z $[M+H]^+$ = 488.0. $^1H$ NMR (400 MHz, DMSO-$d_6$) δ 8.78-8.75 (m, 4 H), 8.36-8.34 (m, 1 H), 8.31 (s, 1 H), 7.97 (d, *J*=8.0 Hz, 1 H), 7.68-7.43 (m, 1 H), 7.41-7.36 (m, 2 H), 5.95 (d, *J*=4.8 Hz, 1 H), 5.73-5.71 (m, 1 H), 5.53-5.47 (m, 2 H), 5.13 (d, *J*=16.4 Hz, 1 H).

**Example 29: (Compound 107)**

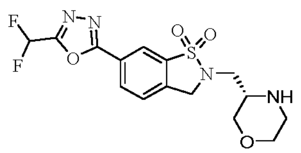

Referring to the synthetic method for compound **108** in Example 1, **1d'** was replaced with (*S*)-*N*-Boc-3-aminomethylmorpholine to give **107** as a white solid.

ESI m/z [M-H]$^-$ = 387.1. $^1$H NMR (400 MHz, Methanol-$d_4$) δ 8.56 (s, 1H), 8.47 (dd, *J* = 8.1, 1.6 Hz, 1H), 7.87 (d, *J* = 8.1 Hz, 1H), 7.29 (t, *J* = 51.6 Hz, 1H), 4.75 (d, *J* = 15.0 Hz, 1H), 4.63 (d, *J* = 14.9 Hz, 1H), 4.04 (d, *J* = 11.0 Hz, 1H), 3.92 (dt, *J* = 12.2, 3.2 Hz, 1H), 3.69 (ddd, *J* = 12.3, 10.0, 2.8 Hz, 1H), 3.55 - 3.36 (m, 4H), 3.22 - 2.95 (m, 2H).

**Example 30: (Compound 109)**

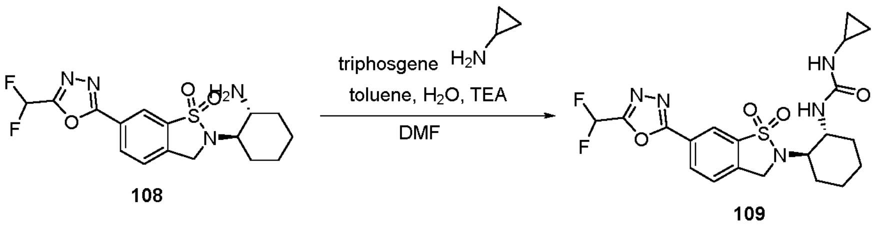


108

109

To a mixture of cyclopropylamine (500 mg, 8.76 mmol) and triethylamine (2.66 g, 26.28 mmol) in toluene (3 mL) and water (2 mL) was added triphosgene (0.91 g, 3.07 mmol) at 0 °C. The mixture was stirred for 2 h. The upper toluene phase was taken and added to a solution of compound **108** (200 mg, 0.52 mmol) and triethylamine (130 mg, 1.3 mmol) in N,N-dimethylformamide (2 mL). The mixture was stirred for 2 h. The reaction solution was washed with water and separated by reversed-phase column chromatography to give **109** (30 mg, 12%) as a white solid.

ESI m/z [M+H]$^+$ = 468.2. $^1$H NMR (400 MHz, DMSO-$d_6$) δ 8.41 (d, *J* = 1.5 Hz, 1H), 8.37 (dd, *J* = 8.1, 1.6 Hz, 1H), 7.91 (d, *J* = 8.1 Hz, 1H), 7.60 (t, *J* = 51.3 Hz, 1H), 6.08 (s, 1H), 5.75 (d, *J* = 9.1 Hz, 1H), 4.80 (d, *J* = 15.5 Hz, 1H), 4.66 (d, *J* = 15.5 Hz, 1H), 3.73 (d, *J* = 11.1 Hz, 1H), 3.52 (t, *J* = 10.2 Hz, 1H), 2.23 (dd, *J* = 7.0, 4.0 Hz, 1H), 2.09 - 1.99 (m, 1H), 1.92 (d, *J* = 13.1 Hz, 2H), 1.75 (d, *J* = 11.0 Hz, 2H), 1.48 (d, *J* = 11.7 Hz, 2H), 0.46 (dt, *J* = 12.4, 7.5 Hz, 2H), 0.24 (dd, *J* = 10.0, 5.0 Hz, 1H), 0.18 - 0.08 (m, 1H).

**Example 31: (Compound 110)**

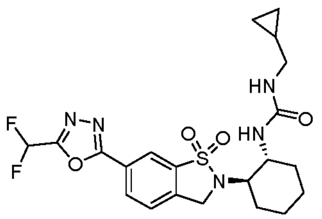

Referring to the synthetic method of compound **109** in Example 30, cyclopropylamine was replaced with cyclopropylmethylamine to give **110** as a white solid.

ESI m/z [M+H]$^+$ = 482.2. $^1$H NMR (400 MHz, DMSO-$d_6$) δ 8.44 - 8.20 (m, 2H), 7.88 (d, *J* = 8.1 Hz, 1H), 7.57 (t, *J* = 51.3 Hz, 1H), 5.85 - 5.68 (m, 2H), 4.78 (d, *J* = 15.4 Hz, 1H), 4.62 (d, *J* = 15.5 Hz, 1H), 3.79 - 3.59 (m, 1H), 2.69 (td, *J* = 6.3, 4.7 Hz, 2H), 1.88 (d, *J* = 11.7 Hz, 2H), 1.71 (q, *J* = 12.0, 10.9 Hz, 3H), 1.45 - 1.19 (m, 4H), 0.64 - 0.53 (m, 1H), 0.10 (ddd, *J* = 9.8, 4.7, 2.7 Hz, 2H), -0.09 (dd, J = 4.8, 1.9 Hz, 2H).

**Example 32: (Compound 111)**

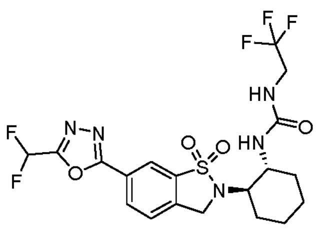

Referring to the synthetic method of compound **109** in Example 30, cyclopropylamine was replaced with trifluoroethylamine to give **111** as a white solid.
ESI m/z $[M+H]^+$ = 510.2. $^1$H NMR (400 MHz, DMSO-$d_6$) δ 8.41 (s, 1H), 8.38 (dd, *J* = 8.1, 1.6 Hz, 1H), 7.92 (d, *J* = 8.1 Hz, 1H), 7.62 (t, *J* = 51.3 Hz, 1H), 6.44 (t, *J* = 6.5 Hz, 1H), 6.13 (d, *J* = 9.3 Hz, 1H), 4.82 - 4.64 (m, 2H), 3.85 - 3.73 (m, 1H), 3.72 - 3.62 (m, 2H), 3.47 (dt, *J* = 11.1, 5.7 Hz, 1H), 1.99 - 1.88 (m, 2H), 1.76 (t, *J* = 12.3 Hz, 3H), 1.50 - 1.30 (m, 3H).

**Example 33: (Compound 112)**

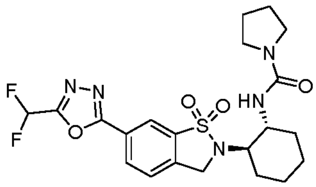

Referring to the synthetic method of compound **109** in Example 30, cyclopropylamine was replaced with tetrahydropyrrole to give **112** as a white solid.
ESI m/z $[M+H]^+$ = 482.3. $^1$H NMR (400 MHz, DMSO-$d_6$) δ 8.40 (d, *J* = 1.5 Hz, 1H), 8.36 (dd, *J*= 8.1, 1.6 Hz, 1H), 7.90 (d, *J* = 8.1 Hz, 1H), 7.60 (t, *J* = 51.3 Hz, 1H), 5.81 (d, *J* = 9.0 Hz, 1H), 4.70 (q, *J* = 15.6 Hz, 2H), 3.75 (t, *J* =11.9 Hz, 1H), 3.59 (td, *J* = 11.1, 10.7, 3.8 Hz, 1H), 3.12 (dt, J = 9.4, 6.1 Hz, 2H), 3.01 (dt, *J* = 9.4, 6.3 Hz, 2H), 1.87 (t, *J* = 10.4 Hz, 2H), 1.74 (q, *J* = 11.3, 10.1 Hz, 3H), 1.69 - 1.62 (m, 4H), 1.56 (d, *J* = 12.2Hz, 1H), 1.33 (t, *J* = 10.5 Hz, 2H).

**Example 34: (Compound 113)**

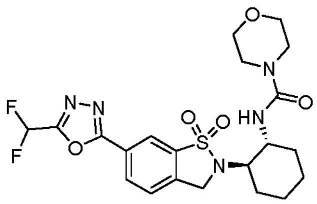

Referring to the synthetic method of compound **109** in Example 30, cyclopropylamine was replaced with morpholine to give **113** as a white solid.
ESI m/z $[M+H]^+$ = 498.2. $^1$H NMR (400 MHz, DMSO-$d_6$) δ 8.60 - 8.30 (m, 2H), 7.82 (d, *J* = 8.2 Hz, 1H), 7.26 (t, *J* = 51.6 Hz, 1H), 6.28 (d, *J* = 8.9 Hz, 1H), 4.73 - 4.60 (m, 2H), 3.98 - 3.80 (m, 1H), 3.63 (td, *J* = 11.2, 4.1 Hz, 1H), 3.51 - 3.36 (m, 4H), 3.23 (t, *J* = 5.0 Hz, 4H), 2.01 (d, *J* = 13.8 Hz, 2H), 1.93 - 1.70 (m, 3H), 1.48 (q, *J* = 13.8, 13.2 Hz, 3H).

**Example 35: (Compound 114)**

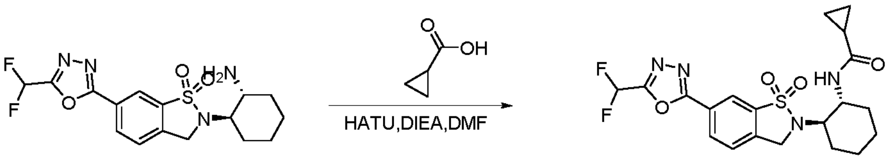


108 114

To a solution of cyclopropylcarboxylic acid (20 mg, 0.23 mmol) in *N*,*N*-dimethylformamide (2 mL) were added diisopropylethylamine (90 mg, 0.70 mmol) and HATU (90 mg, 0.24 mmol). The mixture was stirred at room temperature for 30 min, and then compound **108** (100 mg, 0.26 mmol) was added. The mixture was stirred overnight. The reaction solution was filtered and separated by reversed-phase column chromatography to give **114** (30 mg, 28%) as a white solid.

ESI m/z $[M+H]^+$ = 453.2. $^1$H NMR (400 MHz, DMSO-$d_6$) δ 8.44-8.31 (m, 2H), 7.98 (d, *J* = 9.4 Hz, 1H), 7.89 (d, *J*= 8.1 Hz, 1H), 7.60 (t, *J*= 51.3 Hz, 1H), 4.80-4.53 (m, 2H), 4.00-3.82 (m, 1H), 3.49 (dd, *J* = 11.3, 3.8 Hz, 1H),1.98-1.82 (m, 2H), 1.82-1.67 (m, 3H), 1.59-1.21 (m, 4H), 0.54 (dddd, *J*= 20.3, 10.0, 7.3, 3.3 Hz, 2H), 0.46-0.32 (m, 1H), 0.20 (ddt, *J*= 9.8, 7.2, 3.9 Hz, 1H).

**Example 36: (Compound 118)**

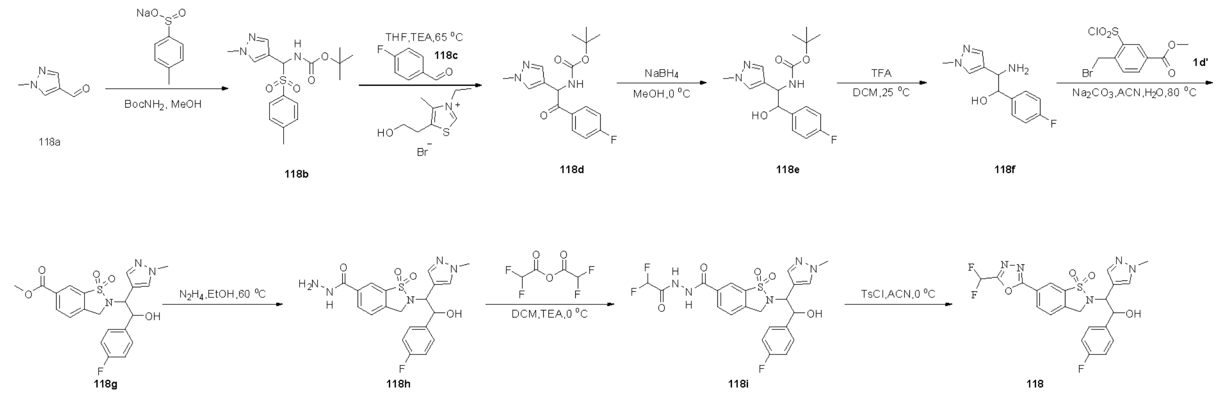


Referring to the synthetic method of Example 21, **84a** was replaced with **118a** and **84c** was replaced with **118c** to give **118.**

ESI m/z $[M+H]^+$= 506.1. $^1$H NMR (400 MHz, DMSO-$d_6$) δ 8.40 (s, 1H), 8.35 (dd, J = 8.1, 1.6 Hz, 1H), 7.87 (d, J = 8.1 Hz, 1H), 7.73 (d, J = 13.3 Hz, 1H), 7.47 (d, J = 7.0 Hz, 1H), 7.41 (dd, J = 8.5, 5.5 Hz, 2H), 7.11 (t, J = 8.8 Hz,2H), 6.09 (d, J = 4.9 Hz, 1H), 5.26 (t, J = 5.1 Hz, 1H), 5.06 - 4.83 (m, 2H), 4.40 (d, J = 16.2 Hz, 1H), 3.79 (s, 3H).

**Example 37: (Compound 116)**

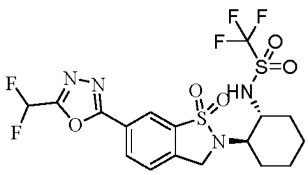

Referring to the synthetic method for compound**2** in Example 2, difluoroacetic anhydride was replaced with trifluoromethanesulfonic anhydride to give **116** as a white solid.

ESI m/z $[M+H]^+$ = 515.2. $^1$H NMR (400 MHz, DMSO-$d_6$) δ 9.36 (d, J = 9.1 Hz, 1H), 8.46 - 8.33 (m, 2H), 7.93 (d, J = 8.1 Hz, 1H), 7.60 (t, J = 51.3 Hz, 1H), 4.73 (d, J = 15.5 Hz, 1H), 4.63 (d, J = 15.4 Hz, 1H), 4.03 - 3.90 (m,1H), 3.69 - 3.59 (m, 1H), 1.82 (d, J = 17.0 Hz, 4H), 1.73 (d, J = 11.0 Hz, 2H), 1.36 (d, J = 10.4 Hz, 2H).

**Example 38: (Compound 117)**

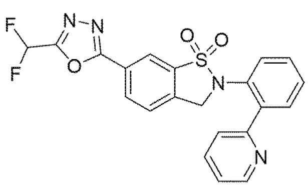

Referring to the synthetic method for compound **72** in Example 19, 4-bromo-1-methyl-1H-pyrazole was replaced with 2-bromopyridine in step 1 to give **117** as a white solid.

ESI m/z $[M+H]^+$ = 441.2. $^1H$ NMR (400 MHz, DMSO-$d_6$) δ 8.60 (d, J = 4.9 Hz, 1H), 8.47 (d, J = 1.4 Hz, 1H), 8.41 (dd, J = 8.1, 1.6 Hz, 1H), 7.90 (d, J = 8.2 Hz, 1H), 7.80- 7.74 (m, 4H), 7.69 - 7.64 (m, 2H), 7.54 (d, J = 51.2 Hz, 1H), 7.32 (ddd, J = 6.7, 4.8, 1.7 Hz, 1H), 4.98 (s, 2H).

**Example 39: (Compound 14)**

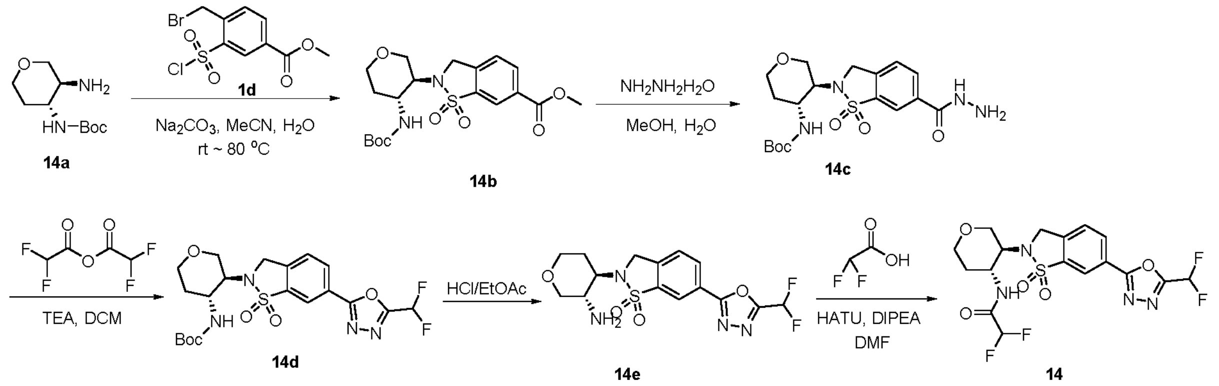


1. (Compound 14e)

Referring to the synthetic method for compound **108** in Example 1, **1d'** was replaced with 14a for synthesis over 4 steps to give **14e** as a white solid.

2. (Compound 14)

Referring to the synthetic method for compound **2** in Example 2, compound **108** was replaced with compound **14e** to give **14** as a white solid.

ESI m/z $[M-H]^-$ = 463.2. $^1H$ NMR (400 MHz, DMSO-$d_6$) δ 8.54 (s, 1H), 8.47 (dd, *J* = 8.1, 1.6 Hz, 1H), 7.88 *(d, J*= 8.1 Hz, 1H), 7.32 (t, *J* = 51.7 Hz, 1H), 5.95 (td, *J* = 54.0, 3.3 Hz, 1H), 4.66 (s, 2H), 4.43 (dt*, J* = 10.5, 5.5 Hz, 1H), 4.13 - 4.05 (m, 2H), 3.89 - 3.77 (m, 2H), 3.65 - 3.58 (m, 1H), 2.05 (ddd, *J*= 18.9, 12.2, 6.6 Hz, 3H).

**Example 40: (Compound 13)**

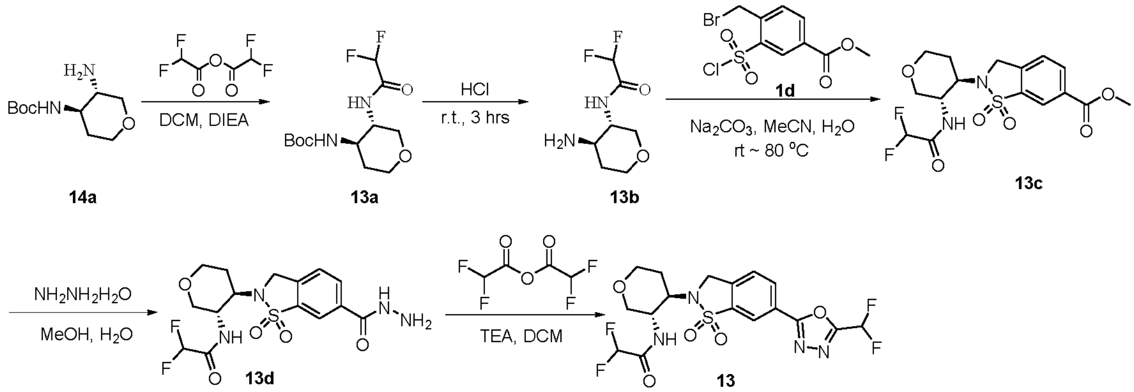


1. (Compound **13a)**

To a solution of compound **14a** (200 mg, 0.92 mmol) in dichloromethane (2 mL) was added diisopropylethylamine (360 mg, 2.76 mmol). The mixture was stirred for 5 min, and difluoroacetic anhydride (240 mg, 1.38 mmol) was added dropwise at 0 °C. The mixture was stirred for 2 h. The reaction solution was treated with water, extracted with ethyl acetate, and concentrated to give **13a** (250 mg, 91%) as a colorless oil.

ESI m/z $[M+H]^+$ = 295.1.

2. (Compound **13b)**

To a solution of compound **13a** (250 mg, 0.85 mmol) in dichloromethane was added a solution of hydrogen chloride in dioxane (1 mL, 4 M). The mixture was stirred at room temperature for 3 h. The reaction solution was concentrated to give **13b** (150 mg, 90%) as a colorless oil.

ESI m/z $[M+H]^+$ = 195.1.

3. (Compound **13)**

Referring to the synthetic method for compound **20** in Example 1, **1d'** was replaced with **13b** to give **13** as a white solid.

ESI m/z $[M-H]^-$ = 463.2. $^1$H NMR (400 MHz, Methanol-$d_4$) δ 8.54 (s, 1H), 8.47 (dd, *J*= 8.1, 1.6 Hz, 1H), 7.88 *(d, J*= 8.1 Hz, 1H), 7.32 (t, *J* = 51.7 Hz, 1H), 5.95 (td, *J* = 54.0, 3.3 Hz, 1H), 4.66 (s, 2H), 4.43 (dt, *J* = 10.5, 5.5 Hz, 1H), 4.13 - 4.05 (m, 2H), 3.89 - 3.77 (m, 2H), 3.65 - 3.58 (m, 1H), 2.05 (ddd, *J* = 18.9, 12.2, 6.6 Hz, 3H).

**Example 41: (Compound 65)**

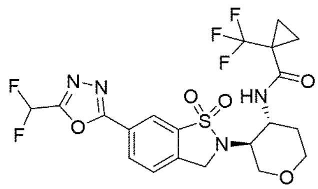

Referring to the synthetic method of compound **114** in Example 35, compound **108** was replaced with compound **14e** and cyclopropylcarboxylic acid was replaced with 1-trifluoromethylcyclopropyl-1-carboxylic acid to give **65** as a white solid.

ESI m/z $[M+H]^+$ = 521.2. $^1$H NMR (400 MHz, DMSO-$d_6$) δ 8.45 (d, *J*= 1.5 Hz, 1H), 8.39 (dd, *J* = 8.1, 1.6 Hz, 1H), 7.92 *(d, J*= 8.2 Hz, 1H), 7.82 *(d, J*= 9.1 Hz, 1H), 7.59 (t, *J* = 51.3 Hz, 1H), 4.77 - 4.63 (m, 2H), 4.25 (dq, *J* = 10.2, 5.3 Hz, 1H), 3.87 (ddd,*J* = 14.5, 10.6, 4.0 Hz, 2H), 3.75 - 3.59 (m, 2H), 3.46 (d, *J* = 10.3 Hz, 1H), 2.06 - 1.85 (m, 1H), 1.81 (dd, *J* = 13.5, 4.6 Hz, 1H), 1.41 - 1.32 (m, 1H), 1.19 - 1.12 (m, 1H), 1.08 (ddd, *J* = 10.4, 8.7, 6.4 Hz, 2H).

**Example 42: (Compound 66)**

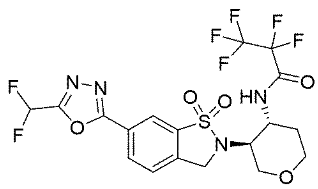

Referring to the synthetic method for compound 2 of Example 2, compound 108 was replaced with compound 14e and difluoroacetic anhydride was replaced with pentafluoropropionic anhydride to give 66 as a white solid.

ESI m/z $[M+H]^+$ = 533.0. $^1$H NMR (400 MHz, DMSO-$d_6$) δ 9.63 (s, 1H), 8.46 (s, 1H), 8.40 (dd,*J* = 8.1, 1.6 Hz, 1H), 7.94 (d,*J* = 8.2 Hz, 1H), 7.60 (d, *J* = 102.6 Hz, 1H), 7.60 (s, 1H), 4.80- 4.62 (m, 2H), 4.36 (s, 1H), 3.91 (dd, *J* = 10.2, 4.6 Hz, 2H), 3.84 - 3.67 (m, 2H), 3.50 (t, *J* = 11.7 Hz, 1H), 2.06 - 1.99 (m, 1H), 1.89 (d,*J* = 10.9 Hz, 1H).

**Example 43: (Compound 119)**

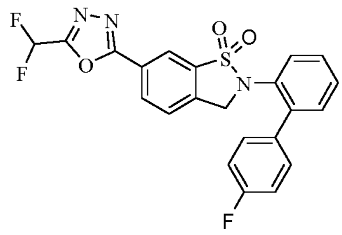

Referring to the synthetic method for compound **72** in Example 19, 4-bromo-1-methyl-1*H*-pyrazole was replaced with 4-bromo-1-fluorobenzene in step 1 to give **119** as a white solid. ESI m/z $[M-H]^-$ = 456.2 $^1$H NMR (400 MHz, DMSO-$d_6$) δ 8.52 *(d, J*= 1.5 Hz, 1H), 8.38 (dd, *J* = 8.1, 1.6 Hz, 1H), 7.84 - 7.78 (m, 2H), 7.73 - 7.48 (m, 6H), 7.24- 7.18 (m, 2H), 4.66 (s, 2H).

**Example 44: (Compound 120)**

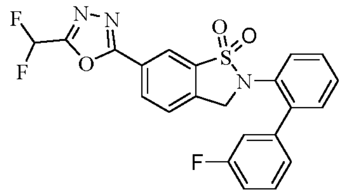

Referring to the synthetic method for compound **72** in Example 19, 4-bromo-1-methyl-1*H*-pyrazole was replaced with 3-bromo-1-fluorobenzene in step 1 to give **120** as a white solid.

$^1$H NMR (400 MHz, DMSO-d6) δ 8.54 (d, *J* = 1.5 Hz, 1H), 8.39 (dd, *J* = 8.1, 1.6 Hz, 1H), 7.85 - 7.78 (m, 2H), 7.67 - 7.47 (m, 4H), 7.44 - 7.37 (m, 3H), 7.18 (tdd, *J* = 9.2, 4.0, 2.2 Hz, 1H), 4.68 (s, 2H).

**Example 45: (Compound 121)**

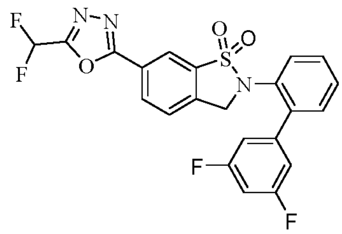

Referring to the synthetic method for compound **72** in Example 19, 4-bromo-1-methyl-1*H*-pyrazole was replaced with 5-bromo-1,3-difluorobenzene in step 1 to give **121** as a white solid.

$^1$H NMR (400 MHz, DMSO-$d_6$) δ 8.54 (d, *J* = 1.5 Hz, 1H), 8.41 (dd, *J*= 8.1, 1.6 Hz, 1H), 7.86 (d, *J* = 8.2 Hz, 1H), 7.83 - 7.78 (m, 1H), 7.75 - 7.46 (m, 4H), 7.33 - 7.26 (m, 2H), 7.23 (tt, *J* = 9.4, 2.4 Hz, 1H), 4.78 (s, 2H).

**Example 46: (Compound 63)**

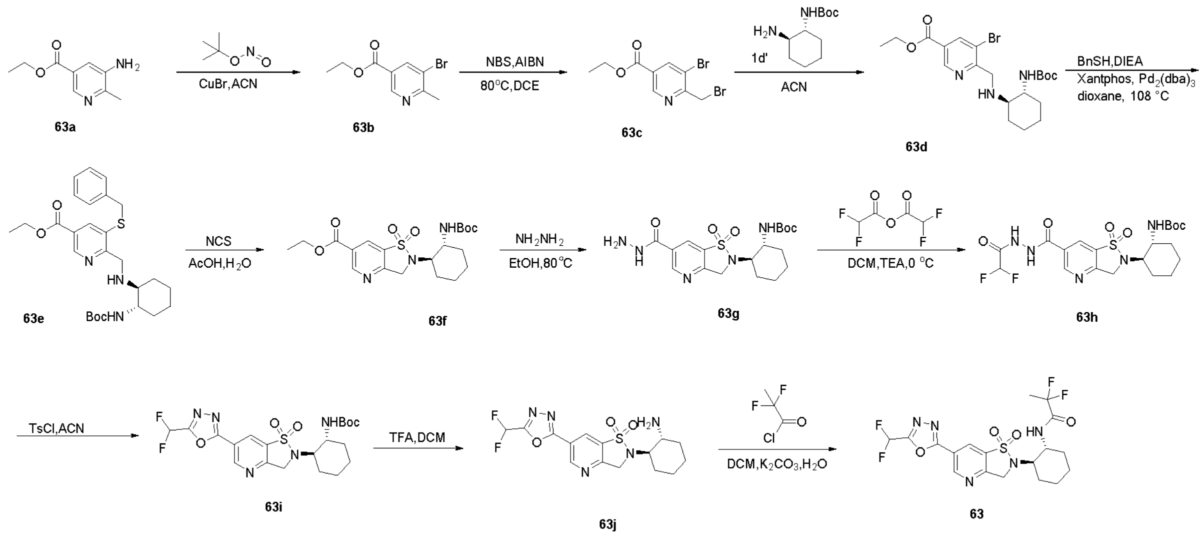


63a 63b 63c 63d 63e 63f 63g 63h 63i 63j 63

1. (Compound **63b)**

To a reaction flask were added compound **63a** (5 g, 27.75 mmol), cuprous bromide (4.78 g, 33.3 mmol), and acetonitrile (100 mL). The mixture was cooled to 0 °C, and *tert*-butyl nitrite (7.73 g, 74.93 mmol) was added dropwise. The mixture was stirred at room temperature for two days. After the reaction was completed as detected by LCMS, the reaction solution was concentrated and subjected to silica gel column chromatography (dichloromethane/ethyl acetate: 20%-50%) to give a product **63b** (5 g, 73.83%) as an off-white solid. MS m/z $[M+H]^+$: 244.0.

2. (Compound **63c)**

To a reaction flask were added compound **63b** (1.5 g, 6.15 mmol), azobisisobutyronitrile (0.1 g, 0.62 mmol), N-bromosuccinimide (1.09 g, 6.15 mmol), and dichloroethane (20 mL). The mixture was heated to 80 °C and stirred overnight. After the reaction was completed as detected by LCMS, the reaction solution was concentrated and purified by silica gel column chromatography (dichloromethane/ethyl acetate: 30%-80%) to give **63c** (1.2 g, 60.46%) as a yellow oil. MS m/z $[M+H]^+$: 323.8

3. (Compound **63d)**

To a reaction flask were added compound **63c** (1.2 g, 4.03 mmol), compound **1d'** (1 g, 4.67 mmol), and acetonitrile (20 mL). The mixture was stirred at room temperature for 5 h. After the reaction was completed as detected by LCMS, water (30 mL) was added to the reaction solution, and the resulting mixture was extracted with dichloromethane (30 mL × 2), and subjected to liquid separation. The extract was washed with water and saturated brine, dried over sodium sulfate, concentrated, and purified by silica gel column chromatography (dichloromethane/ethyl acetate: 50%-90%) to give an off-white product **63d** (1 g, yield: 54.44%). MS m/z $[M+H]^+$: 456.2.

4. (Compound **63e)**

To a reaction flask were added compound **63d** (1 g, 2.19 mmol), benzyl mercaptan (0.5 g, 4.03 mmol), *N,N*-diisopropylethylamine (0.8 g, 6.19 mmol), 4,5-bis(diphenylphosphino)-9,9-dimethylxanthene (0.13 g, 0.22 mmol), tris(dibenzylideneacetone)dipalladium (0.20 g, 0.22 mmol), and dioxane (20 mL). The mixture was purged with nitrogen three times, heated to 106 °C, and stirred for 16 h. After the reaction was completed as detected by LCMS, the reaction solution was purified by silica gel column chromatography (dichloromethane/ethyl acetate: 50%-90%) to give an off-white product **63e** (400 mg, yield: 36.53%). MS m/z $[M+H]^+$: 500.3.

5. (Compound **63f)**

To a reaction flask were added compound **63e** (400 mg, 0.80 mmol), N-chlorosuccinimide (1070 mg, 8 mmol), acetic acid (3 mL), and water (1 mL). The mixture was stirred at room temperature for 3 h. After the reaction was completed as detected by LCMS, water (20 mL) was added to the reaction solution, and the resulting mixture was adjusted to pH 7-8 with a saturated sodium carbonate solution, extracted with dichloromethane (30 mL × 2), and subjected to liquid

separation. The extract was washed with water and saturated brine, concentrated, and separated by silica gel column chromatography (ethyl acetate/dichloromethane: 30%-80%) to give an off-white product **63f** (300 mg, yield: 85.26%). MS m/z $[M-100]^+$: 340.1.

6. (Compound **63g)**

To a reaction flask were added compound **63f** (300 mg, 0.68 mmol), hydrazine hydrate (0.5 mL), and ethanol (5 mL). The mixture was heated to 80 °C and stirred for 2 h. After the reaction was completed as detected by LCMS, the reaction solution was concentrated to give a crude product **63g,** which was directly used in the next step (0.3 g, yield: 100%). MS m/z $[M-100]^+$: 326.1

7. (Compound **63h)**

To a reaction flask were added compound **63g** (300 mg, 0.71 mmol), triethylamine (220 mg, 2.17 mmol), and dichloromethane (5 mL). Difluoroacetic anhydride (250 mg, 1.44 mmol) was added dropwise at 0 °C. The mixture was stirred for 2 h. After the reaction was completed as detected by LCMS, the reaction solution was concentrated to give **63h** (0.3 g, yield: 84.51%) as a pale yellow product. MS m/z $[M-100]^+$: 404.2.

8. (Compound **63i)**

To a reaction flask were added compound **63h** (300 mg, 0.60 mmol), p-toluenesulfonyl chloride (140 mg, 0.72 mmol), triethylamine (180 mg, 1.80 mmol), and acetonitrile (5 mL) at 0 °C. The mixture was stirred at 25 °C for 2 h. After the reaction was completed as detected by LCMS, water (20 mL) was added to the reaction solution, and the resulting mixture was extracted with dichloromethane (30 mL × 2), and subjected to liquid separation. The extract was washed with water, concentrated, and purified by silica gel column chromatography (ethyl acetate/dichloromethane: 40%-90%) to give a yellow product **63i** (0.1 g, yield: 34.57%). MS m/z $[M-100]^+$: 386.1.

9. (Compound **63j)**

To a reaction flask were added compound **63i** (100 mg, 0.21 mmol) and dichloromethane (2 mL). Trifluoroacetic acid (0.2 mL) was added dropwise at 0 °C. The mixture was stirred at room temperature for 1 h. After the reaction was completed as detected by LCMS, water (10 mL) was added to the reaction solution, and the resulting mixture was adjusted to pH 7-9 with a saturated aqueous sodium carbonate solution, extracted with dichloromethane (30 mL × 2), and subjected to liquid separation. The extract was washed with water, dried over sodium sulfate, filtered, and concentrated to give a white product **63j** (60 mg, yield: 75.59%). MS m/z $[M+H]^+$: 386.1.

10. (Compound **63)**

To a reaction flask were added compound 2,2-difluoropropionic acid (400 mg, 3.63 mmol), one drop of DMF, and dichloromethane (3 mL). Thionyl chloride (450 mg, 3.78 mmol) was slowly added dropwise. The mixture was stirred at 0 °C for 2 h to give a 2,2-difluoropropionyl chloride solution.

To another reaction flask were added compound **63j** (60 mg, 0.16 mmol), potassium carbonate (100 mg, 0.72 mmol), DCM (3 mL), and water (1 mL). The 2,2-difluoropropionyl chloride solution described above was added dropwise at 0 °C. The mixture was stirred at room temperature for 1 h. After the reaction was completed as detected by LCMS, the reaction solution was concentrated and purified by reversed-phase column chromatography (acetonitrile/0.05% aqueous formic acid solution: 30%-60%) to give a product **63** (19.2 mg, yield: 25.83%, purity: 96.92%) as a white solid. MS m/z $[M+H]^+$: 478.1

$^1$H NMR (400 MHz, DMSO-$d_6$) δ 9.51 (d, J = 2.0 Hz, 1H), 9.03 (d, J = 2.0 Hz, 1H), 8.64 (d, J = 9.2 Hz, 1H), 7.63 (t, J = 51.2 Hz, 1H), 4.90 - 4.68 (m, 2H), 4.05 - 3.90 (m, 1H), 3.77 - 3.65 (m, 1H), 1.91 -1.70 (m, 6H), 1.53 (t, J = 19.5 Hz, 3H), 1.36 (s, 2H).

**Example 47: (Compound 145)**

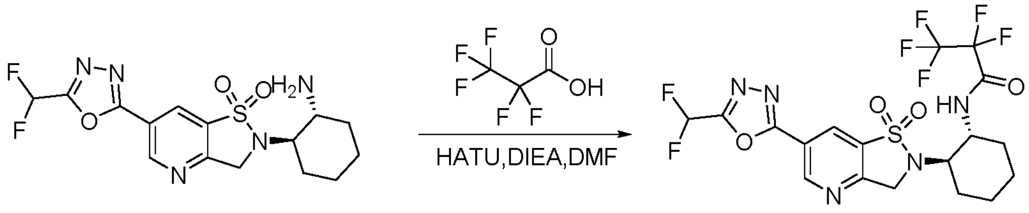


63j 145

To a reaction flask were added pentafluoropropionic acid (20 mg, 0.12 mmol), *N,N-diisopropylethylamine* (30 mg, 0.23 mmol), HATU (44 mg, 0.12 mmol), and DMF (2 mL). The mixture was stirred for half an hour at room temperature, and compound **63j** (30 mg, 0.078 mmol) was added. The mixture was stirred for 2 h at room temperature. After the reaction was completed as detected by LCMS, the reaction solution was purified by reversed-phase column chromatography (acetonitrile/0.05% aqueous formic acid solution: 50%-70%) to give a product **145** (2 mg, yield: 4.83%) as a white solid. MS m/z[M-H]$^+$:530.2

$^1$H NMR (400 MHz, Methanol-$d_4$) δ 9.55 (d, J = 2.0 Hz, 1H), 8.92 (d, J = 2.0 Hz, 1H), 7.30 (t, J = 51.6 Hz, 1H), 4.78 (d, J = 16.2 Hz, 2H), 4.15 (td, J = 11.2, 4.4 Hz, 1H), 3.81 (dt, J = 13.0, 6.6 Hz, 1H), 2.16- 1.84 (m, 6H), 1.50 (q, J = 11.6, 11.1 Hz, 2H).

**Example 48: (Compound 18)**

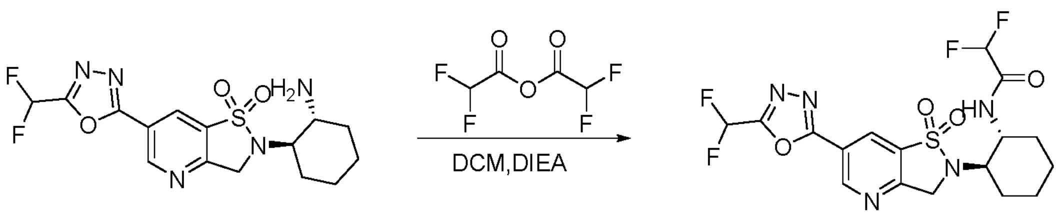


63j 18

To a reaction flask were added compound **63j** (30 mg, 0.078 mmol), *N,N*-diisopropylethylamine (30 mg, 0.039 mmol), and DCM (3 mL). Difluoroacetic anhydride (16 mg, 0.094 mmol) was added dropwise at 0 °C. The mixture was stirred at room temperature for 1 h. After the reaction was completed as detected by LCMS, the reaction solution was concentrated and purified by reversed-phase column chromatography (acetonitrile/0.05% aqueous formic acid solution: 30%-60%) to give a product **18** (16.6 mg, yield: 46.02%, purity: 100%) as a white solid. MS m/z [M+H]$^+$: 464.2

$^1$H NMR (400 MHz, DMSO-$d_6$) δ 9.51 (d, J = 1.9 Hz, 1H), 9.02 (d, J = 1.9 Hz, 1H), 8.79 (d, J = 9.2 Hz, 1H), 7.63 (t, J = 51.2 Hz, 1H), 6.03 (t, J = 53.8 Hz, 1H), 4.78 (q, J = 16.1 Hz, 2H), 3.99 (d, J = 10.4Hz, 1H), 3.66 (td, J = 10.4, 5.5 Hz, 1H), 2.02 - 1.77 (m, 4H), 1.77 - 1.62 (m, 2H), 1.38 (d, J = 9.7 Hz, 2H).

**Example 49: (Compound 159)**

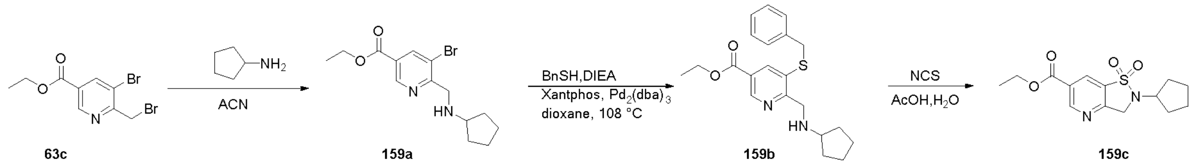


63c 159a 159b 159c

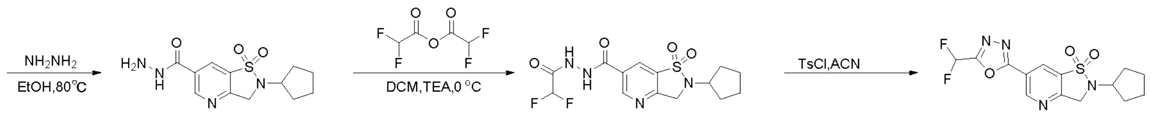


159d 159e 159

1. (Compound **159a)**

To a reaction flask were added compound **63c** (200 mg, 0.62 mmol), cyclopentylamine (60 mg, 0.64 mmol), potassium carbonate (260 mg, 1.86 mmol), and acetonitrile (2 mL). The mixture was stirred at room temperature for 3 h. After the reaction was completed as detected by LCMS, water (30 mL) was added to the reaction solution, and the resulting mixture was extracted with dichloromethane (30 mL × 2), and subjected to liquid separation. The extract was washed with water and saturated brine, dried over sodium sulfate, filtered, and concentrated to give a product **159a** (0.1 g, yield: 49.35%) as a yellow oil. MS m/z $[M+H]^+$: 327.0.

2. (Compound **159b)**

To a reaction flask were added compound **159a** (0.1 g, 0.31 mmol), benzyl mercaptan (0.06 g, 0.46 mmol), N,N-diisopropylethylamine (0.12 g, 0.93 mmol), 4,5-bis(diphenylphosphino)-9,9-dimethylxanthene (0.04 g, 0.06 mmol), tris(dibenzylideneacetone)dipalladium (0.03 g, 0.03 mmol), and dioxane (3 mL). The mixture was purged with nitrogen three times, heated to 108 °C, and stirred for 16 h. After the reaction was completed as detected by LCMS, the reaction solution was purified by silica gel column chromatography (dichloromethane/ethyl acetate: 50%-90%) to give an off-white product **159b** (50 mg, yield: 44.16%). MS m/z $[M+H]^+$: 371.2.

3. (Compound **159c)**

To a reaction flask were added compound **159b** (50 mg, 0.13 mmol), N-chlorosuccinimide (170 mg, 1.3 mmol), acetic acid (3 mL), and water (1 mL). The mixture was stirred at room temperature for 3 h. After the reaction was completed as detected by LCMS, water (20 mL) was added to the reaction solution, and the resulting mixture was adjusted to pH 7-8 with a saturated sodium carbonate solution, extracted with dichloromethane (30 mL × 2), and subjected to liquid separation. The extract was washed with water and saturated brine, concentrated, and purified by silica gel column chromatography (ethyl acetate/dichloromethane: 30%-80%) to give a product **159c** (20 mg, yield: 47.75%) as a yellow oil. MS m/z $[M+H]^+$: 311.1.

4. (Compound **159d)**

To a reaction flask were added compound **159c** (20 mg, 0.064 mmol), hydrazine hydrate (0.1 mL), and ethanol (2 mL). The mixture was heated to 80 °C and stirred for 2 h. After the reaction was completed as detected by LCMS, the reaction solution was concentrated to give a crude product **159d** (19 mg, yield: 100%), which was directly used in the next step. MS m/z $[M+H]^+$: 297.1.

5. (Compound **159c**)

To a reaction flask were added compound **159d** (10 mg, 0.034 mmol), triethylamine (10 mg, 0.099 mmol), and dichloromethane (2 mL). Difluoroacetic anhydride (12 mg, 0.069 mmol) was added dropwise at 0 °C. The mixture was stirred for 2 h. After the reaction was completed as detected by LCMS, the reaction solution was concentrated to give **159e** (10 mg, yield: 79.3%) as a pale yellow product. MS m/z $[M+H]^+$: 375.1.

6. (Compound **159)**

To a reaction flask were added compound **159e** (10 mg, 0.027 mmol), *p*-toluenesulfonyl chloride (3 mg, 0.032 mmol), triethylamine (8 mg, 0.081 mmol), and acetonitrile (2 mL) at 0 °C. The mixture was stirred at 25 °C for 2 h. After the reaction was completed as detected by LCMS, the reaction solution was filtered and purified by reversed-phase column chromatography to give a product **159** (2.6 mg, yield: 27.31%) as an off-white solid.

$^1$H NMR (400 MHz, DMSO-*$d_6$)* δ 9.54 (d, J = 2.0 Hz, 1H), 8.91 (d, J = 2.0 Hz, 1H), 7.29 (t, J = 51.6 Hz, 1H), 4.65 (s, 2H), 4.00 (q, J = 7.6 Hz, 1H), 2.12 (s, 2H), 2.02 - 1.81 (m, 4H), 1.73 (dd, J = 7.3, 4.4Hz, 2H).

**Example 50: (Compound 122)**

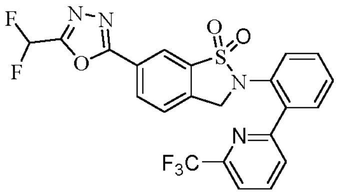

Referring to the synthetic method for compound **72** in Example 19, 4-bromo-1-methyl-1*H*-pyrazole was replaced with 2-bromo-6-trifluoromethylpyridine in step 1 to give **122** as a white solid.

$^1$H NMR (400 MHz, DMSO-*$d_6$)* δ 8.43 (d, *J* = 8.4 Hz, 2H), 8.12 (t, *J* = 7.8 Hz, 1H), 8.04 *(d, J*= 7.9 Hz, 1H), 7.96 (d, *J* = 8.1 Hz, 1H), 7.87 - 7.81 (m, 3H), 7.75 - 7.46 (m, 3H), 5.18 (s, 2H).

**Biological Assays**

**Experimental Example 1.**

The inhibitory activity of the compounds of the present application against HDAC6 was evaluated by an enzymatic activity assay.

HDAC6 (Abcam), test compounds, and a 20 μM substrate solution (Ac-GAK(Ac)-AMC) were each added to a 384-well plate, and the plate was incubated at 37 °C for 30 min. 1 μM Trypsin and 10 μM TSA were added, and the plate was incubated at room temperature for 15 min. The fluorescence intensity at an excitation wavelength of 360 nm and an emission wavelength of 455 nm was measured, and then the inhibition rate at each concentration was calculated. $IC_{50}$ was obtained by fitting using GraphPadPrism 7.0 software.

The obtained experimental results are shown in Table 1.

Table 1

| Compound No. | $IC_{50}$ (nM) | Compound No. | $IC_{50}$ (nM) | Compound No. | $IC_{50}$ (nM) | Compound No. | $IC_{50}$ (nM) |
|---|---|---|---|---|---|---|---|
| **1** | 59 | **2** | 5.7 | **3** | 46.1 | **4** | 154.1 |
| **6** | 395 | **7** | 56.7 | **10** | 43.1 | **20** | 224.1 |
| **21** | 742 | **24** | 193.8 | **39** | 261.1 | **40** | 251.8 |
| **49** | 819.6 | **55** | 48.4 | **60** | 490.5 | **61** | 63 |
| **70i-B** | 37.9 | **72** | 398.8 | **79i-A** | 117.9 | **79i-B** | 75.9 |
| **70i-A** | 136.7 | **70i-B-A** | 216.3 | **70i-B-B** | 108.6 | **84j-A** | 42.7 |
| **84j-B** | 112.6 | **84j-A-A** | 210.5 | **84j-A-B** | 23.1 | **88j-A** | 131.8 |
| **88j-B** | 668 | **88j-A-A** | 584 | **88j-A-B** | 160 | **92** | 164.2 |
| **93** | 133.4 | **94** | 203 | **95j-A** | 230.8 | **95j-B** | 29.9 |
| **95j-B-A** | 66.8 | **95j-B-B** | 183.2 | **99j-A** | 148.5 | **99j-B** | 138.1 |
| **99j-A-A** | 205 | **99j-A-B** | 73.9 | **103j-A** | 285.2 | **103j-B** | 108.1 |
| **103j-C** | 226 | **103j-D** | 231.2 | **109** | 89.8 | **110** | 325.2 |

(continued)

| Compound No. | $IC_{50}$ (nM) | Compound No. | $IC_{50}$ (nM) | Compound No. | $IC_{50}$ (nM) | Compound No. | $IC_{50}$ (nM) |
|---|---|---|---|---|---|---|---|
| **112** | 141.2 | **114** | 97.5 | **116** | 172.8 | **117** | 449.3 |

**Experimental Example 2.**

The inhibitory activity of the compounds of the present application on HDAC1-5 and 7-11 was evaluated by an enzymatic activity assay.

HDAC proteins of different subtypes (purchased from BPS), test compounds, and substrate solutions at concentrations of 2.5-40 μM (purchased from BPS) were each added to a 384-well plate, and the plate was incubated at 37 °C for 30 min. 1 μM Trypsin and 10 μM TSA were added, and the plate was incubated at room temperature for 15 min. The fluorescence intensity at an excitation wavelength of 360 nm and an emission wavelength of 455 nm was measured, and then the inhibition rate at each concentration was calculated. $IC_{50}$ was obtained by fitting using GraphPadPrism 7.0 software.

The obtained experimental results are shown in Tables 2 and 3.

Table 2

| | HADC1 Inhibition rate (%) | | | HADC1 Inhibition rate (%) | |
|---|---|---|---|---|---|
| Compound No. | 10 μM | 1 μM | Compound No. | 10 μM | 1 μM |
| **1** | -3.17 | 8.57 | **2** | -3.91 | -11.89 |

Table 3

| Compound No. | $IC_{50}$ (μM) | | | | | | | | | |
|---|---|---|---|---|---|---|---|---|---|---|
| | HDAC1 | HDAC2 | HDAC3 | HDAC4 | HDAC5 | HDAC7 | HDAC8 | HDAC9 | HDAC 10 | HDAC 11 |
| **2** | > 50 | > 50 | > 50 | > 50 | > 50 | > 50 | > 50 | > 50 | > 50 | > 50 |

**Experimental Example 3. Assay on Acetylation Level of α-Tublin**

A375/HCT116/HCC827 cells at a logarithmic growth phase were taken, digested with a cell lysis buffer (trypsin), centrifuged, counted, and plated in a 96-well plate at an appropriate cell density (30,000 cells/well) at 100 μL/well, and an appropriate amount of PBS was added around the plate for water sealing. The next day, the cells were treated with the compounds at different concentrations (starting at 100 μM, 5-fold dilution, 9 concentration gradients). After 6 h, the medium was pipetted out and the cells were washed twice with 100 μL of PBST. The cells were then immobilized in 4% paraformaldehyde, and the plate was incubated at room temperature for 20 min. The stationary liquid was discarded and the cells were washed with PBST. 150 μL of special blocking buffer (containing 1% Triton-100) was added to each well for blocking at room temperature for 2 h, and the plate was washed with PBS to remove excess blocking buffer. α-tubulin (DM1A) mouse mAb and acetyl-α-tubulin (Lys40) (D20G3) XP rabbit mAb were diluted at a 1:2000 ratio with a blocking buffer containing 0.033% Triton-100. The dilutions were added to the 96-well plate and the plate was incubated overnight at 4 °C. Meanwhile, a blank control group was set. The next day, the plate was washed twice with PBST. Secondary anti-mouse IgG (H + L) (DyLight™ 680 conjugate) and anti-rabbit IgG (H + L) (DyLight™ 800 conjugate) were diluted at a 1:2000 ratio with a blocking buffer containing 0.033% Triton-100. The dilutions were added to a 96-well plate and the plate was incubated at room temperature for 2 h. The plate was washed twice with PBST and once with PBS. Fluorescence signals at 700 nm and 800 nm were detected with a Li-COR Odyssey two-color near-infrared laser imager.

The obtained experimental results are shown in Table 4.

Table 4

| Cells | Compound No. | $EC_{50}$ (nM) |
|---|---|---|
| A375 | **1** | 392.6±192.4 |
| | **2** | 202.5±114.8 |
| HCT116 | **1** | 261.9 |

(continued)

| Cells | Compound No. | $EC_{50}$ (nM) |
|---|---|---|
| | **2** | 139.9±39.1 |
| HCC827 | **1** | 337.9 |
| | **2** | 287.5±49.3 |

| Compound No. | HCT116 cell $EC_{50}$ (nM) | Compound No. | HCT116 cell $EC_{50}$ (nM) | Compound No. | HCT116 cell $EC_{50}$ (nM) | Compound No. | HCT116 cell $EC_{50}$ (nM) |
|---|---|---|---|---|---|---|---|
| **1** | 261.9 | **2** | 139.9±39.1 | **7** | 245.2 | **10** | 262.2 |
| **13** | 133.2 | **14** | 123.8 | **61** | 194.9 | **65** | 467.4 |
| **66** | 322.1 | **70i-B-B** | 926.7 | **84j-A** | 543.9 | **87j-A-B** | 354.9 |
| **88j-A-B** | 618.5 | **113** | 238.9 | **114** | 350.9 | | |

**Experimental Example 4. Stability Test**

Phase I metabolic stability of the test compounds in liver microsomes of CD-1 mice (MLMs), Sprague-Dawley rats (RLMs), beagle dogs (DLMs), cynomolgus monkeys (CLMs), and humans (HLMs) was evaluated.

**Experimental system:**

The animal and human liver microsomes used in the test system were purchased from Xenotech, Corning, or other qualified suppliers and stored in a freezer at a temperature below -60 °C prior to use.

**Brief introduction of the experiment:**

The test samples and control compounds were separately incubated with animal and human liver microsomes at 37 ± 1 °C for a period of incubation time up to 60 min. The samples were removed at the indicated time points, and the reaction was stopped with acetonitrile or other organic solvents containing an internal standard. After centrifugation, the resulting supernatants were assayed by liquid chromatography-tandem mass spectrometry (LC-MS/MS).

**Experimental method:**

1. Preparation of buffer

73.21 g of dipotassium hydrogenphosphate trihydrate and 10.78 g of potassium dihydrogenphosphate were dissolved in 4000 mL of ultrapure water. The solution was adjusted to pH 7.40 ± 0.10 with 10% phosphoric acid or 1 M potassium hydroxide to make a final concentration of 100 mM.

2. Preparation of working solution

The test sample powder was prepared into a stock solution at a certain concentration with DMSO or other organic solvents, which was then further diluted with a proper organic solvent.

Control compounds testosterone, diclofenac, and propafenone were prepared into a 10 mM stock solution with DMSO, which was then further diluted with a proper organic solvent.

3. Preparation of liver microsome solution

Microsomes of each species were diluted into a 2× working solution with a 100 mM potassium phosphate buffer. The final concentration of the microsomes in the reaction system was 0.5 mg/mL.

4. Preparation of reduced nicotinamide adenine dinucleotide phosphate (NADPH) regeneration system

An appropriate amount of nicotinamide adenine dinucleotide phosphate (NADP) and isocitric acid (ISO) powder was weighed out, dissolved in a magnesium chloride solution, and shaken and mixed well. An appropriate amount of isocitrate dehydrogenase (IDH) was added, and the mixture was mixed well by gently turning upside down. The final concentrations in the reaction system were: 1 mM NADP, 1 mM magnesium chloride, 6 mM ISO, and 1 unit/mL IDH.

5. Preparation of stop solution

The stop solution was prepared with acetonitrile or other organic solvent containing an internal standard (tolbutamide or other suitable compounds). The prepared stop solution was stored in a freezer at a temperature of 2-8 °C.

6. Incubation process

Incubation was performed in a 96-well plate. Eight incubation plates were prepared and designated T0, T5, T15, T30, T45, T60, Blank60, and NCF60, respectively. The first 6 plates corresponded to reaction time points of 0, 5, 15, 30, 45, and 60 min, respectively. No test sample or control compound was added to the Blank60 plate, and samples were taken after 60 min of incubation. In the NCF60 plate, incubation was performed for 60 min using the potassium phosphate buffer instead of the NADPH regeneration system solution. Three replicates were made for samples in all conditions.

The microsomes and the test sample or control compound were mixed, and then the incubation plates Blank60, T5, T15, T30, T45, and T60 except for T0 and NCF60 were placed in a 37 °C water bath for preincubation for about 10 min. In the incubation plate T0, a stop solution was first added, followed by addition of the NADPH regeneration system working solution, and 98 μL of the potassium phosphate buffer was added to each sample well of the incubation plate NCF60 to initiate the reaction. After the pre-incubations of the incubation plates Blank60, T5, T15, T30, T45, and T60 were completed, 98 μL of the NADPH regeneration system working solution was added to each sample well to initiate the reaction. The reaction temperature was 37 ± 1 °C, the final volume of the reaction was 200 μL, and the reaction system included 0.5 mg/mL microsomes, 1.0 μM substrate, 1 mM NADP, 6 mM ISO, and 1 unit/mL IDH.

At 5 min, 15 min, 30 min, 45 min, and 60 min, a cold stop solution containing an internal standard was added to the reaction plate to stop the reaction.

All the reaction plates after the reaction was stopped were shaken well and centrifuged at 3220× g at 4 °C for 20 min. After the supernatant was diluted according to a certain proportion, LC-MS/MS analysis was performed.

**Sample analysis**

Sample analysis was performed by the liquid chromatography-tandem mass spectrometry (LC-MS/MS) method without standard curves and quality control samples. Semi-quantitative determinations were performed according to the ratio of the analyte peak area to the internal standard peak area. The retention times of the analyte and internal standard, the chromatogram acquisitions and the integrals of the chromatograms were processed with software Analyst (Sciex, Framingham, Massachusetts, USA).

The CV for the internal standard peak area in each matrix in each analysis batch should be within 20%.

**Data analysis**

The *in vitro* elimination rate constant ke of the compound was obtained by converting the ratio of the peak area of the compound to the internal standard peak area in the following formula into the remaining rate:

$$\text{Remaining rate (\%)} = \frac{\text{Peak area ratio of compound to internal standard at any time point}}{\text{Peak area ratio of compound to internal standard at 0 min}} \times 100$$

$$C_t = C_0 \bullet e^{-k_e \bullet t}$$

*when*

$$C_t = \tfrac{1}{2} C_0,$$

$$T_{1/2} = \frac{Ln\ 2}{k_e} = \frac{0.693}{k_e}$$

$CL_{int\ (mic)}$ = 0.693 / $T_{1/2}$ / microsome protein content (microsome concentration during incubation, mg/mL)
$CL_{int\ (liver)}$ = $CL_{int\ (mic)}$ × microsome protein amount in liver (mg/g) × liver-to-body weight ratio

According to the well stir model, the hepatic intrinsic clearance and hepatic clearance can be converted through the following formula.

$$CL_{(liver)} = (CL_{int(liver)} * Q_h) / (CL_{int(liver)} + Q_h)$$

Parameters in the formula are shown in the table below.

| Species | Liver-to-body weight ratio (g/kg body weight) [1-2] | Liver blood flow volume ($Q_h$) (mL/min/kg) [1-2] | Microsome protein amount (mg/g liver weight) |
|---|---|---|---|
| Mice | 88 | 90.0 | |
| Rat | 40 | 55.2 | |
| Dog | 32 | 30.9 | 45 |
| Monkey | 30 | 43.6 | |
| Human | 20 | 20.7 | |

[1] Brian Davies and Tim Morris, Physiological Parameters in Laboratory Animals and Human. Pharmaceutical Research, Vol. 10 No.7, 1993
[2] Journal of Pharmacology and Experimental Therapeutics, 1997, 283(1): 46-58.

The obtained experimental results are shown in Table 5.

Table 5

| Compound No. | | HLM | RLM | MLM | DLM | CLM |
|---|---|---|---|---|---|---|
| 1 | $R^2$ | 0.2047 | 0.9052 | 0.2775 | 0.0187 | 0.3709 |
| | $T_{1/2}$ (min) | >145 | 96.6 | >145 | >145 | >145 |
| | $CL_{int}$ (mic) (μL/min/mg) | <9.6 | 14.4 | <9.6 | <9.6 | <9.6 |
| | $CL_{int}$ (liver) (mL/min/kg) | <8.6 | 25.8 | <38.0 | <13.8 | <13.0 |
| | Remaining rate (T = 60 min) | 79.0% | 68.9% | 79.8% | 86.6% | 79.1% |
| | Remaining rate (NCF = 60 min) | 83.4% | 100.2% | 97.6% | 98.9% | 94.8% |
| | | | | | | |
| 2 | $R^2$ | 0.7713 | 0.8854 | 0.7684 | 0.5254 | 0.8196 |
| | $T_{1/2}$ (min) | >145 | >145 | >145 | >145 | >145 |
| | $CL_{int}$ (mic) (μL/min/mg) | <9.6 | <9.6 | <9.6 | <9.6 | <9.6 |
| | $CL_{int}$ (liver) (mL/min/kg) | <8.6 | <17.3 | <38.0 | <13.8 | <13.0 |
| | Remaining rate (T = 60 min) | 86.1% | 76.4% | 84.8% | 85.8% | 88.8% |
| | Remaining rate (NCF = 60 min) | 97.3% | 93.0% | 99.8% | 90.9% | 101.5% |
| 61 | $R^2$ | | | | | |
| | $T_{1/2}$ (min) | 239.35 | 128.29 | 294.13 | | |

(continued)

| Compound No. | | HLM | RLM | MLM | DLM | CLM |
|---|---|---|---|---|---|---|
| | $CL_{int}$ (mic) (μL/min/mg) | | | | | |
| | $CL_{int}$ (liver) (mL/min/kg) | 7.26 | 19.36 | 18.55 | | |
| | Remaining rate (T = 60 min) | 85.62% | 72.95% | 85.14% | | |
| | Remaining rate (NCF = 60 min) | 86.25% | 88.24% | 83.81% | | |
| **83** | $R^2$ | | | | | |
| | T1/2 (min) | 270.54 | 42.37 | 131.73 | | |
| | $CL_{int}$ (mic) (μL/min/mg) | | | | | |
| | $CL_{int}$ (liver) (mL/min/kg) | 6.43 | 58.62 | 41.43 | | |
| | Remaining rate (T = 60 min) | 82.70% | 36.99% | 72.27% | | |
| | Remaining rate (NCF = 60 min) | 84.68% | 88.49% | 84.39% | | |

**Experimental Example 5. Pharmacokinetic Test in Mice**

In this example, the pharmacokinetic behavior of the compounds after intravenous injection (IV) and intragastric (PO) administration in BALB/c mice was tested.

On the day of administration, mice were weighed for actual body weight and the administration volume was calculated. Each compound was tested in two groups with 3 mice in each group, one group administered by single intravenous injection and the other group of mice by single intragastric administration. Whole blood samples were collected via the orbit at prescribed time points (0.25 h, 0.5 h, 1 h, 2 h, 4 h, 8 h, and 24 h after administration). After blood sample collection, the samples were immediately transferred to labeled commercial sample tubes containing K2-EDTA (0.85-1.15 mg), followed by centrifugation (3200× g, 4 °C, 10 min) and plasma collection. The plasma was transferred to a pre-cooled centrifuge tube, frozen in dry ice, and stored in an ultra-low temperature freezer at -60 °C or lower until the LC-MS/MS analysis.

Plasma concentrations were determined by a LC-MS/MS method. Plasma drug concentration data for compounds **1** and **2** were processed in a non-compartmental model using WinNonlin Version 6.3 (Pharsight, Mountain View, CA) pharmacokinetic software. The relevant pharmacokinetic parameters were calculated by a linear logarithmic trapezoid method.

The obtained experimental results are shown in Table 6.

Table 6

| Compound No. | | | IV@2mg/kg | PO@10mg/kg | PO@30mg/kg | PO@100mg/kg |
|---|---|---|---|---|---|---|
| | AUC(0-t) | nM*h | 9879.84 | 45181.61 | 73081.85 | 171466 |
| | AUC(0-∞) | nM*h | 9879.841 | 45181.61 | 73081.85 | 171491.9 |
| | MRT(0-∞) | h | 2.194 | 3.347 | 3.652 | 3.715 |
| | t1/2z | h | 1.062 | 0.919 | 0.918 | 1.292 |
| **1** | Vz | L/kg | 0.684 | | | |
| | Tmax | h | | 1 | 0.417 | 1.5 |
| | CLz | L/h/kg | 0.426 | | | |
| | C0/Cmax | nM | 4520.52 | 8971.316 | 17530.96 | 30483.15 |
| | F | % | | 91.46 | 49.31 | 34.72 |
| | | | | | | |
| | AUC(0-t) | nM*h | 12552.15 | 77690.92 | 182495.53 | 600707.51 |
| | AUC(0-∞) | nM*h | 12552.15 | 77770.78 | 182495.53 | 600707.51 |
| | MRT(0-∞) | h | 2.35 | 4.50 | 3.66 | 4.33 |
| | t1/2z | h | 1.01 | 1.62 | 0.87 | 0.81 |

(continued)

| Compound No. | | | IV@2mg/kg | PO@10mg/kg | PO@30mg/kg | PO@100mg/kg |
|---|---|---|---|---|---|---|
| **2** | Vz | L/kg | 0.55 | | | |
| | Tmax | h | | 0.50 | 0.67 | 1.33 |
| | CLz | L/h/kg | 0.36 | | | |
| | C0/Cmax | nM | 6749.28 | 13883.48 | 35033.09 | 87871.06 |
| | F | % | | 123.92 | 96.93 | 95.71 |
| **61** | AUC(0-t) | nM*h | 12782.76 | 57008.607 | | |
| | AUC(0-∞) | nM*h | 15375.84 | 73418.487 | | |
| | MRT(0-∞) | h | 4.379 | 5.305 | | |
| | t1/2z | h | 3.12 | 3.551 | | |
| | Vz | L/kg | 1.175 | | | |
| | Tmax | h | | 1 | | |
| | CLz | L/h/kg | 0.261 | | | |
| | C0/Cmax | nM | 4522.401 | 12550.55 | | |
| | F | % | | 89.2 | | |
| **83** | AUC(0-t) | nM*h | 8606.529 | 50510.891 | | |
| | AUC(0-∞) | nM*h | 8781.805 | 63718.102 | | |
| | MRT(0-∞) | h | 2.064 | 4.569 | | |
| | t1/2z | h | 1.493 | 3.323 | | |
| | Vz | L/kg | 1.01 | | | |
| | Tmax | h | | 0.5 | | |
| | CLz | L/h/kg | 0.469 | | | |
| | C0/Cmax | nM | 5273.247 | 12030.21 | | |
| | F | % | | 117.4 | | |

| Compound No. | | | IV@1mg/kg | PO@5mg/kg |
|---|---|---|---|---|
| **10** | AUC(0-t) | nM*h | 3484.4 | 19868.2 |
| | AUC(0-∞) | nM*h | 3676.0 | 21527.0 |
| | MRT(0-∞) | h | 2.5 | 3.3 |
| | t1/2z | h | 1.9 | 2.1 |
| | Vz | L/kg | 1.5 | |
| | Tmax | h | | 0.5 |
| | CLz | L/h/kg | 0.5 | |
| | C0/Cmax | nM | 1718.9 | 4774.7 |
| | F | % | | 117.1 |
| **13** | AUC(0-t) | nM*h | 4418.6 | 21397.5 |
| | AUC(0-∞) | nM*h | 4530.5 | 21968.1 |
| | MRT(0-∞) | h | 1.846 | 2.63 |
| | t1/2z | h | 1.52 | 1.52 |
| | Vz | L/kg | 1.05 | |

(continued)

| Compound No. | | | IV@1mg/kg | PO@5mg/kg |
|---|---|---|---|---|
| | Tmax | h | | 0.5 |
| | CLz | L/h/kg | 0.48 | |
| | C0/Cmax | nM | 3053.4 | 7127.4 |
| | F | % | | 97.0 |
| **14** | AUC(0-t) | nM*h | 4222.2 | 14822.7 |
| | AUC(0-∞) | nM*h | 4274.3 | 15232.1 |
| | MRT(0-∞) | h | 1.497 | 1.876 |
| | t1/2z | h | 1.277 | 1.48 |
| | Vz | L/kg | 0.928 | |
| | Tmax | h | | 0.5 |
| | CLz | L/h/kg | 0.504 | |
| | C0/Cmax | nM | 3145.9 | 7271.1 |
| | F | % | | 71.27 |
| **80** | AUC(0-t) | nM*h | 5045.6 | 25524.5 |
| | AUC(0-∞) | nM*h | 5279.7 | 34006.5 |
| | MRT(0-∞) | h | 2.6 | 5.6 |
| | t1/2z | h | 1.8 | 4.2 |
| | Vz | L/kg | 1.0 | |
| | Tmax | h | | 1.0 |
| | CLz | L/h/kg | 0.4 | |
| | C0/Cmax | nM | 1913.6 | 7169.9 |
| | F | % | | 101.2 |
| **87** | AUC(0-t) | nM*h | 1622.8 | 15052.8 |
| | AUC(0-∞) | nM*h | 1768.2 | 15544.8 |
| | MRT(0-∞) | h | 0.8 | 2.3 |
| | t1/2z | h | 0.6 | 1.5 |
| | Vz | L/kg | 0.9 | |
| | Tmax | h | | 1.0 |
| | CLz | L/h/kg | 1.1 | |
| | C0/Cmax | nM | 1894.4 | 5747.0 |
| | F | % | | 175.8 |
| **93** | AUC(0-t) | nM*h | 1074.0 | 4562.9 |
| | AUC(0-∞) | nM*h | 1081.7 | 4565.5 |
| | MRT(0-∞) | h | 0.6 | 1.2 |
| | t1/2z | h | 0.7 | 0.7 |
| | Vz | L/kg | 1.9 | |
| | Tmax | h | | 0.5 |
| | CLz | L/h/kg | 2.0 | |
| | C0/Cmax | nM | 2106.4 | 3413.6 |

(continued)

| Compound No. | | | IV@1mg/kg | PO@5mg/kg |
|---|---|---|---|---|
| | F | % | | 84.4 |
| 113 | AUC(0-t) | nM*h | 1702.7 | 8637.18 |
| | AUC(0-∞) | nM*h | 1711.1 | 8711.55 |
| | MRT(0-∞) | h | 0.542 | 1.469 |
| | t1/2z | h | 0.605 | 1.149 |
| | Vz | L/kg | 1.026 | |
| | Tmax | h | | 0.25 |
| | CLz | L/h/kg | 1.175 | |
| | C0/Cmax | nM | 3469.3 | 5133.66 |
| | F | % | | 101.8 |

**Experimental Example 6. Pharmacokinetic Test in Rats**

In this example, the pharmacokinetic behavior of the compounds after intravenous injection (IV) and intragastric (PO) administration in SD rats was tested.

On the day of administration, rats were weighed for actual body weight and the administration volume was calculated. Each compound was tested in two groups with 3 rats in each group, one group administered by single intravenous injection and the other by single intragastric administration. Whole blood samples were collected via the jugular vein at prescribed time points (0.25, 0.5, 1, 2, 4, 8, and 24 h after administration). After blood sample collection, the samples were immediately transferred to labeled commercial sample tubes containing K2-EDTA (0.85-1.15 mg), followed by centrifugation (3200× g, 4 °C, 10 min) and plasma collection. The plasma was transferred to a pre-cooled centrifuge tube, frozen in dry ice, and stored in an ultra-low temperature freezer at -60 °C or lower until the LC-MS/MS analysis.

Plasma concentrations were determined by a LC-MS/MS method. Plasma drug concentration data for the compounds were processed in a non-compartmental model using WinNonlin Version 6.3 (Pharsight, Mountain View, CA) pharmacokinetic software. The relevant pharmacokinetic parameters were calculated by a linear logarithmic trapezoid method.

**Experimental Example 7. Brain-to-Blood Drug Concentration Ratio of Mice**

On the day of administration, mice were weighed for actual body weight and the administration volume was calculated. In each group of 9 mice, the compound was administered by single intravenous (IV) injection or intragastric (PO) administration. Whole blood samples were collected via the orbit at prescribed time points. After blood sample collection, the samples were immediately transferred to labeled commercial sample tubes containing K2-EDTA (0.85-1.15 mg), followed by centrifugation (3200× g, 4 °C, 10 min) and plasma collection. The plasma was transferred to a pre-cooled centrifuge tube, frozen in dry ice, and stored in an ultra-low temperature freezer at -60 °C or lower until the LC-MS/MS analysis. 20 μL of a plasma sample or brain tissue homogenate was taken and 80 μL of an internal standard solution (50 ng/mL solution of propafenone in acetonitrile) was added. The plate was sealed with a film sealer at 165 °C, shaken on a micro oscillator (maximum vibration speed) for 10 min, and centrifuged at 4000 rpm for 20 min using a low-speed benchtop centrifuge, 10 μL of supernatant was transferred and 90 μL of acetonitrile was added. The mixture was vortexed and mixed well, and then the plate was sealed with the film sealer at 165 °C. After the mixture was vortexed and mixed well, 1 μL of supernatant was taken for LC-MS/MS analysis. Plasma drug concentration data for the compounds were processed in a non-compartmental model using WinNonlin Version 6.3 (Pharsight, Mountain View, CA) pharmacokinetic software. The relevant pharmacokinetic parameters were calculated by a linear logarithmic trapezoid method.

Data processing: brain-to-blood drug concentration ratio of mice at the same time point = drug concentration in brain tissue/drug concentration in plasma

| Compound No. | Administration dose | Time (h) | Brain drug concentration (ng/mL) | Blood drug concentration (ng/mL) | **Brain/blood drug concentration ratio** |
|---|---|---|---|---|---|
| **1** | 2 mg/kg IV | 1 | 285 | 986 | **0.29** |
| | | 4 | 161 | 453 | **0.36** |
| | 10 mg/kg PO | 1 | 759 | 4271 | **0.18** |
| | | 4 | 373 | 2503 | **0.15** |
| **2** | 2 mg/kg IV | 1 | 268 | 1090 | **0.26** |
| | | 4 | 149 | 595 | **0.25** |
| | 10 mg/kg PO | 1 | 882 | 4993 | **0.18** |
| | | 4 | 435 | 2833 | **0.15** |
| **4** | 5mg/kg PO | 2 | 190 | 2263 | **0.08** |
| **10** | 5mg/kg PO | 2 | 338 | 2018 | **0.17** |
| | | 8 | 80 | 277 | **0.29** |
| **13** | 5mg/kg PO | 1 | 218 | 2950 | **0.07** |
| | | 2 | 174 | 1776 | **0.09** |
| **14** | 5mg/kg PO | 1 | 85.56 | 2453 | **0.03** |
| | | 2 | 42.755 | 1382 | **0.03** |
| **80** | 5mg/kg PO | 2 | 160 | 1908 | **0.08** |
| | | 8 | 72 | 695 | **0.10** |
| **87** | 5mg/kg PO | 2 | 298 | 1056 | **0.28** |
| **93** | 5mg/kg PO | 2 | 27 | 252 | **0.11** |
| **113** | 5mg/kg PO | 1 | 44.8 | 1403 | **0.03** |
| | | 2 | 16.209 | 930 | **0.02** |

**Experimental Example 8. hERG Inhibition Test**

The HEK293 cells were cultured in a DMEM medium containing 10% fetal bovine serum and 0.8 mg/mL G418 at 37 °C with 5% $CO_2$. The cells were digested by TrypLE™ Express and centrifuged. The cell density was adjusted to $2 \times 10^6$ cells/mL. The cells were then gently mixed for 15-20 min using a shaker equilibrated at room temperature, and subjected to patch clamping on a machine. The medium of the prepared cells was replaced with extracellular fluid. The intracellular fluid and the extracellular fluid were taken from the fluid pool, and added to the intracellular fluid pool and the cell and the test substance pool of the QPlate chip, respectively. The voltage stimulation of the whole-cell hERG potassium current was recorded by the whole-cell patch clamp, and the test data was collected and stored by Qpatch. The compound was subjected to a 3-fold dilution starting from 30 μM to obtain 6 concentration points, each for two administrations over a period of at least 5 min. The current detected in compound-free extracellular fluid for each cell was served as its own control group, and the detection was repeated at least twice independently using two cells per concentration. All electrophysiological experiments were performed at room temperature.

For data analysis, the current after the action of each drug concentration and the current of the blank control were standardized ( $\frac{\text{Peak tail current}_{\text{compound}}}{\text{Peak tail current}_{\text{vehicle}}}$ ), and then the inhibition rate corresponding to each drug concentration was calculated ( $1-\frac{\text{Peak tail current}_{\text{compound}}}{\text{Peak tail current}_{\text{vehicle}}}$ ). The mean and standard error were calculated for each concentration, and the half maximal inhibitory concentration of each compound was calculated: $Y = \text{Bottom} + \frac{\text{Top - Bottom}}{1 + 10\text{^}((\text{LogIC}_{50} - C)*\text{HillSlope})}$ . The dose-dependent effect was fitted non-linearly using the above equation, wherein Y represents the inhibition rate, C represents the concentration of the test substance, $IC_{50}$ is the half maximal inhibitory concentration, and HillSlope

represents the Hill coefficient. Curve fitting and calculation of $IC_{50}$ were performed by Graphpad software.

**Experimental Example 9. Cytochrome Oxidase P450 Inhibition Test**

1) Preparation of buffer:

100 mM K-Buffer: 9.5 mL of stock solution A was mixed with 40.5 mL of stock solution B, the total volume was adjusted to 500 mL with ultrapure water, and the buffer was titrated to pH 7.4 with KOH or $H_3PO_4$.
Stock solution A (1 M potassium dihydrogen phosphate): 136.5 g of potassium dihydrogen phosphate in 1 L of water.
Stock solution B (1 M potassium dihydrogen phosphate): 174.2 g of potassium dihydrogen phosphate in 1 L of water.

2) Preparation of test substance

The test substance powder was prepared into a stock solution with a certain concentration using DMSO or other organic solvents, which was then further diluted using a proper organic solvent.

3) *In vitro* incubation

The extracorporeal incubation system for CYP450 enzyme metabolism phenotype study was biochemical reaction of a prepared liver microsome, a redox coenzyme, and an enzyme-specific selective inhibitor in simulated physiological temperature and environment.

4) Detection of parent drug or metabolite

The concentration of the parent drug or a metabolite thereof in the incubation solution was determined by LC-MS/MS.

**Experimental Example 10. Human, Rat, And Mouse Plasma Protein Binding Assay**

(1) Preparation of solution: 0.05 M sodium phosphate and 0.07 M NaCl buffers, a control compound (the final concentration of warfarin and quinidine in the system was 1 $\mu$M), and an administration solution (the final concentration was 1 $\mu$M) were prepared.
(2) Pretreatment of dialysis membrane: Firstly, the membrane was soaked in distilled water for 60 min; then, a 20% (v/v) ethanol solution (diluted with distilled water) was added, and the mixture was soaked for 20 min until the assay was started. The membrane was washed twice with distilled water to remove residual ethanol before use.
(3) Pretreatment of plasma : 1) human, rat, and mouse plasma was thawed at room temperature, and centrifuged at room temperature for 5 min at a maximum speed of 2000$\times$ g after thawing, and the supernatant was retained (freezing and thawing of plasma resulted in fibrous precipitation; plasma samples were stored at room temperature for the rest of the experiments); 2) the pH value of the plasma was measured and adjusted to 7.4 $\pm$ 0.02 by the addition of a small amount of solid powder $NaH_2PO_4$.
(4) Control and test groups: 100 $\mu$L of plasma solution containing a positive control or a test compound was added to the donor end (Donor) of the equilibrium dialysis device, and 100 $\mu$L of blank buffer was added to the receiver end (Receiver).
(5) The equilibrium dialysis device was incubated at 37 °C at 100 rpm for 5 h.
(6) Preparation of $T_0$ samples for control and test groups: 25 $\mu$L of plasma solution containing a positive control or test compound was taken and supplemented with 25 $\mu$L of blank buffer.
(7) After incubation for 5 h in the control group and the test group, 25 $\mu$L of the sample at the donor end was taken and supplemented with 25 $\mu$L of blank buffer; 25 $\mu$L of the sample at the receiver end was taken and supplemented with 25 $\mu$L of blank plasma.
(8) 200 $\mu$L of acetonitrile containing an internal standard was added to all the samples, and the samples were vortexed and shaken for 10 min and then centrifuged at 4000 rpm for 10 min. The prepared samples were sent for UPLC-MS/MS analysis. Note: the internal standard was 40 ng/mL tolbutamide.
(9) Data processing

The plasma protein binding rates of the test compounds were calculated by testing the $T_0$ samples, the samples at the donor end and the receiver end after 5 h of equilibration and dialysis, according to the following formula:

$$\text{Binding rate \%} = 100 \times ([\text{donor end}]5\text{h} - [\text{receiver end}]5\text{h}) / [\text{donor end}]5\text{h}$$

**Experimental Example 11. Ames Assay**

The test strains adopted in the mutagenicity test were Salmonella typhimurium auxotroph strains TA98 and TA100. The test was performed using a 6-well plate in the presence or absence of the S9 metabolic activation system, with both a solvent control group (DMSO) and a positive control group set, with 2 replicate wells per treatment group. Compounds were set at 5 concentrations ranging from 62.5 μg/well to 1000.0 μg/well (equivalent to 312.5 μg/dish to 5000.0 μg/dish in the standard Ames assay). The plate was incubated at 37 °C for 48-72 h, and then bacterial toxicity was measured in each well and the number of mutant colonies in each well was counted. When the number of revertant colonies of the test compound groups is more than twice the number of spontaneous revertant colonies of the solvent control group in a reproducible and dose-dependent manner, the test substance is considered to have positive mutagenicity for the test strain.

In this test, dimethyl sulfoxide (DMSO) was used to dissolve the test substance and served as a negative (solvent) control. In the S9-free group (-S9), 2-nitrofluorene and sodium azide were used as positive controls for TA98 and TA100, respectively; in the S9 group (+S9), 2-aminofluorene was used as a positive control for TA98 and TA100.

| Compound No. | Pollutant mutagenicity test results |
|---|---|
| 61 | Negative |

Various modifications of the present invention in addition to those described herein will be apparent to those skilled in the art from the foregoing description. Such modifications are also intended to fall within the scope of the appended claims. References (including all patents, patent applications, journal articles, books, and any other publications) cited in the present application are incorporated herein by reference in its entirety.

## Claims

1. A compound, or a pharmaceutically acceptable salt, an ester, a stereoisomer, a tautomer, a polymorph, a solvate, a metabolite, an isotopically labeled compound or a prodrug thereof, wherein the compound has a structure of formula (I):

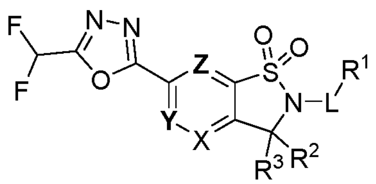

**(I)**

wherein

L is selected from a direct bond, -$C_{1-6}$ alkylene-, -$C_{2-6}$ alkenylene-, and -$C_{2-6}$ alkynylene-;
X is $CR^6$ or N;
Y is $CR^4$ or N;
Z is $CR^5$ or N;
$R^1$ is selected from H, halogen, -OH, -$NH_2$, -CN, -$NO_2$, $C_{1-6}$ alkyl, $C_{2-6}$ alkenyl, $C_{2-6}$ alkynyl, $C_{3-6}$ cyclohydrocarbyl, 3- to 10-membered heterocyclyl, $C_{6-10}$ aryl, 5- to 14-membered heteroaryl, $C_{6-12}$ aralkyl, - $C(=O)R^a$, -$OC(=O)R^a$, -$OC(=O)NR^aR^b$, -$C(=O)OR^a$, -$OR^a$, -$SR^a$, -$S(=O)R^a$, -$S(=O)_2R^a$, -$S(=O)_2NR^aR^b$, - $NR^aR^b$, -$C(=O)NR^aR^b$, -$NR^a$-$C(=O)R^b$, -$NR^a$-$C(=O)OR^b$, -$NR^a$-$S(=O)_2$-$R^h$, -$NR^a$-$C(=O)$-$NR^aR^b$, -$C_{1-6}$ alkylene-$OR^a$, -$C_{1-6}$ alkylene-$NR^aR^b$, and -O-$C_{1-6}$ alkylene-$NR^aR^b$;
$R^2$ and $R^3$ are each independently selected from H, halogen, -OH, -$NH_2$, -CN, -$NO_2$, $C_{1-6}$ alkyl, $C_{2-6}$ alkenyl, $C_{2-6}$

alkynyl, $C_{3-6}$ cyclohydrocarbyl, 3- to 10-membered heterocyclyl, $C_{6-10}$ aryl, 5- to 14-membered heteroaryl, $C_{6-12}$ aralkyl, -C(=O)$R^a$, -OC(=O)$R^a$, -OC(=O)N$R^aR^b$, -C(=O)O$R^a$, -O$R^a$, -S$R^a$, -S(=O)$R^a$, - S$(=O)_2R^a$, -S$(=O)_2$N$R^aR^b$, -N$R^aR^b$, -C(=O)N$R^aR^b$, -N$R^a$-C(=O)$R^b$, -N$R^a$-C(=O)O$R^b$, -N$R^a$-S$(=O)_2$-$R^h$, -N$R^a$-C(=O)-N$R^aR^b$, -$C_{1-6}$ alkylene-O$R^a$, -$C_{1-6}$ alkylene-N$R^aR^b$, and -O-$C_{1-6}$ alkylene-N$R^aR^b$; or $R^2$ and $R^3$ together form oxo (=O); or $R^2$ and $R^3$, together with the carbon atom to which they are attached, form $C_{3-6}$ cyclohydrocarbyl or 3- to 10-membered heterocyclyl;

$R^4$, $R^5$, and $R^6$ are each independently selected from H, halogen, -OH, -$NH_2$, -CN, -$NO_2$, $C_{1-6}$ alkyl, $C_{2-6}$ alkenyl, $C_{2-6}$ alkynyl, $C_{3-6}$ cyclohydrocarbyl, 3- to 10-membered heterocyclyl, $C_{6-10}$ aryl, 5- to 14-membered heteroaryl, $C_{6-12}$ aralkyl, -C(=O)$R^a$, -OC(=O)$R^a$, -OC(=O)N$R^aR^b$, -C(=O)O$R^a$, -O$R^a$, -S$R^a$, -S(=O)$R^a$, - S$(=O)_2R^a$, -S$(=O)_2$N$R^aR^b$, -N$R^aR^b$, -C(=O)N$R^aR^b$, -N$R^a$-C(=O)$R^b$, -N$R^a$-C(=O)O$R^b$, -N$R^a$-S$(=O)_2$-$R^h$, -N$R^a$-C(=O)-N-$R^aR^b$, -$C_{1-6}$ alkylene-O$R^a$, -$C_{1-6}$ alkylene-N$R^aR^b$, and -O-$C_{1-6}$ alkylene-N$R^aR^b$;

$R^a$ and $R^b$, at each occurrence, are each independently selected from H, $C_{1-6}$ alkyl, $C_{3-10}$ cyclohydrocarbyl, 3-to 10-membered heterocyclyl, $C_{6-10}$ aryl, 5- to 14-membered heteroaryl, and $C_{6-12}$ aralkyl;

the alkyl, alkylene, alkenyl, alkenylene, alkynyl, alkynylene, cyclohydrocarbyl, heterocyclyl, aryl, heteroaryl, and aralkyl, at each occurrence, are each optionally substituted with one or more substituents independently selected from: halogen, -OH, =O , -$NH_2$, -CN, -$NO_2$, $C_{1-6}$ alkyl, $C_{3-6}$ cyclohydrocarbyl, 3- to 10-membered heterocyclyl, $C_{6-10}$ aryl, 5- to 14-membered heteroaryl, $C_{6-12}$ aralkyl, -C(=O)$R^c$, -OC(=O)$R^c$, -OC(=O)N$R^cR^d$, -C(=O)O$R^c$, -O$R^c$, -S$R^a$, -S(=O)$R^a$, -S$(=O)_2R^c$, -S$(=O)_2$N$R^cR^d$, -N$R^cR^d$, -C(=O)N$R^cR^d$, -N$R^c$-C(=O)$R^d$, - N$R^c$-C(=O)O$R^d$, -N$R^c$-S$(=O)_2$-$R^d$, -N$R^c$-C(=O)-N$R^cR^d$, -$C_{1-6}$ alkylene-O$R^c$, -$C_{1-6}$ alkylene-N$R^cR^d$, and -O-$C_{1-6}$ alkylene-N$R^cR^d$; the alkyl, cyclohydrocarbyl, heterocyclyl, aryl, heteroaryl, and aralkyl are further optionally substituted with one or more substituents independently selected from: halogen, -OH, =O, - C(=O)O-tert-butyl, -$NH_2$, -CN, -$NO_2$, $C_{1-6}$ alkyl, halogenated $C_{1-6}$ alkyl, -O$C_{1-6}$ alkyl, -O-halogenated $C_{1-6}$ alkyl, $C_{3-6}$ cyclohydrocarbyl, 3- to 10-membered heterocyclyl, $C_{6-10}$ aryl, 5- to 14-membered heteroaryl, and $C_{6-12}$ aralkyl; preferably, the alkyl, cyclohydrocarbyl, heterocyclyl, aryl, heteroaryl, and aralkyl are further optionally substituted with one or more substituents independently selected from: halogen, -OH, =O, - C(=O)O-tert-butyl, -$NH_2$, -CN, -$NO_2$, $C_{1-6}$ alkyl, $C_{3-6}$ cyclohydrocarbyl, 3- to 10-membered heterocyclyl, $C_{6-10}$ aryl, 5- to 14-membered heteroaryl, and $C_{6-12}$ aralkyl;

$R^c$ and $R^d$, at each occurrence, are each independently selected from H, $C_{1-6}$ alkyl, $C_{3-10}$ cyclohydrocarbyl, 3-to 10-membered heterocyclyl, $C_{6-10}$ aryl, 5- to 14-membered heteroaryl, and $C_{6-12}$ aralkyl; the alkyl, cyclohydrocarbyl, heterocyclyl, aryl, heteroaryl, and aralkyl are further optionally substituted with one or more substituents independently selected from: halogen, -OH, =O, -C(=O)O-tert-butyl, -$NH_2$, -CN, -$NO_2$, $C_{1-6}$ alkyl, halogenated $C_{1-6}$ alkyl, $C_{3-6}$ cyclohydrocarbyl, 3- to 10-membered heterocyclyl, $C_{6-10}$ aryl, 5- to 14-membered heteroaryl, and $C_{6-12}$ aralkyl.

**2.** The compound, or the pharmaceutically acceptable salt, the ester, the stereoisomer, the tautomer, the polymorph, the solvate, the metabolite, the isotopically labeled compound or the prodrug thereof according to claim 1, wherein L is a direct bond or -$C_{1-6}$ alkylene-, wherein the alkylene is optionally substituted with one or more substituents independently selected from: -OH, -$OCH_3$, $C_{1-6}$ alkyl, $C_{3-6}$ cyclohydrocarbyl, 3- to 10-membered heterocyclyl, $C_{6-10}$ aryl, and 5- to 14-membered heteroaryl; the alkyl, cyclohydrocarbyl, heterocyclyl, aryl, and heteroaryl are further optionally substituted with one or more halogen or $C_{1-6}$ alkyl; preferably, L is a direct bond or -$C_{1-6}$ alkylene-, wherein the alkylene is optionally substituted with one or more substituents independently selected from: -OH, $C_{3-6}$ cyclohydrocarbyl, 3- to 10-membered heterocyclyl, $C_{6-10}$ aryl, and 5- to 14-membered heteroaryl; the cyclohydrocarbyl, heterocyclyl, aryl, and heteroaryl are further optionally substituted with one or more halogen or $C_{1-6}$ alkyl;

preferably, L is a direct bond, methylene, or ethylene, wherein the methylene and ethylene are optionally substituted with one or more substituents independently selected from: -OH, -$OCH_3$, methyl, cyclopropyl, phenyl, pyrazolyl, pyridinyl, pyrimidinyl, and pyridazinyl; the groups described above are optionally further substituted with one or more halogen and/or methyl;

preferably, L is a direct bond, methylene, or ethylene, wherein the methylene and ethylene are optionally substituted with one or more substituents independently selected from: -OH, cyclopropyl, phenyl, pyrazolyl, and pyridinyl; the groups described above are optionally further substituted with one or more halogen and/or methyl;

preferably, L is a direct bond, -$CH_2$-,

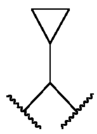 , 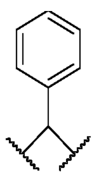 ,

$-CH_2CH_2-$,

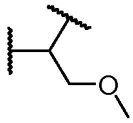, 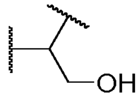,

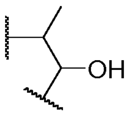, 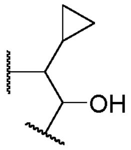, 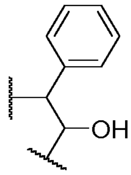, 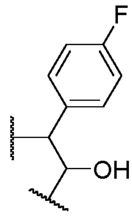, 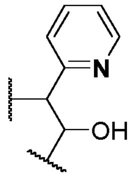, 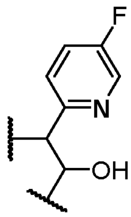, 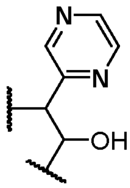,

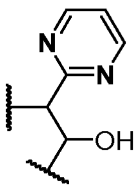, or 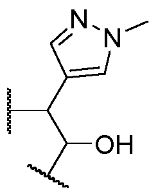;

preferably, L is a direct bond, $-CH_2-$,

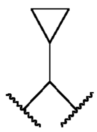,

$-CH_2CH_2-$,

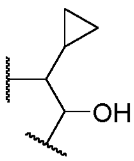, 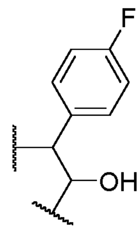, 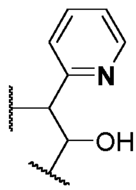,

or

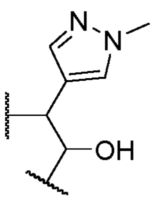.

**3.** The compound, or the pharmaceutically acceptable salt, the ester, the stereoisomer, the tautomer, the polymorph, the solvate, the metabolite, the isotopically labeled compound or the prodrug thereof according to claim 1 or 2, wherein X, Y, and Z are each independently CH, CF, or N.

**4.** The compound, or the pharmaceutically acceptable salt, the ester, the stereoisomer, the tautomer, the polymorph, the solvate, the metabolite, the isotopically labeled compound or the prodrug thereof according to any one of claims 1-3,

wherein $R^1$ is selected from $C_{1-6}$ alkyl, $C_{3-6}$ cyclohydrocarbyl, 3- to 10-membered heterocyclyl, $C_{6-10}$ aryl, and 5- to 14-membered heteroaryl;
the alkyl, cyclohydrocarbyl, heterocyclyl, aryl, and heteroaryl are each optionally substituted with one or more substituents independently selected from: halogen, -OH, -CN, $C_{1-6}$ alkyl, $C_{3-6}$ cyclohydrocarbyl, 3- to 10-membered heterocyclyl, $C_{6-10}$ aryl, 5- to 14-membered heteroaryl, $-C(=O)R^c$, $-OC(=O)R^c$, $-C(=O)OR^c$, - $OR^c$, $-S(=O)_2R^c$, $-NR^cR^d$, $-C(=O)NR^cR^d$, $-NR^c-C(=O)R^d$, $-NR^c-C(=O)OR^d$, $-NR^c-S(=O)_2-R^d$, and $-NR^c-C(=O)-NR^cR^d$; the alkyl, cyclohydrocarbyl, heterocyclyl, aryl, and heteroaryl described above are further optionally substituted with one or more substituents independently selected from: halogen (e.g., fluoro), - OH, =O, $C_{1-6}$ alkyl (e.g., methyl), halogenated $C_{1-6}$ alkyl (e.g., trifluoromethyl), $-OC_{1-6}$ alkyl (e.g., methoxy), and -O-halogenated $C_{1-6}$ alkyl (e.g., trifluoromethoxy); preferably, the alkyl, cyclohydrocarbyl, heterocyclyl, aryl, and heteroaryl are further optionally substituted with one or more substituents independently selected from: halogen (e.g., fluorine), -OH, =O, and $C_{1-6}$ alkyl (e.g., methyl).

**5.** The compound, or the pharmaceutically acceptable salt, the ester, the stereoisomer, the tautomer, the polymorph, the solvate, the metabolite, the isotopically labeled compound or the prodrug thereof according to any one of claims 1-4, wherein $R^c$ and $R^d$, at each occurrence, are each independently selected from H, $C_{1-6}$ alkyl (e.g., methyl, ethyl, or *tert*-butyl), $C_{3-10}$ cyclohydrocarbyl (e.g., cyclopropyl), 3- to 10-membered heterocyclyl (e.g., pyrrolidinyl, morpholinyl, or piperidinyl optionally substituted with F, preferably piperidinyl optionally substituted with F), $C_{6-10}$ aryl (phenyl optionally substituted with F), and 5- to 14-membered heteroaryl (e.g., pyridinyl); the alkyl, cyclohydrocarbyl, heterocyclyl, aryl, and heteroaryl are further optionally substituted with one or more substituents independently selected from: halogen (e.g., F), - OH, halogenated $C_{1-6}$ alkyl (e.g., trifluoromethyl), and $C_{3-6}$ cyclohydrocarbyl (e.g., cyclopropyl).

**6.** The compound, or the pharmaceutically acceptable salt, the ester, the stereoisomer, the tautomer, the polymorph, the solvate, the metabolite, the isotopically labeled compound or the prodrug thereof according to any one of claims 1-5, wherein $R^1$ is selected from methyl, cyclopropyl, cyclohexyl, tetrahydropyrrolyl, tetrahydropyranyl, piperidinyl, morpholinyl, phenyl, pyrazolyl, pyridinyl, pyrimidinyl, pyridazinyl, and imidazopyridinyl; the groups are each optionally substituted with one or more substituents independently selected from: -F, -Cl, -OH, -CN, $-NH_2$, $-CH_3$, $-CF_3$, $-CH_2CF_2CF_3$, $-NHCH_2CF_3$,

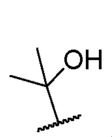 , 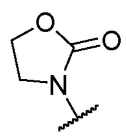 ,

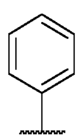 , 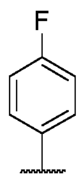 , 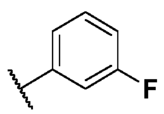 , 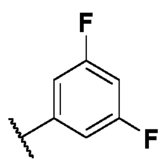 , 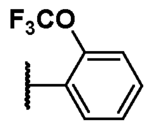 , 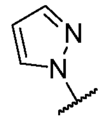 , 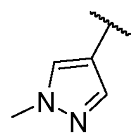 , 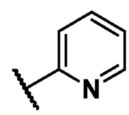 ,

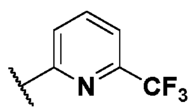 , 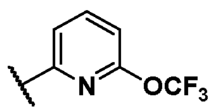 , 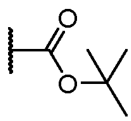 , 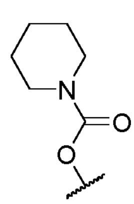 , 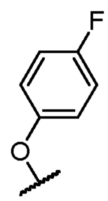 , 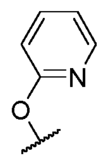 , 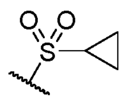 ,

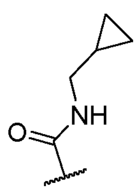 , 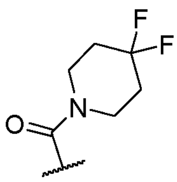 , 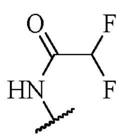 , 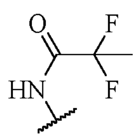 , 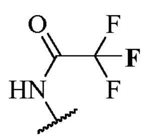 , 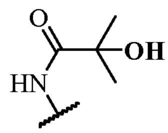 , 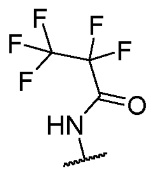 ,

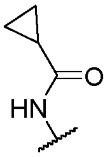, 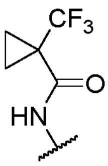, 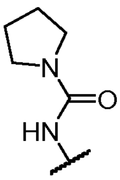, 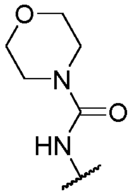, 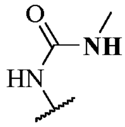, 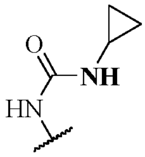, 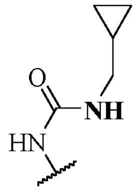, 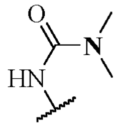,

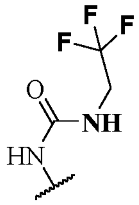, 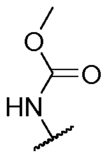, 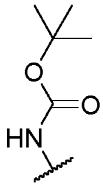, 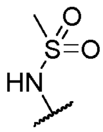, 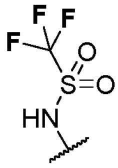, and 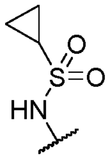;

preferably, $R^1$ is selected from methyl, cyclohexyl, tetrahydropyrrolyl, tetrahydropyranyl, piperidinyl, morpholinyl, phenyl, pyrazolyl, pyridinyl, and imidazopyridinyl; the groups are each optionally substituted with one or more substituents independently selected from: -F, -Cl, -OH, -CN, $-CH_3$, $-CF_3$, $-CH_2CF_2CF_3$, $-NHCH_2CF_3$,

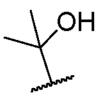, 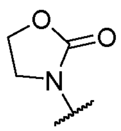, 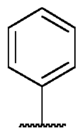, 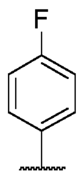, 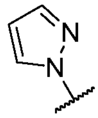, 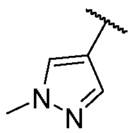, 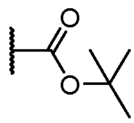, 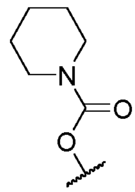,

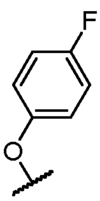, 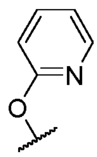, 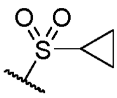, 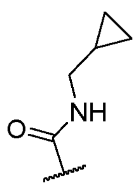, 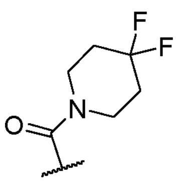, 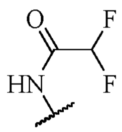, 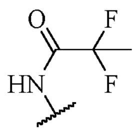, 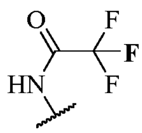,

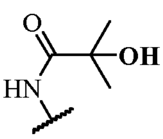, 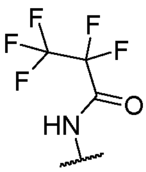, 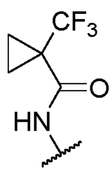, 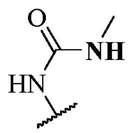, 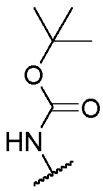, 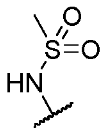, and 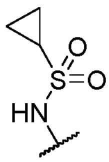.

**7.** The compound, or the pharmaceutically acceptable salt, the ester, the stereoisomer, the tautomer, the polymorph, the solvate, the metabolite, the isotopically labeled compound or the prodrug thereof according to any one of claims 1-6, wherein $R^1$ has the following structure:

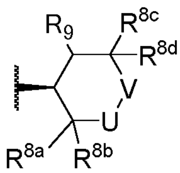

wherein:

U and V are each independently $CR^{8e}R^{8f}$, $NR^{8g}$, or O;

$R^{8a}$, $R^{8b}$, $R^{8c}$, $R^{8d}$, $R^{8e}$, $R^{8f}$, and $R^{8g}$ are each independently H or halogen (e.g., F);
$R^9$ is $-OR^c$, $-NR^c-C(=O)R^d$, $-NR^cR^d$, $-NR^c-C(=O)OR^d$, $-NR^c-S(=O)_2-R^d$, $-NR^c-C(=O)-NR^cR^d$, or $-OC(=O)R^c$.

**8.** The compound, or the pharmaceutically acceptable salt, the ester, the stereoisomer, the tautomer, the polymorph, the solvate, the metabolite, the isotopically labeled compound or the prodrug thereof according to any one of claims 1-7, wherein $R^1$ is selected from methyl,

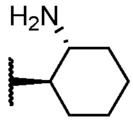 , 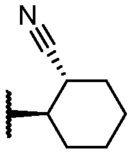 , 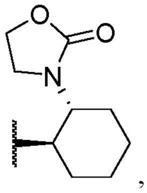 ,

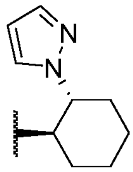 , 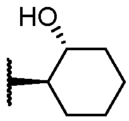 , 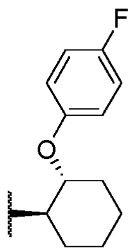 , 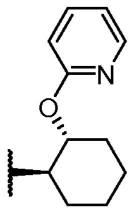 , 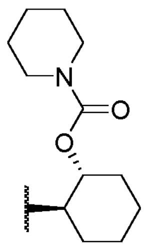 , 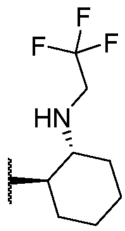 , 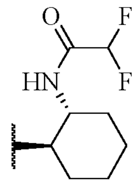 ,

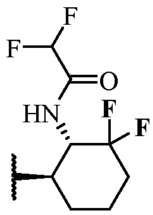 , 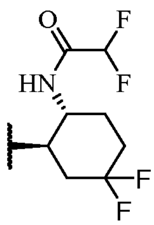 , 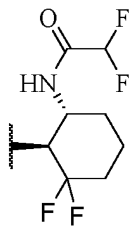 , 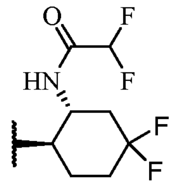 , 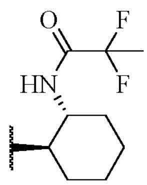 , 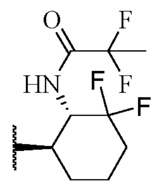 ,

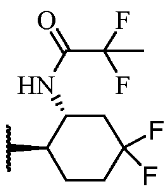 , 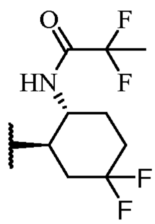 , 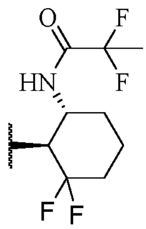 , 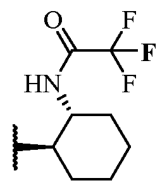 , 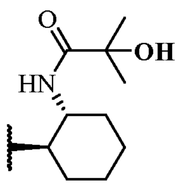 , 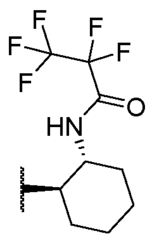 ,

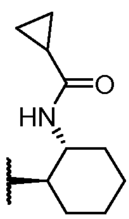 , 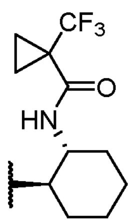 , 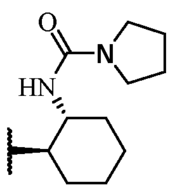 , 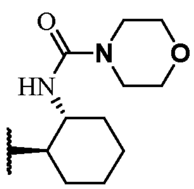 , 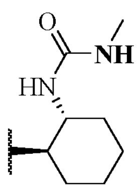 , 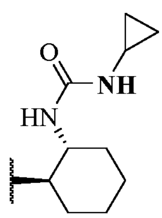 , 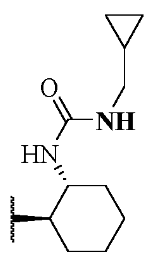 ,

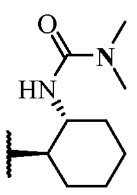 , 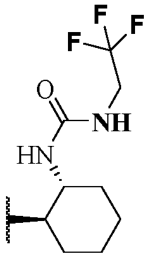 , 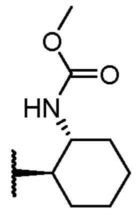 , 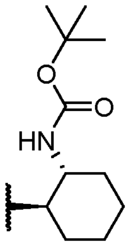 , 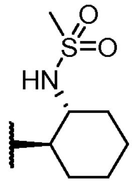 , 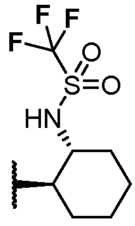 , 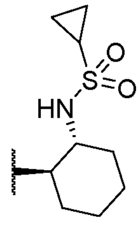 ,

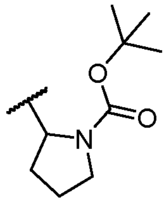 , 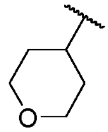 , 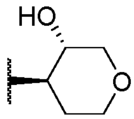 , 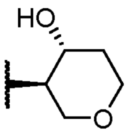 , 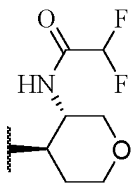 , 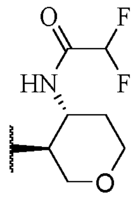 , 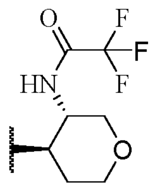 ,

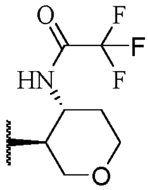 , 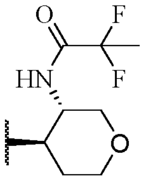 , 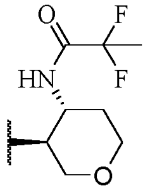 , 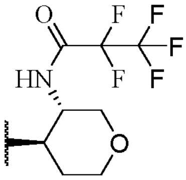 , 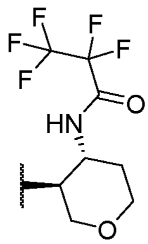 , 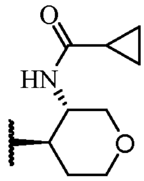 , 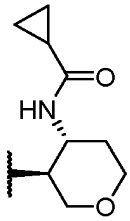 ,

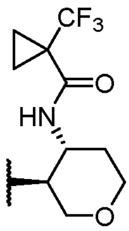 , 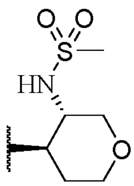 , 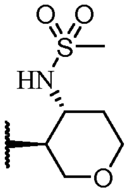 , 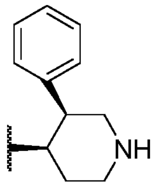 , 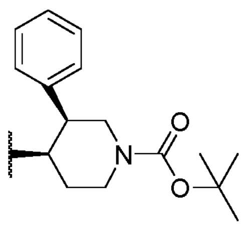 , 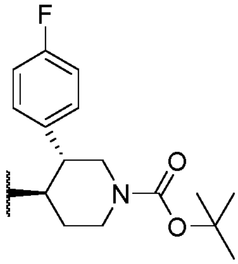 ,

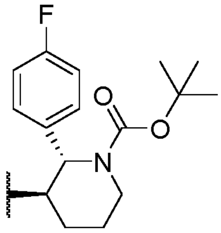 , 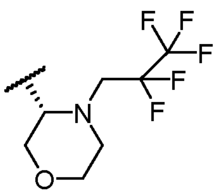 , 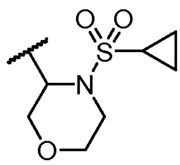 , 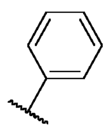 , 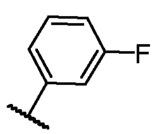 , 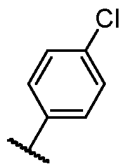 ,

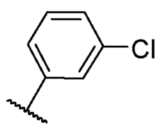 , 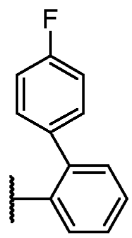 , 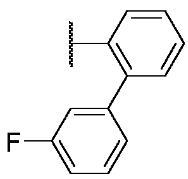 , 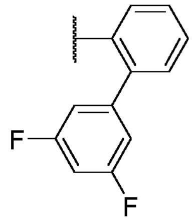 , 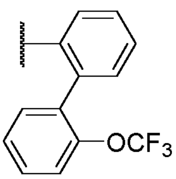 , 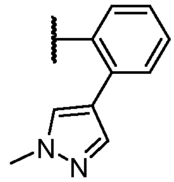 ,

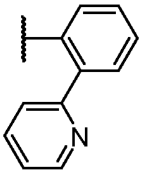 , 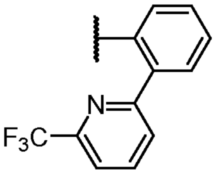 , 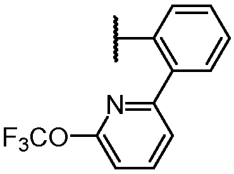 , 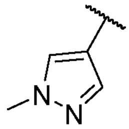 , 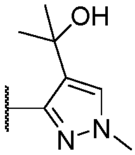 , 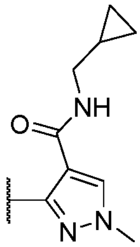 ,

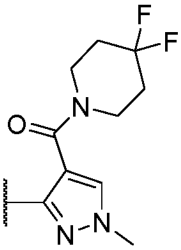 , 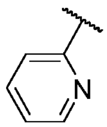 , 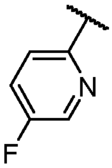 , 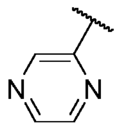 , 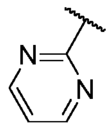 , and 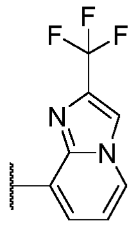 ;

preferably, $R^1$ is selected from methyl,

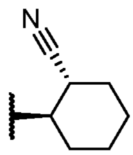 , 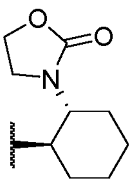 , 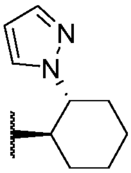 , 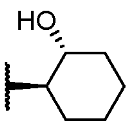 , 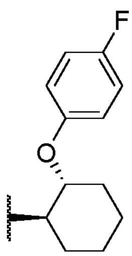 ,

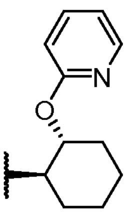 , 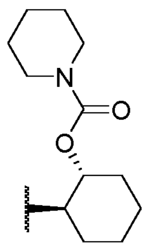 , 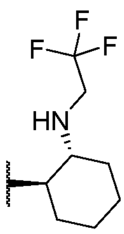 , 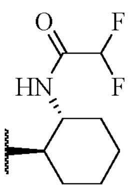 , 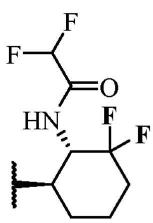 , 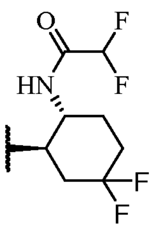 , 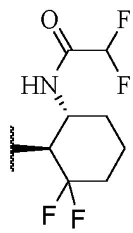 ,

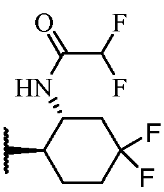 , 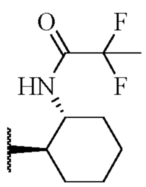 , 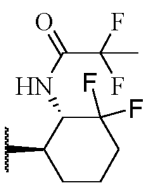 , 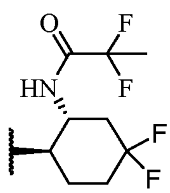 , 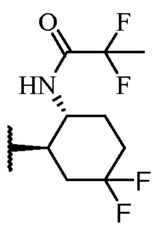 , 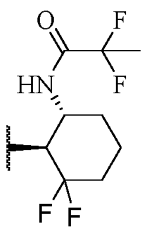 ,

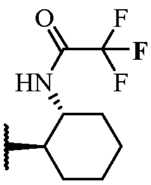 , 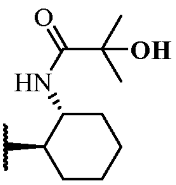 , 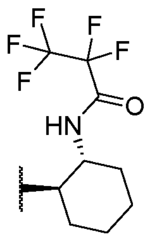 , 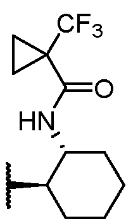 , 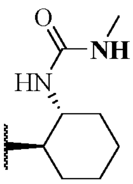 , 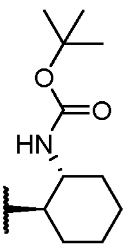 , 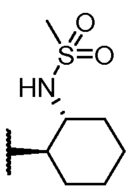 ,

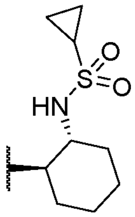, 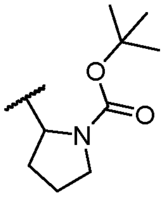, 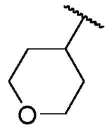, 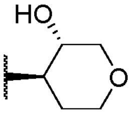, 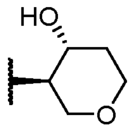, 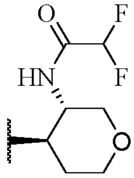, 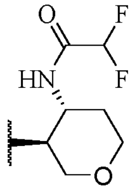,

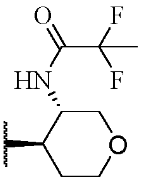, 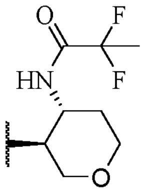, 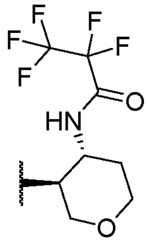, 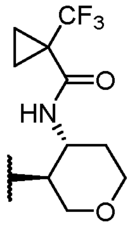, 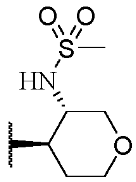, 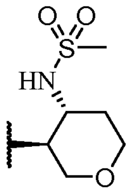, 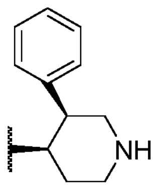,

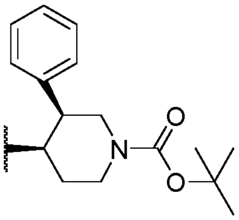, 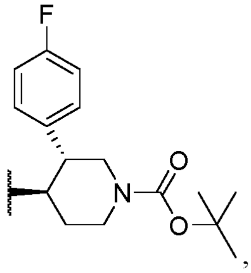, 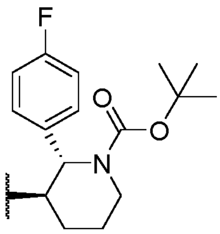, 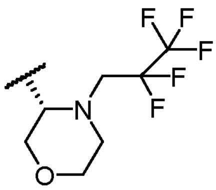, 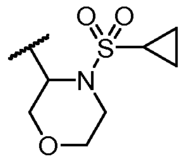,

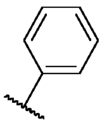, 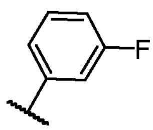, 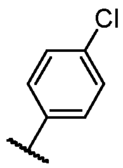, 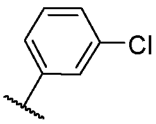, 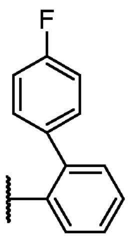, 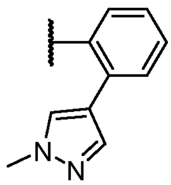, 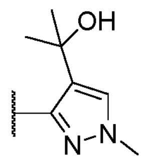,

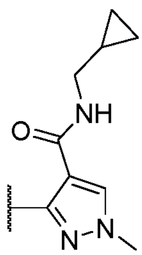, 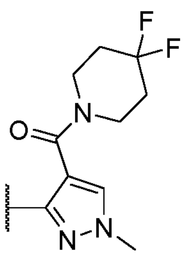, 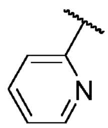, and 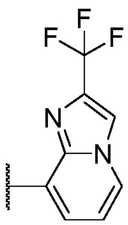.

**9.** The compound, or the pharmaceutically acceptable salt, the ester, the stereoisomer, the tautomer, the polymorph, the solvate, the metabolite, the isotopically labeled compound or the prodrug thereof according to any one of claims 1-8, wherein $R^2$ and $R^3$ are H or $C_{1-6}$ alkyl, preferably H or methyl; or $R^2$ and $R^3$ together form oxo (=O); or $R^2$ and $R^3$, together with the carbon atom to which they are attached, form $C_{3-6}$ cyclohydrocarbyl, preferably cyclopropyl.

**10.** The compound, or the pharmaceutically acceptable salt, the ester, the stereoisomer, the tautomer, the polymorph, the solvate, the metabolite, the isotopically labeled compound or the prodrug thereof according to any one of claims 1-9, wherein $R^4$, $R^5$, and $R^6$ are each independently H or halogen; preferably, $R^4$, $R^5$, and $R^6$ are each independently H or F.

**11.** The compound, or the pharmaceutically acceptable salt, the ester, the stereoisomer, the tautomer, the polymorph, the solvate, the metabolite, the isotopically labeled compound or the prodrug thereof according to any one of claims 1-10,

wherein the compound has a structure of formula (II) or formula (III):

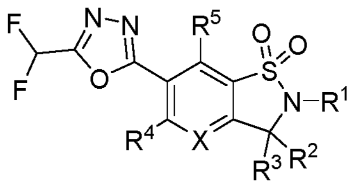

(II)

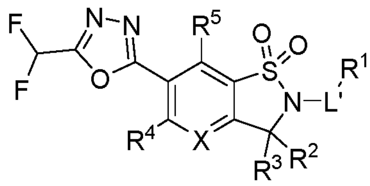

(III)

wherein:

L' is -$C_{1-6}$ alkylene-, wherein the alkylene is optionally substituted with one or more substituents independently selected from: -OH, $C_{3-6}$ cyclohydrocarbyl, 3- to 10-membered heterocyclyl, $C_{6-10}$ aryl, and 5-to 14-membered heteroaryl; preferably, the alkylene is optionally substituted with one or more substituents independently selected from: -OH, cyclopropyl, and phenyl optionally substituted with one or more halogen;
the other groups are as defined in any one of claims 1-10.

**12.** The compound, or the pharmaceutically acceptable salt, the ester, the stereoisomer, the tautomer, the polymorph, the solvate, the metabolite, the isotopically labeled compound or the prodrug thereof according to any one of claims 1-11, wherein the compound is selected from:

| No. | Structure |
|---|---|
| 1. | 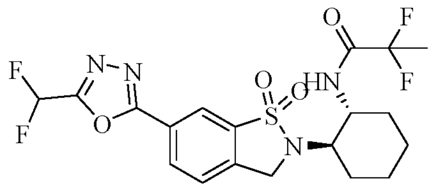 |
| 2. | 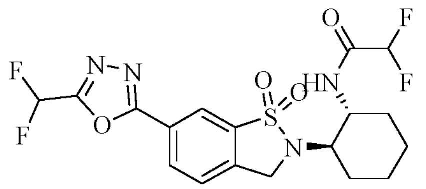 |
| 3. | 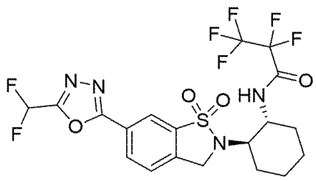 |
| 4. | 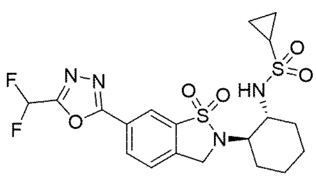 |
| 5. | 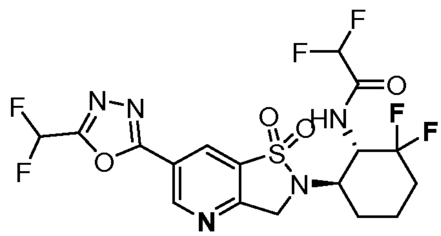 |

(continued)

| No. | Structure |
|---|---|
| 6. | 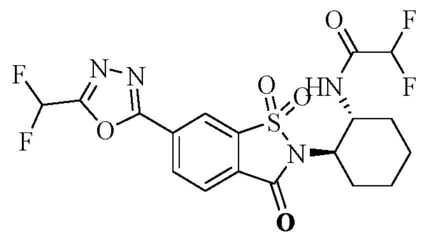 |
| 7. | 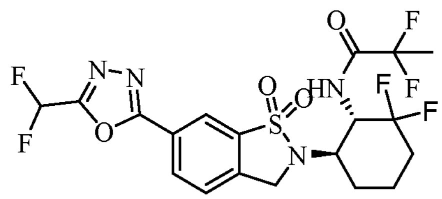 |
| 8. | 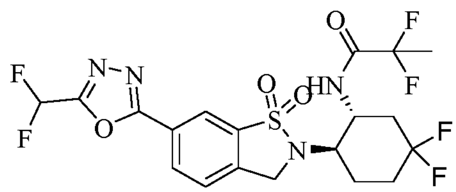 |
| 9. | 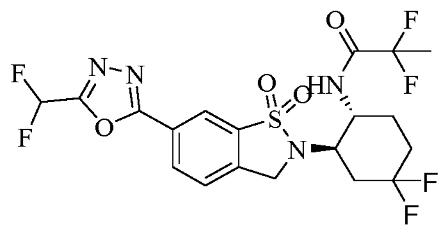 |
| 10. | 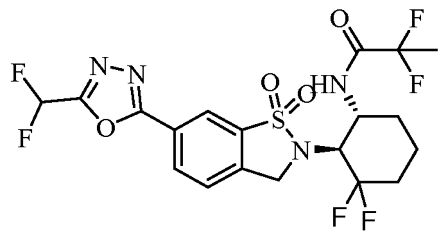 |
| 11. | |
| 12. | |
| 13. | 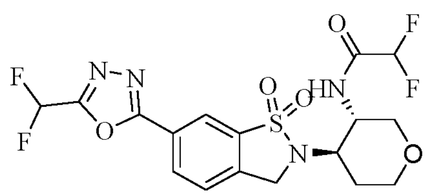 |
| 14. | 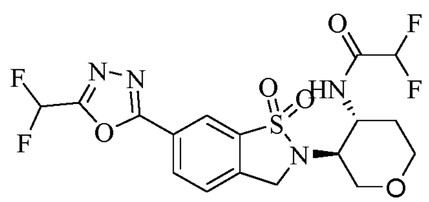 |

(continued)

| No. | Structure |
|---|---|
| 15. | 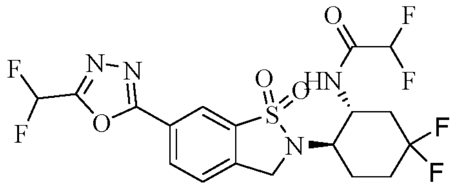 |
| 16. | 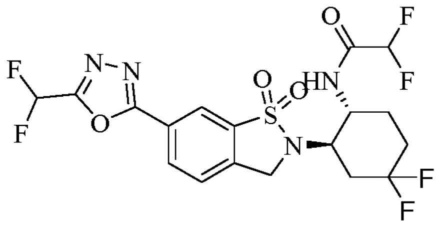 |
| 17. | 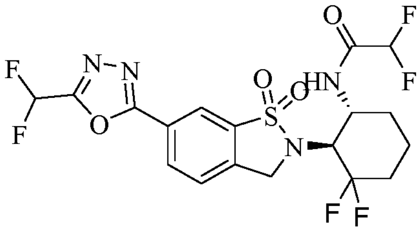 |
| 18. | 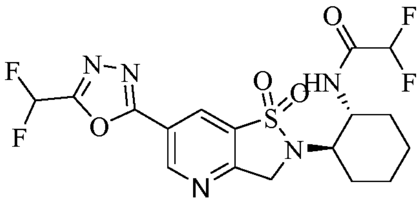 |
| 19. | 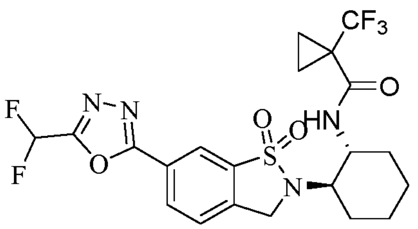 |
| 20. | 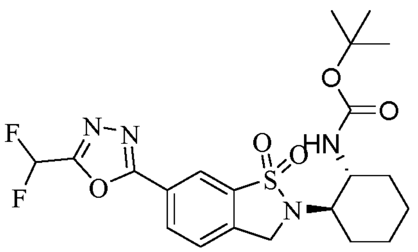 |
| 21. | 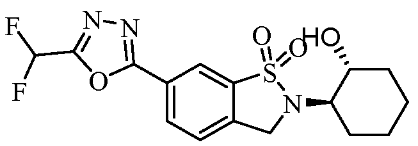 |

(continued)

| No. | Structure |
|---|---|
| **22.** | 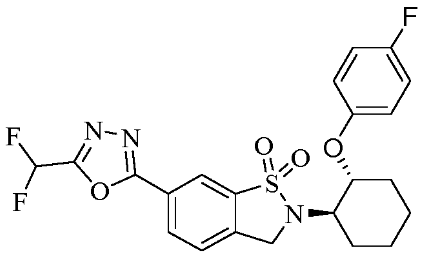 |
| **23.** | 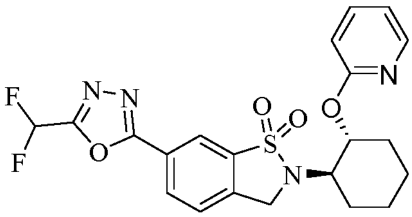 |
| **24.** | 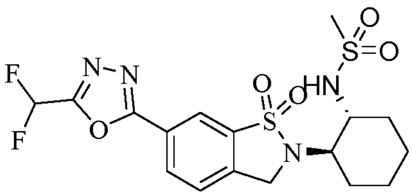 |
| **25.** | 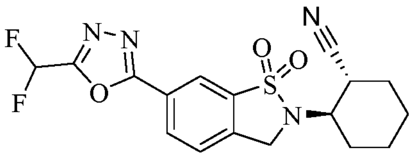 |
| **26.** | 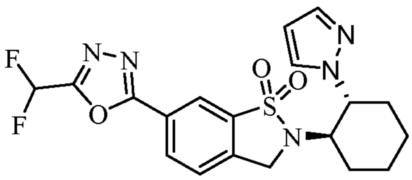 |
| **27.** | 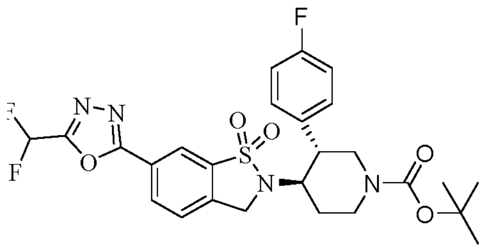 |
| **28.** | 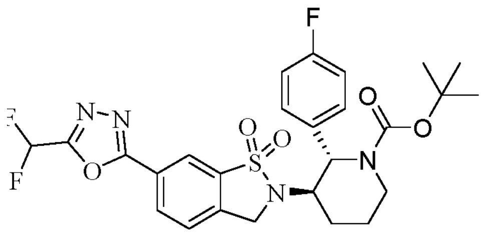 |

(continued)

| No. | Structure |
|---|---|
| 29. | 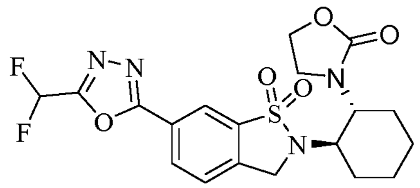 |
| 30. | 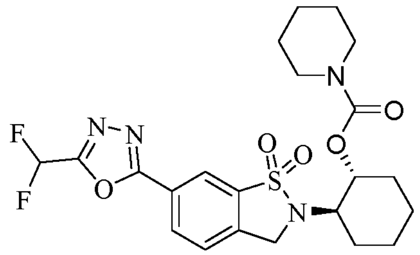 |
| 31. | 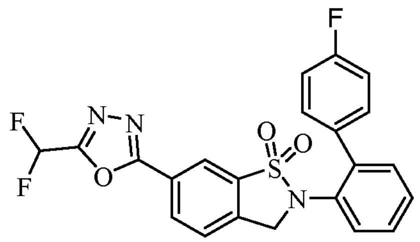 |
| 32. | 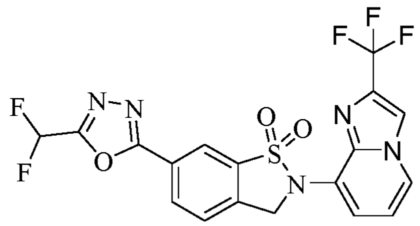 |
| 33. | 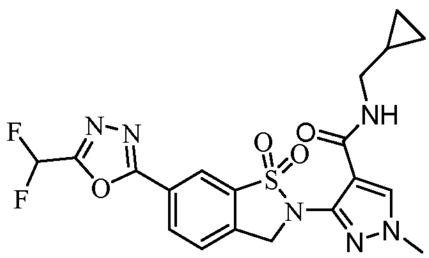 |
| 34. | 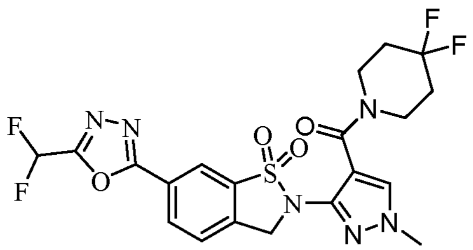 |
| 35. | 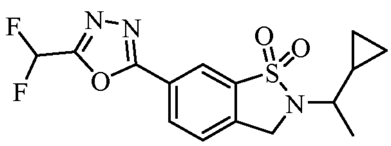 |

(continued)

| No. | Structure |
|---|---|
| 36. | 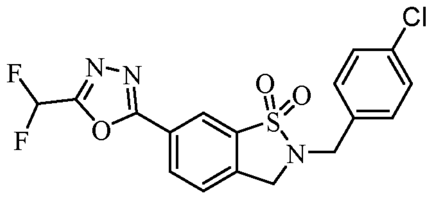 |
| 37. | 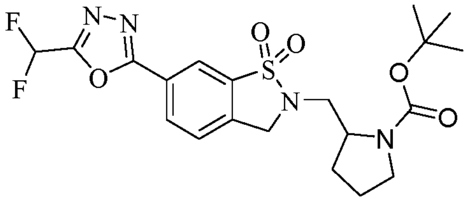 |
| 38. | 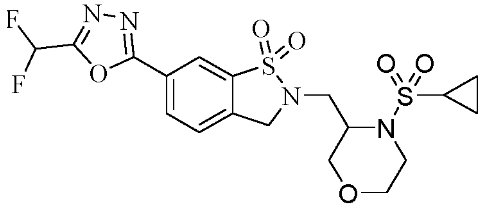 |
| 39. | 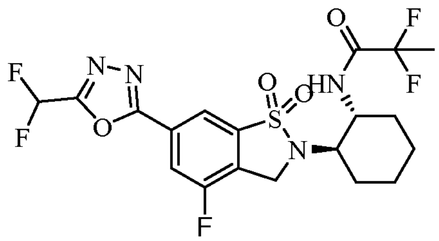 |
| 40. | 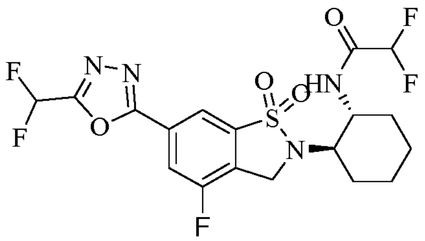 |
| 41. | 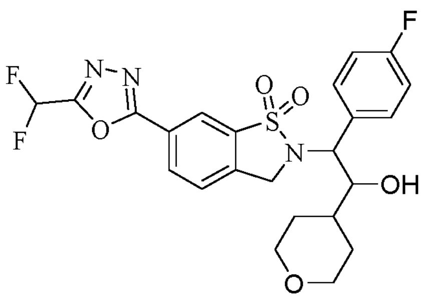 |

(continued)

| No. | Structure |
|---|---|
| 42. | 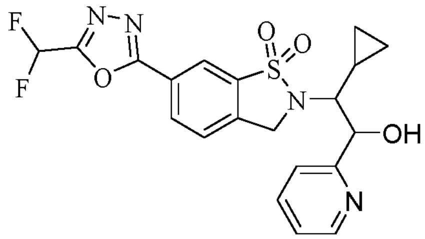 |
| 43. | 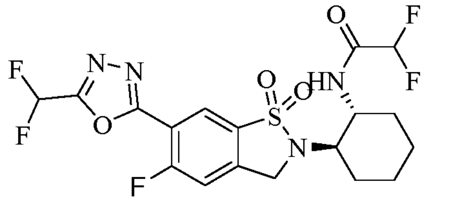 |
| 44. | 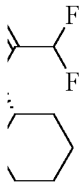 |
| 45. | |
| 46. | |
| 47. | |
| 48. | |
| 49. | |
| 50. | |
| 51. | |
| 52. | |
| 53. | |
| 54. | |
| 55. | |
| 56. | |
| 57. | 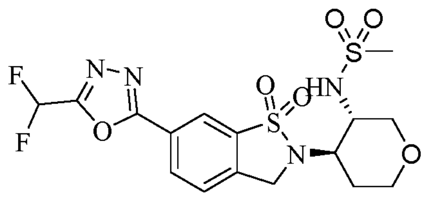 |
| 58. | 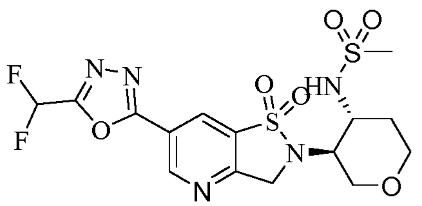 |

(continued)

| No. | Structure |
|---|---|
| 59. | 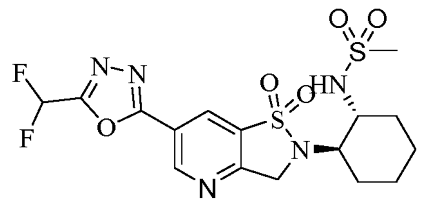 |
| 60. | 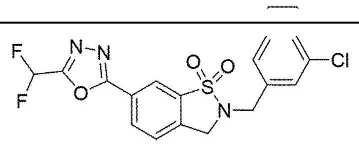 |
| 61. | 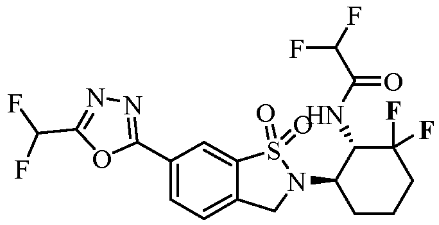 |
| 62. | 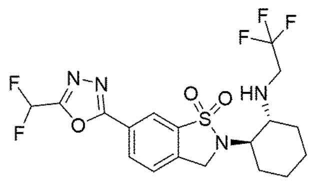 |
| 63. | 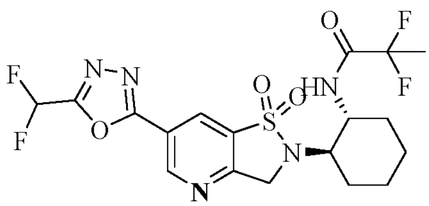 |
| 64. | 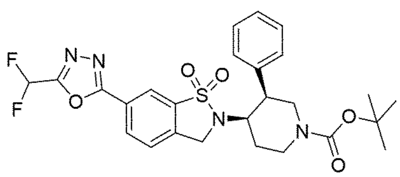 |
| 65. | 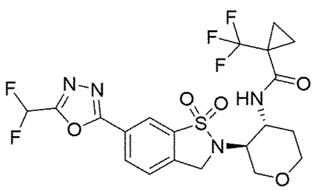 |
| 66. | 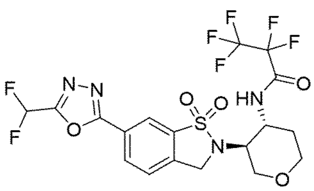 |

(continued)

| No. | Structure |
|---|---|
| 67. | 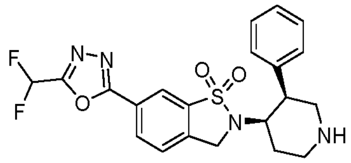 |
| 68. | 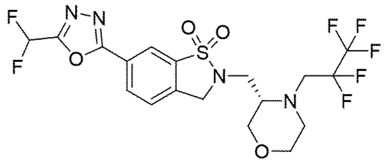 |
| 69. | 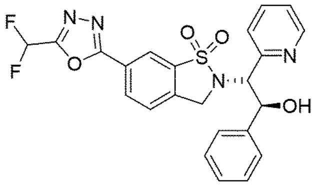 |
| 70. | 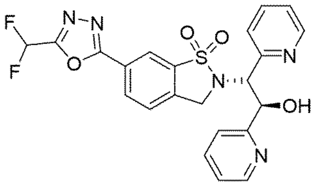 |
| 71. | 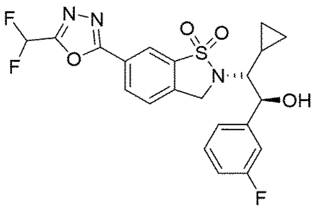 |
| 72. | 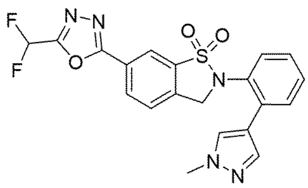 |
| 73. | 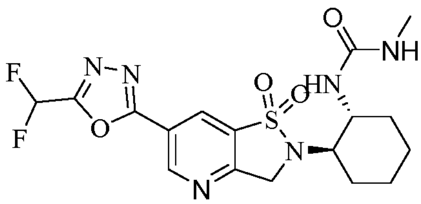 |
| 74. | 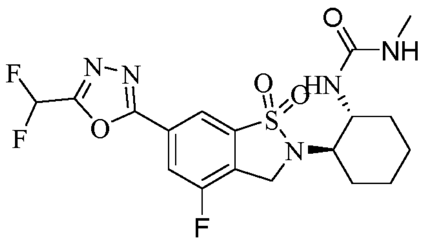 |

(continued)

| No. | Structure |
|---|---|
| 75. | |
| 76. | |
| 77. | |
| 78. | 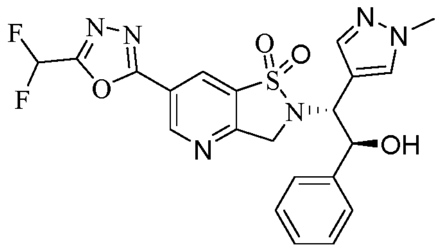 |
| 79. | 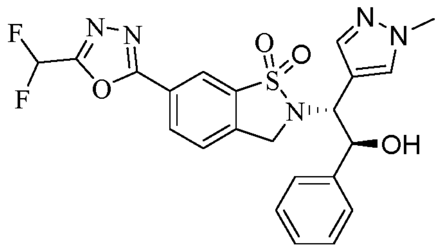 |
| 80. | 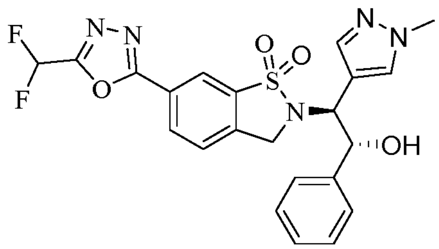 |
| 81. | 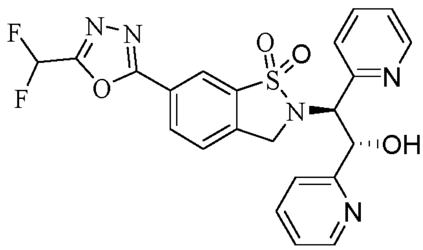 |
| 82. | 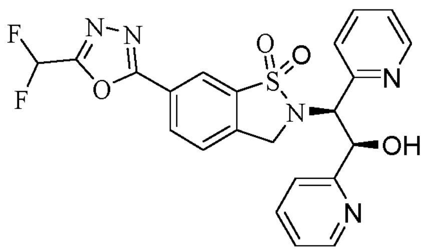 |
| 83. | 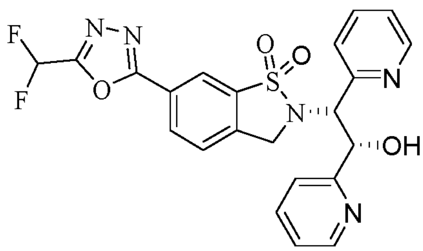 |

(continued)

| No. | Structure |
|---|---|
| 84. | 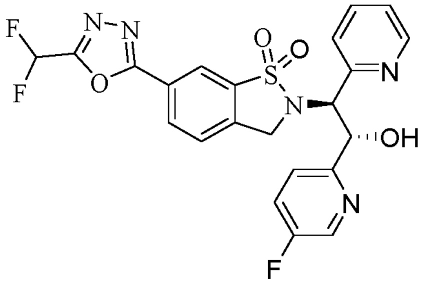 |
| 85. | 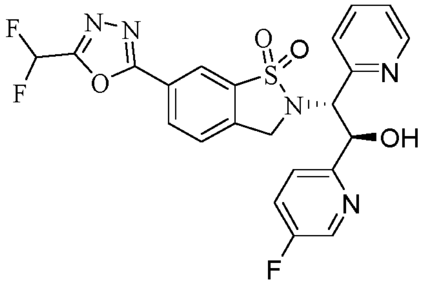 |
| 86. | 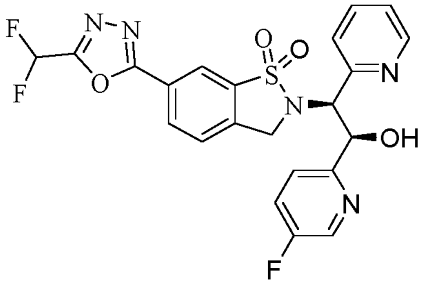 |
| 87. | 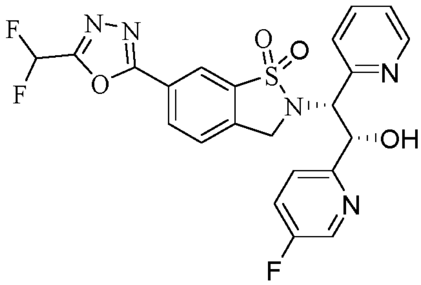 |
| 88. | 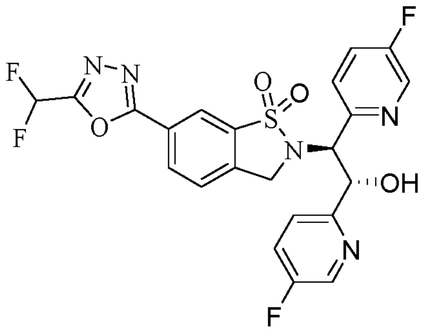 |

(continued)

| No. | Structure |
|---|---|
| 89. | 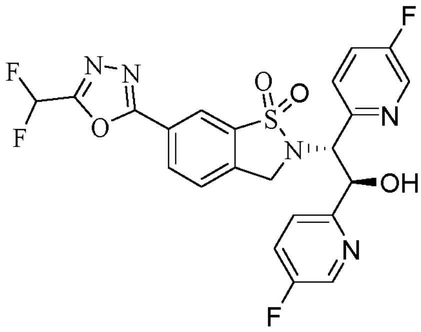 |
| 90. | 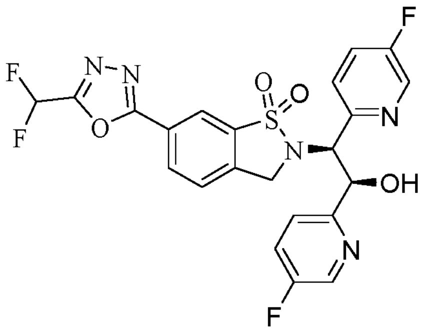 |
| 91. | 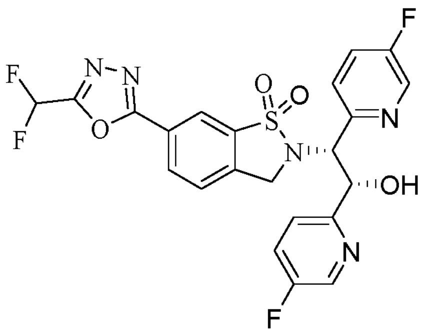 |
| 92. | 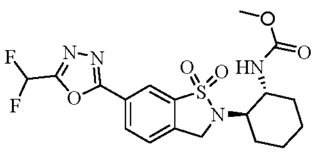 |
| 93. | 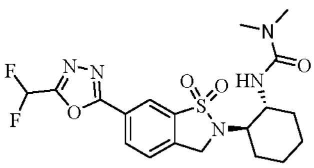 |
| 94. | 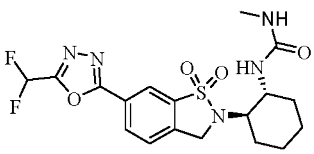 |

(continued)

| No. | Structure |
|---|---|
| 95. | 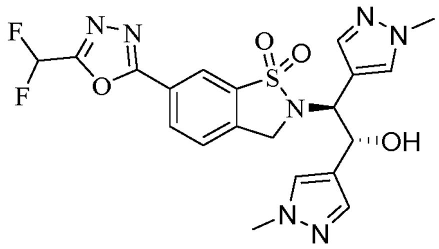 |
| 96. | 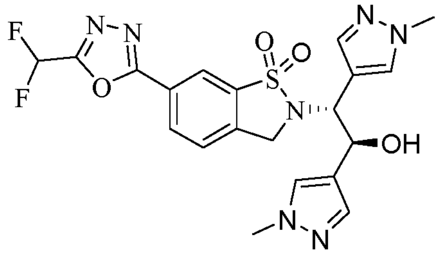 |
| 97. | 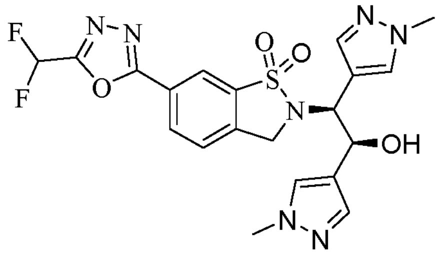 |
| 98. | 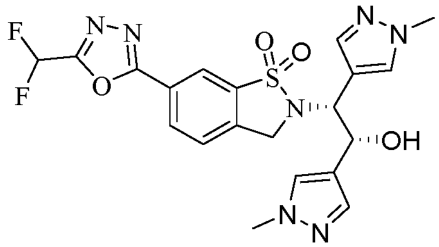 |
| 99. | 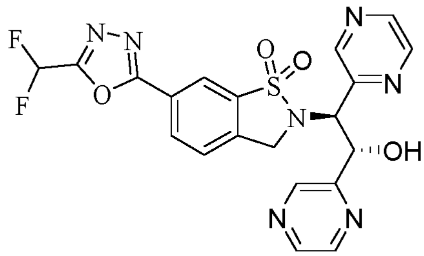 |
| 100. | 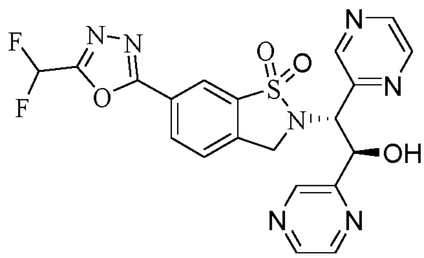 |

(continued)

| No. | Structure |
|---|---|
| 101. | 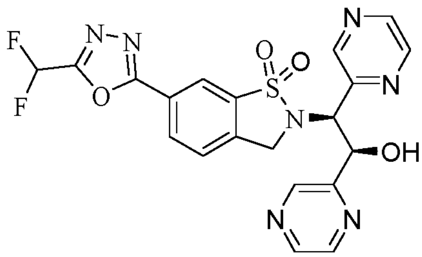 |
| 102. | 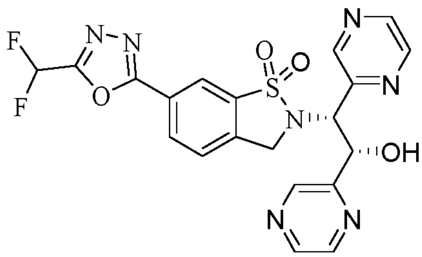 |
| 103. | |
| 104. | |
| 105. | |
| 106. | |
| 107. | 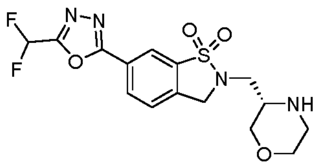 |
| 108. | 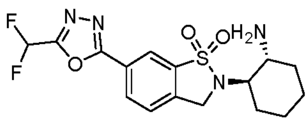 |
| 109. | 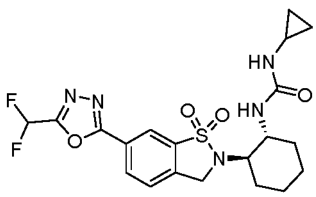 |
| 110. | 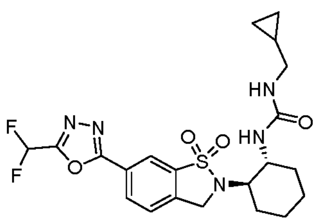 |

(continued)

| No. | Structure |
|---|---|
| 111. | 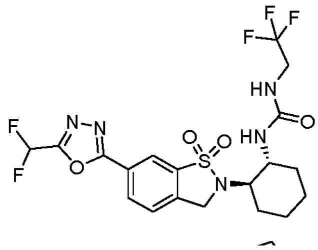 |
| 112. | 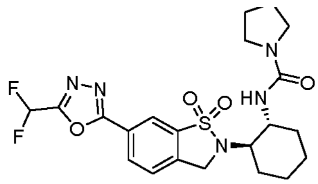 |
| 113. | 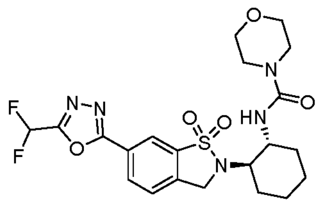 |
| 114. | 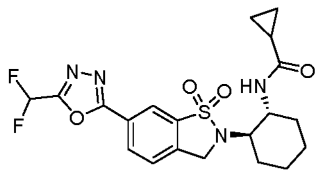 |
| 115. | |
| 116. | 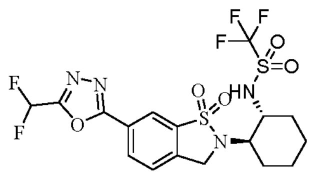 |
| 117. | 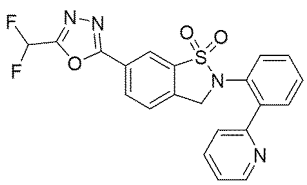 |
| 118. | 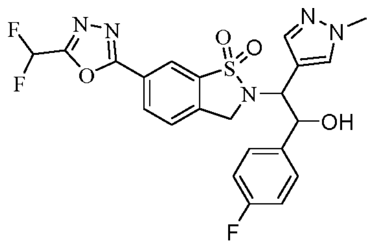 |

(continued)

| No. | Structure |
|---|---|
| 119. | 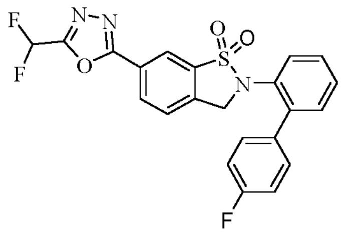 |
| 120. | 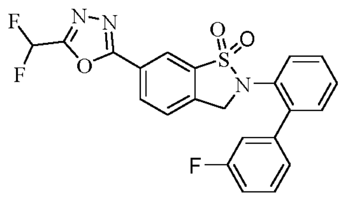 |
| 121. | 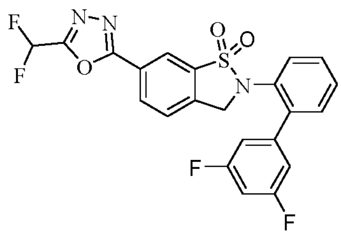 |
| 122. | 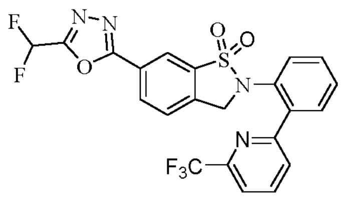 |
| 123. | 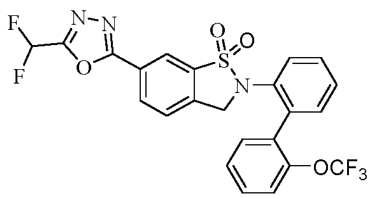 |
| 124. | 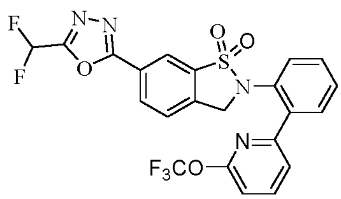 |
| 125. | 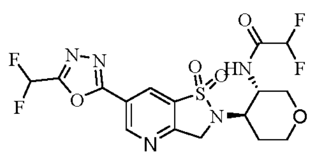 |
| 126. | 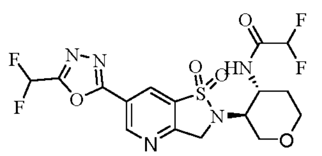 |

(continued)

| No. | Structure |
|---|---|
| 127. | 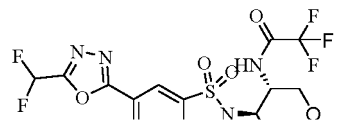 |
| 128. | 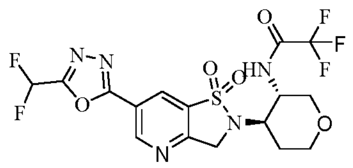 |
| 129. | 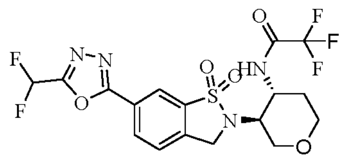 |
| 130. | 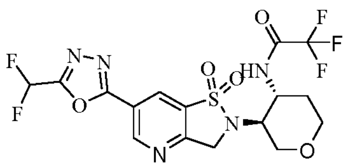 |
| 131. | 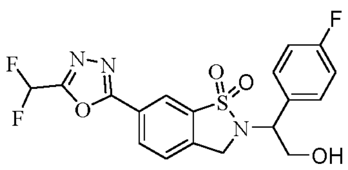 |
| 132. | 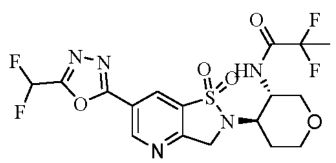 |
| 133. | 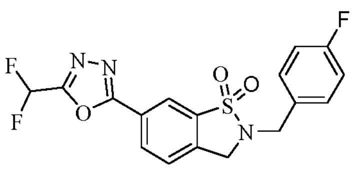 |
| 134. | 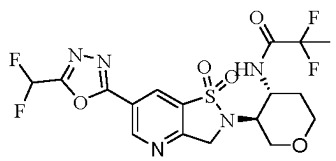 |
| 135. | 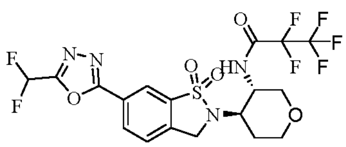 |

(continued)

| No. | Structure |
|---|---|
| 136. | 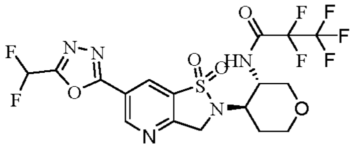 |
| 137. | 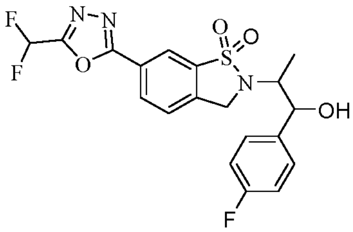 |
| 138. | 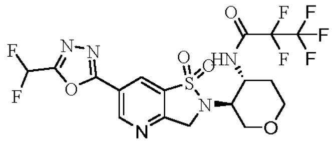 |
| 139. | 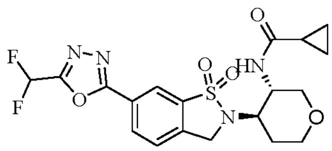 |
| 140. | 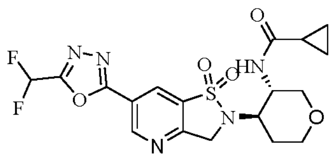 |
| 141. | 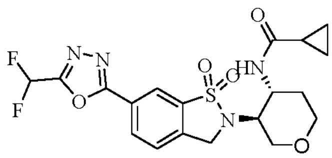 |
| 142. | 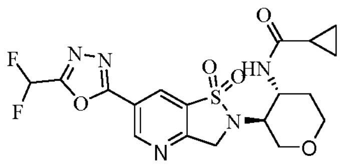 |
| 143. | 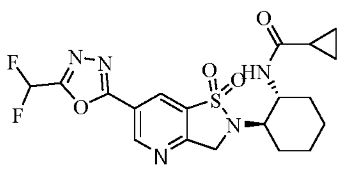 |
| 144. | 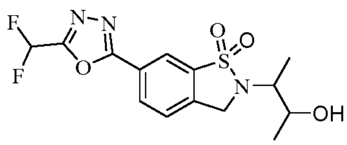 |

(continued)

| No. | Structure |
|---|---|
| 145. | 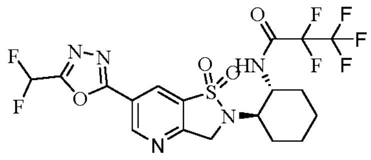 |
| 146. | 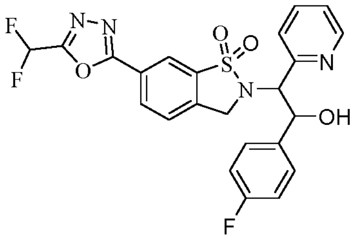 |
| 147. | 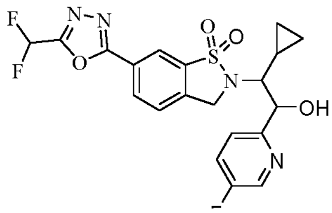 |
| 148. | 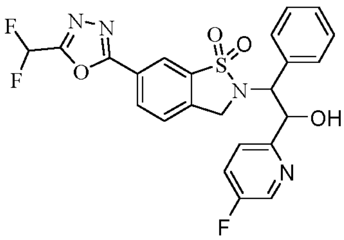 |
| 149. | 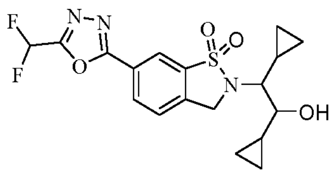 |
| 150. | 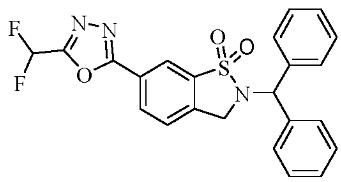 |
| 151. | 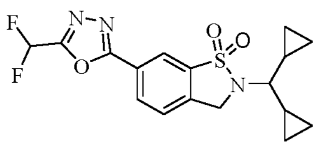 |

(continued)

| No. | Structure |
|---|---|
| 152. | 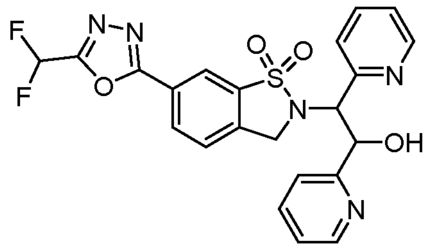 |
| 153. | 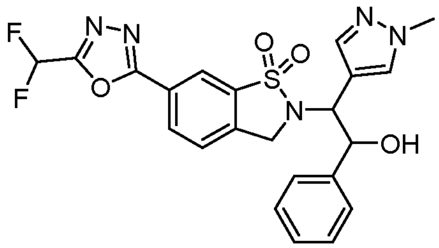 |
| 154. | 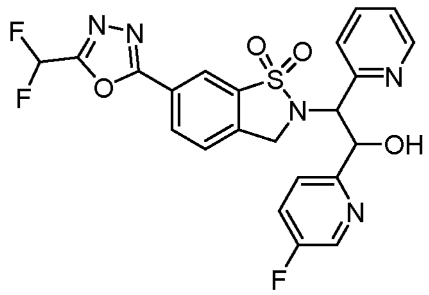 |
| 155. | 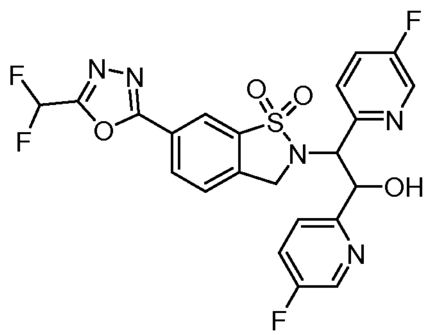 |
| 156. | 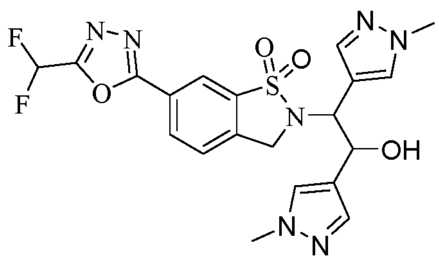 |

(continued)

| No. | Structure |
|---|---|
| 157. | 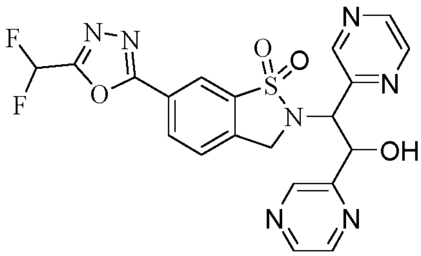 |
| 158. | 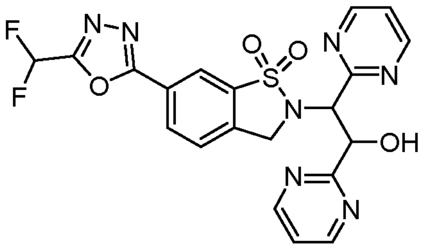 |
| 159. | 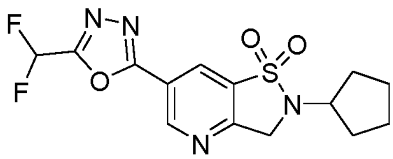 |

**13.** A pharmaceutical composition comprising the compound, or the pharmaceutically acceptable salt, the ester, the stereoisomer, the tautomer, the polymorph, the solvate, the metabolite, the isotopically labeled compound or the prodrug thereof according to any one of claims 1-12, and one or more pharmaceutically acceptable carriers, wherein the pharmaceutical composition is preferably a solid formulation, a liquid formulation, or a transdermal formulation.

**14.** Use of the compound, or the pharmaceutically acceptable salt, the ester, the stereoisomer, the tautomer, the polymorph, the solvate, the metabolite, the isotopically labeled compound or the prodrug thereof according to any one of claims 1-12, or the pharmaceutical composition according to claim 13 in the preparation of a medicament for preventing or treating an HDAC6-related disease.

**15.** The use according to claim 14, wherein the HDAC6-related disease is selected from cancer or proliferative diseases (e.g., lung cancer, colon cancer, breast cancer, prostate cancer, liver cancer, brain cancer, kidney cancer, ovarian cancer, stomach cancer, skin cancer, bone cancer, pancreatic cancer, glioma, glioblastoma, hepatocellular carcinoma, papillary renal carcinoma, head and neck squamous cell carcinoma, leukemia, lymphoma, myeloma, multiple myeloma, and solid tumors); Wilson's disease, spinocerebellar ataxia, prion disease, Parkinson's disease, Huntington's disease, amyotrophic lateral sclerosis, amyloidosis, Alzheimer's disease, Alexander's disease, alcoholic liver disease, cystic fibrosis, Pick's disease, spinal muscular atrophy, or Lewy body dementia; rheumatoid arthritis, osteoarthritis; rheumatoid spondylitis; psoriasis; inflammatory bowel disease; chronic inflammatory lung disease, eczema, asthma, ischemia/reperfusion injury, ulcerative colitis, acute respiratory distress syndrome, psoriatic arthritis, infectious arthritis, progressive chronic arthritis, arthritis deformans, osteoarthritis, traumatic arthritis, gouty arthritis, Reiter's syndrome, polychondritis, acute synovitis and spondylitis, glomerulonephritis, hemolytic anemia, aplastic anemia, idiopathic thrombocytopenic purpura, neutropenia, ulcerative colitis, Crohn's disease, host-versus-graft disease, graft-versus-host disease, allograft rejection, chronic thyroiditis, Graves' disease, scleroderma, diabetes, active hepatitis, primary biliary cirrhosis, myasthenia gravis, multiple sclerosis (MS), systemic lupus erythematosus, atopic dermatitis, contact dermatitis, sunburn of the skin, chronic renal insufficiency, Stevens-Johnson syndrome, idiopathic steatorrhea, sarcoidosis, Guillain-Barre syndrome, uveitis, conjunctivitis, keratoconjunctivitis, otitis media, periodontal disease, pulmonary interstitial fibrosis, asthma, bronchitis, rhinitis, sinusitis, pneumoconiosis, pulmonary insufficiency syndrome, emphysema, pulmonary fibrosis, or silicosis.

**INTERNATIONAL SEARCH REPORT**

International application No.
**PCT/CN2023/088285**

**A. CLASSIFICATION OF SUBJECT MATTER**

C07D417/04(2006.01)i； A61K31/545(2006.01)i； A61K31/5415(2006.01)i； A61P35/00(2006.01)i； A61P25/28(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

STN(REGISTRY, CAPLUS), VEN, CNABS, CNTXT, CNKI: 噁二唑, 磺酰胺, 组蛋白去乙酰化酶抑制剂, 中枢神经系统, 癌症, HDAC, oxadiazol, sulfonamide, histone deacetylase, inhibitor, central nervous system disease, cancer, tumor, STN中的结构式检索, structural formula search in STN

**C. DOCUMENTS CONSIDERED TO BE RELEVANT**

| Category* | Citation of document, with indication, where appropriate, of the relevant passages | Relevant to claim No. |
|---|---|---|
| X | CN 111032651 A (TAKEDA PHARMACEUTICALS CO., LTD.) 17 April 2020 (2020-04-17) description, page 1, paragraph 1, pages 198-264, table 1-1 to table 1-67, and claims 1-22 | 1-15 |
| X | US 20220098180 A1 (TAKEDA PHARMACEUTICALS COMPANY LIMITED) 31 March 2022 (2022-03-31) description, page 3, paragraphs 20-22, pages 73-86, table 3, and claims 1-16 | 1-15 |
| X | WO 2021060567 A1 (TAKEDA PHARMACEUTICALS COMPANY LIMITED) 01 April 2021 (2021-04-01) description, page 1, paragraph 1, pages 94-115, table 1-1 to table 1-22, and claims 1-15 | 1-15 |
| A | WO 2021255091 A1 (BAYER AKTIENGESELLSCHAFT) 23 December 2021 (2021-12-23) description, page 1, paragraph 1, and claim 1 | 1-15 |

☐ Further documents are listed in the continuation of Box C.

☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **19 June 2023** | **25 June 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/088285**

| Patent document cited in search report | Publication date (day/month/year) | Patent family member(s) | Publication date (day/month/year) |
|---|---|---|---|
| CN 111032651 A | 17 April 2020 | US 2019135799 A1 | 09 May 2019 |
| | | US 10435399 B2 | 08 October 2019 |
| | | JP 2020529393 A | 08 October 2020 |
| | | JP 7135007 B2 | 12 September 2022 |
| | | TW 201910328 A | 16 March 2019 |
| | | WO 2019027054 A1 | 07 February 2019 |
| | | EP 3661931 A1 | 10 June 2020 |
| | | CA 3071825 A1 | 07 February 2019 |
| US 20220098180 A1 | 31 March 2022 | JPWO 2020158762 A1 | 02 December 2021 |
| | | EP 3919055 A1 | 08 December 2021 |
| | | EP 3919055 A4 | 09 November 2022 |
| | | WO 2020158762 A1 | 06 August 2020 |
| WO 2021060567 A1 | 01 April 2021 | AU 2020353984 A1 | 21 April 2022 |
| | | AU 2020353984 A2 | 04 August 2022 |
| | | ECSP 22026524 A | 31 May 2022 |
| | | JP 2022552444 A | 15 December 2022 |
| | | BR 112022005595 A2 | 19 July 2022 |
| | | US 2021094944 A1 | 01 April 2021 |
| | | US 11453661 B2 | 27 September 2022 |
| | | KR 20220070465 A | 31 May 2022 |
| | | TW 202126643 A | 16 July 2021 |
| | | EP 4034536 A1 | 03 August 2022 |
| | | CL 2022000763 A1 | 20 January 2023 |
| | | IL 291413 A | 01 May 2022 |
| | | CO 2022005059 A2 | 08 July 2022 |
| | | PE 20220711 A1 | 04 May 2022 |
| | | AR 120040 A1 | 26 January 2022 |
| | | CA 3156060 A1 | 01 April 2021 |
| WO 2021255091 A1 | 23 December 2021 | UY 39282 A | 31 January 2022 |
| | | BR 112022025692 A2 | 28 February 2023 |
| | | TW 202214613 A | 16 April 2022 |
| | | AR 122690 A1 | 28 September 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **T. HIGUCHI** ; **V. STELLA**. Pro-drugs as Novel Delivery Systems. *ACS Symposium Series*, vol. 14
- Bioreversible Carriers in Drug Design. Pergamon Press, 1987
- **H. BUNDGAARD**. Design of Prodrugs. Elsevier, 1985
- Protective Groups in Organic Chemistry. Plenum Press, 1973
- **T.W. GREENE** ; **P.G.M. WUTS**. Protective Groups in Organic Synthesis. John Wiley & Sons, 1991
- **BRIAN DAVIES** ; **TIM MORRIS**. Physiological Parameters in Laboratory Animals and Human. *Pharmaceutical Research*, 1993, vol. 10 (7)
- *Journal of Pharmacology and Experimental Therapeutics*, 1997, vol. 283 (1), 46-58